# EUROPEAN PATENT APPLICATION

(11) **EP 3 756 689 A1**
(43) Date of publication of application: **30.12.2020**
(21) Application number: 19757616.8
(22) Date of filing: 22.02.2019
(51) Int. Cl.: A61K 45/00, A61K 31/166, A61K 31/37, A61K 31/4166, A61K 31/426, A61K 31/439, A61K 31/445, A61K 31/455, A61K 31/4741, A61K 31/496, A61K 31/498, A61K 31/502, A61K 31/519, A61K 31/529, A61K 31/5377, A61K 31/538, A61K 31/553, A61K 31/7028, A61K 38/05, A61P 19/08, A61P 43/00

(54) **IL-17A ACTIVITY INHIBITOR AND USE THEREOF**

(30) Priority: 22.02.2018 JP 2018030061
(71) Applicant: Tokai University Educational System, Tokyo 151-0063 (JP); Nippon Zoki Pharmaceutical Co., Ltd., Chuo-ku Osaka-shi, Osaka 541-0046 (JP)
(72) Inventor: SAKAI Daisuke, Isehara-shi, Kanagawa 259-1193 (JP); HIRAYAMA Noriaki, Hiratsuka-shi, Kanagawa 259-1292 (JP); SUYAMA Kaori, Isehara-shi, Kanagawa 259-1193 (JP)
(74) Representative: Hoffmann Eitle
(86) International application number: PCT/JP2019/006786
(87) International publication number: WO 2019/163945

(57) **Abstract**

The present invention addresses the problem of providing a low-molecular-weight compound (IL-17 activity inhibitor) having a more superior IL-17 activity-inhibiting ability than those of the conventional compounds. The IL-17RA inhibitor according to the present invention is a compound which can bind to interleukin 17 receptor A (IL-17RA) through a non-covalent interaction including at least one intermolecular interaction selected from the group that includes a van der Waals force acting among at least 13 amino acid residues selected from amino acid residues Phe60, Gln87, Asp121,Pro122, Asp123, Gln124,Asp153,Cys154, Glu155,Lys160,Pro164, Cys165,Ser167,Ser168,Gly169,Ser170,Leu171,Trp172,Asp173,Pro174,Pro254,Phe256, Ser258,Cys259,Asp262,Cys263,Leu264 and His266 contained in, for example, an extracellular domain of human IL-17RA and preferably consists of an ionic bond, a hydrogen bond, a CH-π interaction and a hydrophobic interaction each acting among specified amino acid residues among the above-mentioned amino acid residues in a space surrounded by the above-mentioned amino acid residues, and which has an activity to inhibit the binding of interleukin-17A(IL-17A) to IL-17RA originated from human or the like, or a pharmaceutically acceptable salt, solvate or prodrug of the compound.

SEQUENCE LISTING

<110>  Tokai University Educational System

<120>  Inhibitor of IL-17A activity and the use thereof

<130>  PN58-9001WO

<150>  JP 2018-30061
<151>  2018-02-22

<160>  3

<170>  PatentIn version 3.5

<210>  1
<211>  866
<212>  PRT
<213>  Homo sapiens

<400>  1

Met Gly Ala Ala Arg Ser Pro Pro Ser Ala Val Pro Gly Pro Leu Leu
1               5                   10                  15

Gly Leu Leu Leu Leu Leu Leu Gly Val Leu Ala Pro Gly Gly Ala Ser
            20                  25                  30

Leu Arg Leu Leu Asp His Arg Ala Leu Val Cys Ser Gln Pro Gly Leu
        35                  40                  45

Asn Cys Thr Val Lys Asn Ser Thr Cys Leu Asp Asp Ser Trp Ile His
    50                  55                  60

Pro Arg Asn Leu Thr Pro Ser Ser Pro Lys Asp Leu Gln Ile Gln Leu
65                  70                  75                  80

His Phe Ala His Thr Gln Gln Gly Asp Leu Phe Pro Val Ala His Ile
                85                  90                  95

Glu Trp Thr Leu Gln Thr Asp Ala Ser Ile Leu Tyr Leu Glu Gly Ala
            100                 105                 110

Glu Leu Ser Val Leu Gln Leu Asn Thr Asn Glu Arg Leu Cys Val Arg
        115                 120                 125

Phe Glu Phe Leu Ser Lys Leu Arg His His His Arg Arg Trp Arg Phe
    130                 135                 140

Thr Phe Ser His Phe Val Val Asp Pro Asp Gln Glu Tyr Glu Val Thr
145                 150                 155                 160

Val His His Leu Pro Lys Pro Ile Pro Asp Gly Asp Pro Asn His Gln

                165                 170                 175

Ser Lys Asn Phe Leu Val Pro Asp Cys Glu His Ala Arg Met Lys Val
            180                 185                 190

Thr Thr Pro Cys Met Ser Ser Gly Ser Leu Trp Asp Pro Asn Ile Thr
        195                 200                 205

Val Glu Thr Leu Glu Ala His Gln Leu Arg Val Ser Phe Thr Leu Trp
    210                 215                 220

Asn Glu Ser Thr His Tyr Gln Ile Leu Leu Thr Ser Phe Pro His Met
225                 230                 235                 240

Glu Asn His Ser Cys Phe Glu His Met His His Ile Pro Ala Pro Arg
                245                 250                 255

Pro Glu Glu Phe His Gln Arg Ser Asn Val Thr Leu Thr Leu Arg Asn
            260                 265                 270

Leu Lys Gly Cys Cys Arg His Gln Val Gln Ile Gln Pro Phe Phe Ser
        275                 280                 285

Ser Cys Leu Asn Asp Cys Leu Arg His Ser Ala Thr Val Ser Cys Pro
    290                 295                 300

Glu Met Pro Asp Thr Pro Glu Pro Ile Pro Asp Tyr Met Pro Leu Trp
305                 310                 315                 320

Val Tyr Trp Phe Ile Thr Gly Ile Ser Ile Leu Leu Val Gly Ser Val
                325                 330                 335

Ile Leu Leu Ile Val Cys Met Thr Trp Arg Leu Ala Gly Pro Gly Ser
            340                 345                 350

Glu Lys Tyr Ser Asp Asp Thr Lys Tyr Thr Asp Gly Leu Pro Ala Ala
        355                 360                 365

Asp Leu Ile Pro Pro Pro Leu Lys Pro Arg Lys Val Trp Ile Ile Tyr
    370                 375                 380

Ser Ala Asp His Pro Leu Tyr Val Asp Val Val Leu Lys Phe Ala Gln
385                 390                 395                 400

Phe Leu Leu Thr Ala Cys Gly Thr Glu Val Ala Leu Asp Leu Leu Glu
                405                 410                 415

Glu Gln Ala Ile Ser Glu Ala Gly Val Met Thr Trp Val Gly Arg Gln
        420                 425                 430

Lys Gln Glu Met Val Glu Ser Asn Ser Lys Ile Ile Val Leu Cys Ser
            435                 440                 445

Arg Gly Thr Arg Ala Lys Trp Gln Ala Leu Leu Gly Arg Gly Ala Pro
    450                 455                 460

Val Arg Leu Arg Cys Asp His Gly Lys Pro Val Gly Asp Leu Phe Thr
465                 470                 475                 480

Ala Ala Met Asn Met Ile Leu Pro Asp Phe Lys Arg Pro Ala Cys Phe
                485                 490                 495

Gly Thr Tyr Val Val Cys Tyr Phe Ser Glu Val Ser Cys Asp Gly Asp
            500                 505                 510

Val Pro Asp Leu Phe Gly Ala Ala Pro Arg Tyr Pro Leu Met Asp Arg
        515                 520                 525

Phe Glu Glu Val Tyr Phe Arg Ile Gln Asp Leu Glu Met Phe Gln Pro
    530                 535                 540

Gly Arg Met His Arg Val Gly Glu Leu Ser Gly Asp Asn Tyr Leu Arg
545                 550                 555                 560

Ser Pro Gly Gly Arg Gln Leu Arg Ala Ala Leu Asp Arg Phe Arg Asp
                565                 570                 575

Trp Gln Val Arg Cys Pro Asp Trp Phe Glu Cys Glu Asn Leu Tyr Ser
            580                 585                 590

Ala Asp Asp Gln Asp Ala Pro Ser Leu Asp Glu Glu Val Phe Glu Glu
        595                 600                 605

Pro Leu Leu Pro Pro Gly Thr Gly Ile Val Lys Arg Ala Pro Leu Val
    610                 615                 620

Arg Glu Pro Gly Ser Gln Ala Cys Leu Ala Ile Asp Pro Leu Val Gly
625                 630                 635                 640

Glu Glu Gly Gly Ala Ala Val Ala Lys Leu Glu Pro His Leu Gln Pro
                645                 650                 655

Arg Gly Gln Pro Ala Pro Gln Pro Leu His Thr Leu Val Leu Ala Ala
            660                 665                 670

Glu Glu Gly Ala Leu Val Ala Ala Val Glu Pro Gly Pro Leu Ala Asp
        675                 680                 685

Gly Ala Ala Val Arg Leu Ala Leu Ala Gly Glu Gly Glu Ala Cys Pro
    690                 695                 700

Leu Leu Gly Ser Pro Gly Ala Gly Arg Asn Ser Val Leu Phe Leu Pro
705                 710                 715                 720

Val Asp Pro Glu Asp Ser Pro Leu Gly Ser Ser Thr Pro Met Ala Ser
                725                 730                 735

Pro Asp Leu Leu Pro Glu Asp Val Arg Glu His Leu Glu Gly Leu Met
            740                 745                 750

Leu Ser Leu Phe Glu Gln Ser Leu Ser Cys Gln Ala Gln Gly Gly Cys
        755                 760                 765

Ser Arg Pro Ala Met Val Leu Thr Asp Pro His Thr Pro Tyr Glu Glu
    770                 775                 780

Glu Gln Arg Gln Ser Val Gln Ser Asp Gln Gly Tyr Ile Ser Arg Ser
785                 790                 795                 800

Ser Pro Gln Pro Pro Glu Gly Leu Thr Glu Met Glu Glu Glu Glu Glu
                805                 810                 815

Glu Glu Gln Asp Pro Gly Lys Pro Ala Leu Pro Leu Ser Pro Glu Asp
            820                 825                 830

Leu Glu Ser Leu Arg Ser Leu Gln Arg Gln Leu Leu Phe Arg Gln Leu
        835                 840                 845

Gln Lys Asn Ser Gly Trp Asp Thr Met Gly Ser Glu Ser Glu Gly Pro
    850                 855                 860

Ser Ala
865

<210>  2
<211>  868
<212>  PRT
<213>  Rattus norvegicus

<400>  2

Met Ala Thr Pro Arg Arg Trp Pro Arg Val Val Pro Gly Pro Ala Leu
1               5                   10                  15

Gly Trp Leu Leu Leu Leu Leu Ser Ala Leu Phe Arg Gly Arg Ala Ser
            20                  25                  30

Leu Arg Leu Leu Asp Phe Pro Ala Leu Val Cys Ser Gln Glu Gly Leu
        35                  40                  45

Asn Cys Arg Val Glu Asn Ser Thr Cys Leu Asp Asp Ser Trp Ile Tyr
    50                  55                  60

Pro Arg Asn Leu Thr Pro Ser Ser Pro Lys Asn Ile Tyr Leu His Leu
65                  70                  75                  80

Asn Val Ser Ser Thr Gln His Gly Asp Leu Val Pro Val Leu His Val
                85                  90                  95

Glu Trp Thr Leu Gln Thr Asp Ala Ser Ile Leu Tyr Leu Glu Gly Ala
            100                 105                 110

Glu Leu Ser Ile Leu Lys Leu Asn Thr Asn Glu Gln Leu Cys Val Lys
        115                 120                 125

Phe Lys Phe Leu Ser Lys Leu Ser Asn His His Lys Arg Trp Arg Phe
    130                 135                 140

Ser Phe Ser His Phe Val Val Asp Pro Gly Gln Glu Tyr Glu Val Thr
145                 150                 155                 160

Val His His Leu Pro Lys Pro Ile Pro Asp Gly Asp Pro Asn His Lys
                165                 170                 175

Ser Lys Thr Ile Leu Val Pro Gly Cys Glu Asp Thr Glu Met Lys Met
            180                 185                 190

Thr Thr Pro Cys Val Ser Ser Gly Ser Leu Trp Asn Pro Asn Ile Thr
        195                 200                 205

Val Glu Thr Leu Asp Thr Gln His Leu Arg Val Asp Phe Thr Leu Trp
    210                 215                 220

Asn Glu Ser Thr Pro Tyr Gln Ile Leu Leu Glu Ser Phe Ser Gly Ser
225                 230                 235                 240

Glu Asn Gln Ser Cys Phe Glu Asp Ile Lys Gln Ile Ser Ala Pro Gly
                245                 250                 255

Gln Glu Glu Phe His Gln Arg Ser Asn Val Thr Phe Thr Leu Ser Lys
            260                 265                 270

Ser Gly Trp Cys Cys His His Leu Val Gln Val Gln Pro Phe Phe Asn
        275                 280                 285

Ser Cys Gln Asn Asp Cys Leu Arg His Ala Val Thr Val Pro Cys Pro
    290                 295                 300

Val Ile Ser Glu Thr Pro Val Ser Ile Thr Ala Ala Asp Tyr Ile Pro
305                 310                 315                 320

Leu Trp Val Tyr Gly Leu Ile Thr Leu Ile Ala Ile Leu Leu Val Gly
                325                 330                 335

Ser Val Ile Leu Leu Ile Ile Cys Met Thr Trp Arg Leu Ser Gly Ala
            340                 345                 350

Asp Gln Glu Lys His Gly Asp Asp Ser Lys Val Asn Gly Ile Leu Pro
        355                 360                 365

Thr Thr Asp Leu Thr Pro Pro Pro Leu Gln Pro Arg Lys Val Trp Ile
    370                 375                 380

Val Tyr Ser Ala Asp His Pro Leu Tyr Val Asp Val Val Leu Lys Phe
385                 390                 395                 400

Ala Gln Phe Leu Ile Thr Ala Cys Gly Thr Glu Val Ala Leu Asp Leu
                405                 410                 415

Leu Glu Glu Gln Thr Ile Ser Glu Met Gly Val Met Thr Trp Val Ser
            420                 425                 430

Arg Gln Lys Gln Glu Met Glu Glu Ser Gly Ser Lys Ile Ile Val Leu
        435                 440                 445

Cys Ser Arg Gly Thr Arg Ala Lys Trp Lys Ala Ile Leu Gly Trp Ala
    450                 455                 460

Glu Pro Ala Val Gln Leu Arg Cys Asp His Trp Lys Pro Ala Gly Asp
465                 470                 475                 480

Leu Phe Thr Ala Ala Met Asn Met Ile Leu Pro Asp Phe Lys Arg Pro
                485                 490                 495

Ala Cys Phe Gly Thr Tyr Ile Val Cys Tyr Phe Ser Gly Ile Cys Asn
            500                 505                 510

Glu Arg Asp Val Pro Asp Leu Phe Asn Ile Thr Ser Arg Tyr Pro Leu

515                 520                 525

Met Asp Lys Phe Glu Glu Val Tyr Phe Arg Ile Gln Asp Leu Glu Met
    530                 535                 540

Phe Glu Pro Gly Arg Met His His Val Lys Glu Leu Thr Gly Glu Asn
545                 550                 555                 560

Tyr Leu Gln Ser Pro Ser Gly Arg Gln Leu Lys Glu Ala Val Val Arg
                565                 570                 575

Phe Gln Glu Trp Gln Thr Arg Tyr Pro Asp Trp Phe Glu Arg Glu Asn
            580                 585                 590

Leu Cys Leu Ala Gly Asp Gln Asp Leu Pro Ser Leu Asp Glu Glu Val
        595                 600                 605

Phe Glu Asp Pro Leu Leu Pro Pro Gly Gly Arg Ile Val Lys Gln Gln
    610                 615                 620

Pro Leu Val Arg Glu Leu Pro Ser Glu Gly Cys Leu Val Val Asp Val
625                 630                 635                 640

Cys Ala Arg Glu Glu Glu Ser Gly Val Val Lys Leu Glu Pro Gln Leu
                645                 650                 655

Trp Pro Gln Arg Gln Leu Met Ala Gln Thr Leu Gln Thr Leu Val Leu
            660                 665                 670

Pro Thr Glu Gln Val Pro Ala Ala His Val Val Glu Pro Val Arg Leu
        675                 680                 685

Leu Asp Gly Thr Gly Ala Ala Thr Gln Leu Ala Ile Ala Glu Asp Gly
    690                 695                 700

Glu Ala Cys Pro Leu Leu Gly Val Arg Arg Asn Ser Ile Leu Cys Leu
705                 710                 715                 720

Pro Met Asp Ser Asp Asp Ser Pro Leu Cys Ser Thr Pro Met Met Ser
                725                 730                 735

Pro Asp His Leu Gln Gly Asp Ala Arg Glu Gln Leu Glu Ser Leu Met
            740                 745                 750

Leu Ser Val Leu Gln His Ser Leu Ser Ala Gln Ala Gln Glu Gly Trp
        755                 760                 765

Pro Arg Pro Glu Val Val Leu Lys Asp Cys Met Pro Ser Glu Glu Glu
    770                 775                 780

Gln Arg Gln Ser Val Gln Ser Asp Gln Gly Tyr Ile Ser Arg Ser Ser
785                 790                 795                 800

Pro Gln Pro Pro Asp Trp Leu Thr Glu Glu Glu Glu Leu Glu Leu Gly
                805                 810                 815

Glu Ser Gly Glu Ser Leu Ser Pro Glu Glu Leu Arg Ser Leu Arg Ser
            820                 825                 830

Val Gln Arg Arg Leu Phe Phe Trp Glu Leu Glu Lys Asn Pro Gly Trp
        835                 840                 845

Asp Ser Arg Gly Gln Glu Gln Lys Ile Ser Leu Pro Pro Gly Pro Pro
    850                 855                 860

Asn Pro Ala Thr
865

<210>  3
<211>  864
<212>  PRT
<213>  Mus musculus

<400>  3
   Met Ala Ile Arg Arg Cys Trp Pro Arg Val Val Pro Gly Pro Ala Leu
   1               5                   10                  15
   Gly Trp Leu Leu Leu Leu Leu Asn Val Leu Ala Pro Gly Arg Ala Ser
               20                  25                  30
   Pro Arg Leu Leu Asp Phe Pro Ala Pro Val Cys Ala Gln Glu Gly Leu
           35                  40                  45
   Ser Cys Arg Val Lys Asn Ser Thr Cys Leu Asp Asp Ser Trp Ile His
       50                  55                  60
   Pro Lys Asn Leu Thr Pro Ser Ser Pro Lys Asn Ile Tyr Ile Asn Leu
   65                  70                  75                  80
   Ser Val Ser Ser Thr Gln His Gly Glu Leu Val Pro Val Leu His Val
                   85                  90                  95
   Glu Trp Thr Leu Gln Thr Asp Ala Ser Ile Leu Tyr Leu Glu Gly Ala
               100                 105                 110
   Glu Leu Ser Val Leu Gln Leu Asn Thr Asn Glu Arg Leu Cys Val Lys
           115                 120                 125
   Phe Gln Phe Leu Ser Met Leu Gln His His Arg Lys Arg Trp Arg Phe
       130                 135                 140
   Ser Phe Ser His Phe Val Val Asp Pro Gly Gln Glu Tyr Glu Val Thr
   145                 150                 155                 160
   Val His His Leu Pro Lys Pro Ile Pro Asp Gly Asp Pro Asn His Lys
                   165                 170                 175
   Ser Lys Ile Ile Phe Val Pro Asp Cys Glu Asp Ser Lys Met Lys Met
               180                 185                 190
   Thr Thr Ser Cys Val Ser Ser Gly Ser Leu Trp Asp Pro Asn Ile Thr
           195                 200                 205
   Val Glu Thr Leu Asp Thr Gln His Leu Arg Val Asp Phe Thr Leu Trp
       210                 215                 220
   Asn Glu Ser Thr Pro Tyr Gln Val Leu Leu Glu Ser Phe Ser Asp Ser
   225                 230                 235                 240
   Glu Asn His Ser Cys Phe Asp Val Val Lys Gln Ile Phe Ala Pro Arg
                   245                 250                 255
   Gln Glu Glu Phe His Gln Arg Ala Asn Val Thr Phe Thr Leu Ser Lys
               260                 265                 270
   Phe His Trp Cys Cys His His His Val Gln Val Gln Pro Phe Phe Ser
           275                 280                 285
   Ser Cys Leu Asn Asp Cys Leu Arg His Ala Val Thr Val Pro Cys Pro
       290                 295                 300
   Val Ile Ser Asn Thr Thr Val Pro Lys Pro Val Ala Asp Tyr Ile Pro
   305                 310                 315                 320
   Leu Trp Val Tyr Gly Leu Ile Thr Leu Ile Ala Ile Leu Leu Val Gly
                   325                 330                 335
   Ser Val Ile Val Leu Ile Ile Cys Met Thr Trp Arg Leu Ser Gly Ala
               340                 345                 350
   Asp Gln Glu Lys His Gly Asp Asp Ser Lys Ile Asn Gly Ile Leu Pro
           355                 360                 365
   Val Ala Asp Leu Thr Pro Pro Pro Leu Arg Pro Arg Lys Val Trp Ile
       370                 375                 380
   Val Tyr Ser Ala Asp His Pro Leu Tyr Val Glu Val Val Leu Lys Phe
   385                 390                 395                 400
   Ala Gln Phe Leu Ile Thr Ala Cys Gly Thr Glu Val Ala Leu Asp Leu
                   405                 410                 415
   Leu Glu Glu Gln Val Ile Ser Glu Val Gly Val Met Thr Trp Val Ser
               420                 425                 430
   Arg Gln Lys Gln Glu Met Val Glu Ser Asn Ser Lys Ile Ile Ile Leu
           435                 440                 445
   Cys Ser Arg Gly Thr Gln Ala Lys Trp Lys Ala Ile Leu Gly Trp Ala
       450                 455                 460
   Glu Pro Ala Val Gln Leu Arg Cys Asp His Trp Lys Pro Ala Gly Asp
   465                 470                 475                 480
   Leu Phe Thr Ala Ala Met Asn Met Ile Leu Pro Asp Phe Lys Arg Pro
                   485                 490                 495
   Ala Cys Phe Gly Thr Tyr Val Val Cys Tyr Phe Ser Gly Ile Cys Ser
               500                 505                 510
   Glu Arg Asp Val Pro Asp Leu Phe Asn Ile Thr Ser Arg Tyr Pro Leu
           515                 520                 525
   Met Asp Arg Phe Glu Glu Val Tyr Phe Arg Ile Gln Asp Leu Glu Met
       530                 535                 540
   Phe Glu Pro Gly Arg Met His His Val Arg Glu Leu Thr Gly Asp Asn
   545                 550                 555                 560
   Tyr Leu Gln Ser Pro Ser Gly Arg Gln Leu Lys Glu Ala Val Leu Arg
                   565                 570                 575
   Phe Gln Glu Trp Gln Thr Gln Cys Pro Asp Trp Phe Glu Arg Glu Asn
               580                 585                 590
   Leu Cys Leu Ala Asp Gly Gln Asp Leu Pro Ser Leu Asp Glu Glu Val
           595                 600                 605
   Phe Glu Asp Pro Leu Leu Pro Pro Gly Gly Gly Ile Val Lys Gln Gln
       610                 615                 620
   Pro Leu Val Arg Glu Leu Pro Ser Asp Gly Cys Leu Val Val Asp Val
   625                 630                 635                 640
   Cys Val Ser Glu Glu Glu Ser Arg Met Ala Lys Leu Asp Pro Gln Leu
                   645                 650                 655
   Trp Pro Gln Arg Glu Leu Val Ala His Thr Leu Gln Ser Met Val Leu
               660                 665                 670
   Pro Ala Glu Gln Val Pro Ala Ala His Val Val Glu Pro Leu His Leu
           675                 680                 685
   Pro Asp Gly Ser Gly Ala Ala Ala Gln Leu Pro Met Thr Glu Asp Ser
       690                 695                 700
   Glu Ala Cys Pro Leu Leu Gly Val Gln Arg Asn Ser Ile Leu Cys Leu
   705                 710                 715                 720
   Pro Val Asp Ser Asp Asp Leu Pro Leu Cys Ser Thr Pro Met Met Ser
                   725                 730                 735
   Pro Asp His Leu Gln Gly Asp Ala Arg Glu Gln Leu Glu Ser Leu Met
               740                 745                 750
   Leu Ser Val Leu Gln Gln Ser Leu Ser Gly Gln Pro Leu Glu Ser Trp
           755                 760                 765
   Pro Arg Pro Glu Val Val Leu Glu Gly Cys Thr Pro Ser Glu Glu Glu
       770                 775                 780
   Gln Arg Gln Ser Val Gln Ser Asp Gln Gly Tyr Ile Ser Arg Ser Ser
   785                 790                 795                 800
   Pro Gln Pro Pro Glu Trp Leu Thr Glu Glu Glu Glu Leu Glu Leu Gly
                   805                 810                 815
   Glu Pro Val Glu Ser Leu Ser Pro Glu Glu Leu Arg Ser Leu Arg Lys
               820                 825                 830
   Leu Gln Arg Gln Leu Phe Phe Trp Glu Leu Glu Lys Asn Pro Gly Trp
           835                 840                 845
   Asn Ser Leu Glu Pro Arg Arg Pro Thr Pro Glu Glu Gln Asn Pro Ser
       850                 855                 860

## Description

### [Technical Field]

The present invention relates to an interleukin-17A(IL-17A) activity inhibitor which is a low-molecular-weight compound having an action of inhibiting binding of IL-17A to interleukin-17 receptor A (IL-17RA). In addition, the present invention relates to a medicament for treating or prophylaxis of symptoms or diseases in an intervertebral disc tissue such as intervertebral disc degeneration, and inflammatory skin diseases such as psoriasis, the medicament containing the IL-17A activity inhibitor as an active ingredient.

### [Background Art]

Interleukin-17A (IL-17A) is a cytokine produced by a T helper 17 (Th17) cell which is one of the T cell subsets. The produced IL-17A regulates expression of various genes by binding to interleukin-17 receptors (IL-17R) present in various cells and causing JAK-STAT intracellular signal transduction. An abnormal production of IL-17 or an abnormality of JAK-STAT intracellular signal transduction is deeply related to an inflammatory reaction of tissues, an autoimmune disease, formation of a tumor, and the like. Recently, it has been reported that IL-17 increases along with IL-4, IL-6, IL-12, IFN-γ, and the like in degenerated or herniated intervertebral disc nucleus pulposus cells (Non-Patent Documents 1 and 2).

IL-17A is a homodimer (A chain and B chain) protein. Meanwhile, IL-17R is a protein composed of two subunits, interleukin-17 receptor A (IL-17RA), and interleukin-17 receptor C (IL-17RC). In addition, IL-17RA is composed of two fibronectin type III domains (D1 and D2). A crystal structure of a complex of IL-17A and an extracellular domain of IL-17RA is specified. Three of the main binding sites (pockets) with IL-17A, that is, a site formed by Ans89 to Glu92 and Asp121 to Glu125 in the D1 domain, Ser257 to Asp262 in the D2 domain, and Thr163 to Ser167 of a helix linker linking the D1 and D2 domains to each other is included in the two domains of IL-17RA.

Research and development only on a biological preparation containing a so-called neutralizing antibody as a main component, such as an anti-IL-17A antibody inhibiting binding with IL-17RA by targeting IL-17A, or reversely, an anti-IL-17RA antibody inhibiting binding with IL-17A by targeting IL-17RA, as an IL-17A activity inhibitor, have been conducted.

For example, in Patent Document 1 (Published Japanese Translation No. 2016-508508 of PCT International Publication, Novartis AG), it is described that an antibody (anti-IL-17A antibody) includes CDR having a specific amino acid sequence, and binds specifically to homodimer IL-17A and heterodimer IL-17AF of a human, a mouse, or the like and does not bind specifically to homodimer IL-17F, the antibody being capable of inhibiting or blocking binding between IL-17A and a receptor thereof though binding to IL-17A, and reducing or neutralizing IL-17A activity. In addition, in Patent Document 1, it is also described that such an antibody can be used for treating an autoimmune and inflammatory disorder, such as arthritis, rheumatoid arthritis, psoriasis, chronic obstructive pulmonary diseases, systemic lupus erythematosus (SLE), lupus nephritis, asthma, multiple sclerosis, or cystic fibrosis.

In Patent Document 2 (Published Japanese Translation No. 2010-505416 of PCT International Publication, Amgen Inc.), it is described that an antibody (anti-IL-17RA antibody) including CDR having a specific amino acid sequence and inhibiting binding of IL-17A and/or IL-17F of a human or the like to IL-17RA of a human or the like, and a pharmaceutical composition for treating inflammation (for example, arthritis), asthma, autoimmune diseases, and the like, the pharmaceutical composition including the antibody. In addition, in Patent Document 2, it is also described that a method including administering the IL-17RA to a patient to inhibit production of at least one of cytokines, chemokines, matrix metalloproteinases, or other molecules associated with IL-17RA activation (for example, IL-6, IL-8, CXCL1, CXCL2, GM-CSF, G-CSF, M-CSF, IL-1β, TNFα, RANK-L, LIF, PGE2, IL-12, MMP3, MMP9, GROα, NO, and C-telopeptides). In Patent Document 3 (Published Japanese Translation No. 2017-511316 of PCT International Publication, Kirin Amgen Inc.), it is described that a method for treating nail or scalp psoriasis by using an antibody (preferably, an antibody including CDR having a specific amino acid sequence) that binds specifically to IL-17RA and has an antagonistic activity.

As a psoriasis medicine containing antibodies described in Patent Documents 1 to 3, a subcutaneous injection containing anti-IL-17A antibody "Secukinumab" (trade name: "COSENTYX", Novartis Pharmaceuticals Corporation) as an active ingredient, and a subcutaneous injection containing anti-IL-17RA antibody "Brodalumab" (trade name: "LUMICEF", Kyowa Kirin Co., Ltd.) as an active ingredient have been already manufactured and sold in Japan.

Meanwhile, in Non-Patent Document 3, it is disclosed that a "pocket" in the extracellular domain of IL-17RA, that is, a region composed of Asn89, Thr90, Asn91, Glu92, Asp121, Pro122, Asp123, Gln124, and Glu125 in a D1 domain, Ser257, Ser258, Cys259, Leu260, Asn261, and Asp262 in a D2 domain, and Thr163, Pro164, Cys165, Met166, and Ser167 of a helix linker is determined as a target site of the drug inhibiting binding of IL-17A, and a cyanidin compound (A18) represented by the following structural formula interacts with Asp121, Gln124, Ser168, and Asp262 in the pocket, such that binding of IL-17Ato IL-17RA can be competitively inhibited. In addition, it is described that a significant reduction in inhibitory activity of the compound A18 with respect to mouse IL-17RA in which Asp262 conserved in human IL-17RA is mutated (for example, substituted with Ala) shows that the amino acid residue is important for binding of IL-17A to IL-17RA, and, in particular, a hydrogen bond between a hydroxyl group (-OH) at the 3'-position of the B ring and Gln124, a hydrogen bond between a hydroxyl group at the 3'-position of the C ring and Asp262, and although the influence is slightly smaller than those of these bonds, a hydrogen bond between a hydroxyl group at the 5'-position of the C ring and Leu264 greatly affect IL-17RA inhibitory activity, such that the IL-17RA inhibitory activity is almost eliminated in a compound in which the C ring is modified from a 6-membered ring to a 5-membered ring.

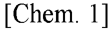

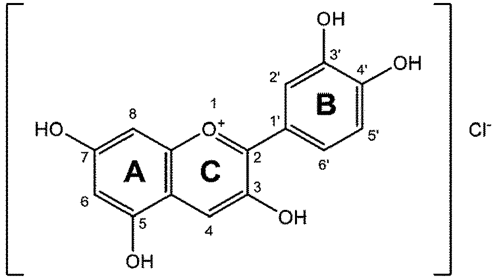

In addition, in Non-Patent Document 3 (Liu et al), it is disclosed that by using the compound A18, expression of genes induced by IL-17A in human or mouse cells can be inhibited, IL-17A-dependent skin hyperplasia in a mouse can be suppressed, Th17 cell-dependent inflammation in a mouse can be suppressed, and airway inflammation in a mouse model with steroid-resistant severe asthma in a mouse can be alleviated.

### [Prior Art Documents]

### [Patent Documents]

Patent Document 1: Published Japanese Translation No. 2016-508508 of PCT International Publication
Patent Document 2: Published Japanese Translation No. 2010-505416 of PCT International Publication
Patent Document 3: Published Japanese Translation No. 2017-511316 of PCT International Publication

Non-Patent Document 1: Aggarwal, S. et al., The Journal of biological chemistry 278, 1910-1914 (2003)
Non-Patent Document 2: Park, H. et al., Nature immunology 6, 1133-1141 (2005)
Non-Patent Document 3: Liu et al., Sci Signal. 10(647), eaaf8823 (2017)

### [Summary of the Invention]

### [Problem to be Solved by the Invention]

In the case of the medicament (biological preparation) containing the antibodies (neutralizing antibodies) described in Patent Documents 1 to 3 as an active ingredient, serious side effects may occur, and a price thereof is high. Therefore, if a low-molecular-weight compound that may solve the above problems can be used as the IL-17 activity inhibitor, its value becomes high.

Meanwhile, in Non-Patent Document 3, it is described that a specific low-molecular-weight compound (cyanidin) can be used as an IL-17A activity inhibitor, but there was room for improvement in IL-17A activity inhibiting ability.

In an aspect, an object of the present invention is to provide a low-molecular-weight compound (IL-17A activity inhibitor) having an excellent IL-17A activity inhibiting ability as compared to that in the related art.

In addition, a relationship between IL-17A and degeneration of an intervertebral disc is shown in the above document, but details of a specific role of IL-17A in the degeneration of the intervertebral disc have not been clarified. In the conventional studies, an intervertebral disc nucleus pulposus cell is cultured in an atmosphere of a normal oxygen concentration which is significantly different from an actual low oxygen environment of an intervertebral disc tissue in vivo, and it was unclear when an intervertebral disc tissue is cultured in a low oxygen environment in which a microenvironment of the intervertebral disc tissue is mimicked, what kind of influence is expected by inhibitory activity of IL-17A in the intervertebral disc nucleus pulposus cell, and in particular, whether intervertebral disc degeneration progression or production of substances causing pain can be suppressed.

Therefore, in another aspect, an object of the present invention is to provide a novel use of a low-molecular-weight compound (IL-17A activity inhibitor) having IL-17A activity inhibiting ability for a treatment or prophylaxis of intervertebral disc degeneration by clarifying details of a mechanism of involvement of IL-17A in intervertebral disc degeneration.

### [Means for Solving the Problems]

In order to find an IL-17A activity inhibitory candidate compound that can solve the above problems, the present inventors conducted in silico analysis in the following three stages. First, a region on IL-17RA that interacts with IL-17A (in the present specification, referred to as an "interaction region") was specified by using complex crystal structure information (PDB ID: 4HSA) on IL-17A and a receptor thereof (IL-17RA), and a structural chemical property for a compound group that can be bound in the interaction region and inhibit binding of IL-17A was obtained by software "DRFF" (Horio K, Muta H, Goto J, Hirayama N (2007) A simple method to improve the odds in finding 'lead-like' compounds from chemical libraries. Chem. Pharm. Bull., 55, 980-984.). The interaction region clarified in the present study is a space surrounded by 28 amino acid residues, and the space is partially overlapped with the pocket composed of 20 amino acid residues mentioned in Non-Patent Document 3, but is a wider space. Second, 5,500 compounds that most satisfy the structural chemical property obtained in the previous study were searched from an in-house compound database including information of about 6 million kinds of commercially available compounds. Third, the interaction between the interaction region and 5,500 compounds was precisely determined by docking software "ASEDock" (Goto, J.; Kataoka, R.; Muta, H.; Hirayama, N. (2008) ASEDock-docking based on alpha spheres and excluded volumes. J. Chem. Inf. Model, 48, 583-590.), and a candidate compound to be used for biological evaluation was screened based on interaction energy between the compounds and IL-17RA(GBVI/WSA_dG. Corbeil, C. R.; Williams, C. I.; Labute, P. (2012) Variability in docking success rates due to dataset preparation. J. Comput.-Aided Mol. Des., 26, 775-786.).

Meanwhile, the present inventors have, for the first time, found that expression levels of several genes (factors) that promote inflammation or nucleus pulposus degeneration in an intervertebral disc are increased by culturing nucleus pulposus cells (NP cells) collected from a rat intervertebral disc under a 1% low oxygen condition similar to a growing environment of an intervertebral disc in vivo, and adding IL-17A thereto. In addition, the present inventors added a candidate compound together with IL-17A to the nucleus pulposus cells cultured under the low oxygen condition as described above in order to test whether some compounds with high IL-17A activity inhibiting ability (GBVI/WSA_dG was a negative number, which was low) in the above in silico analysis actually have the IL-17A activity inhibiting ability in human or rat nucleus pulposus cells. As a result, it was found that by adding the candidate compound according to the present invention, the expression levels of the specific genes were suppressed, for example, an expression level of COX-2 known as a pain-inducing factor was significantly suppressed as compared to that of the compound in Non-Patent Document 3, and it was demonstrated that the candidate compound according to the present invention has excellent IL-17A activity inhibiting ability as compared to that of the compound in Non-Patent Document 3.

The present inventors have found through these studies that it can be presumed that the candidate compound in in silico which was shown to interact with the amino acid residues constituting the interaction region specified as described above with a predetermined intensity has IL-17A activity inhibiting ability by binding to IL-17RA competitively with IL-17A, similarly to compounds used in examples of the present invention and other compounds, thereby completing the present invention.

The compound disclosed in Non-Patent Document 3 was found by the following procedure. First, a site (pocket) on IL-17RA to which an inhibitor can bind was determined based on a partial structure of a crystal structure of IL-17A (ligand) that interacts with IL-17RA. Second, molecules most appropriately binding to the pocket were searched from the NCI compound library including about 90,000 compounds by a docking method. On the contrary, in the approach of the present invention, a region on IL-17RA that can inhibit the interaction with IL-17A was specified in advance based on a three-dimensional structure of only IL-17RA (receptor). The region that can be specified by this method is significantly wider than the region specified in Non-Patent Document 3. In addition, in this region, a region that is not involved in a so-called receptor-ligand binding but inhibits the interaction between a ligand and a receptor by binging of the low-molecular-weight compound is included. That is, a compound having a structure completely different from that of the compound binding to the pocket specified in Non-Patent Document 3 can strongly bind to the region as an inhibitor. It can be said that the compound of the present invention was resulted from searching for a compound having a strong binding force to the interaction region. It was presumed that the compound of the present invention has further excellent IL-17A activity inhibiting ability by further stable interaction through covering of a wider portion of the interaction region due to its molecular size being larger than that of the compound of Non-Patent Document 3. For example, a representative compound of the present invention interacts with amino acids such as Cys154, Lys160, and Ser170 of IL-17RA that are not targeted in Non-Patent Document 3, and in particular, Cys154 which highly common in the compound of the present invention, by a hydrogen bonding, a CH-π interaction, or the like. It is considered that the compound of the present invention has excellent inhibitory activity with respect to IL-17A as described above by binding to IL-17RA so that the compound interacts with such amino acid residues.

That is, for example, the following inventions are provided through the present invention.
[Item 1]
An IL-17A activity inhibitor containing:
a compound having an action of inhibiting binding of interleukin-17A (IL-17A) to human or non-human animal interleukin-17 receptor A (IL-17RA), or a pharmaceutically acceptable salt, solvate, or prodrug thereof,
the compound being capable of binding to IL-17RA through a non-covalent interaction including a van der Waals force acting between the compound and at least 13 amino acid residues among 28 amino acid residues of Phe60, Gln87, Asp121, Pro122, Asp123, Gln124, Asp153, Cys154, Glu155, Lys160, Pro164, Cys165, Ser167, Ser168, Gly169, Ser170, Leu171, Trp172, Asp173, Pro174, Pro254, Phe256, Ser258, Cys259, Asp262, Cys263, Leu264, and His266 that are contained in an extracellular domain of human IL-17RA in a space surrounded by the 28 amino acid residues, or being capable of binding to IL-17RA through a non-covalent interaction including a van der Waals force acting between the compound and at least 13 amino acid residues among amino acid residues (where homology between the amino acid residues is 80% or more) corresponding to the 28 amino acid residues and contained in an extracellular domain of non-human animal IL-17RA in the space surrounded by the amino acid residues corresponding to the 28 amino acid residues.

[Item 2]
The IL-17A activity inhibitor according to Item 1, wherein the non-covalent interaction includes at least one intermolecular interaction selected from the group consisting of an ionic bonding, a hydrogen bonding, a CH-π interaction, a cation-π interaction, and a hydrophobic interaction, the intermolecular interaction acting between the compound and at least one amino acid residue selected from the group consisting of Asp121, Pro122, Asp123, Gln124, Asp153, Cys154, Glu155, Lys160, Ser168, Ser170, Ser258, Asp262, Leu264, and His266.
[Item 3]
The IL-17A activity inhibitor according to Item 2, wherein the intermolecular interaction includes at least a hydrogen bonding or CH-π interaction with Cys154.
[Item 4]
The IL-17A activity inhibitor according to Item 2 or 3, wherein the intermolecular interaction optionally includes at least one selected from the group consisting of a hydrogen bonding with Asp121, a CH-π interaction or hydrogen bonding with Pro 122, a CH-π interaction or hydrogen bonding with Asp 123, an ionic bonding, hydrogen bonding, or CH-π interaction with Lys160, and a CH-π interaction with Ser170.
[Item 5]
An IL-17A activity inhibitor containing a compound represented by General Formula (I) (hereinafter, referred to as a "compound (I)"), or a pharmaceutically acceptable salt, solvate, or prodrug thereof,
[Chem. 2]

A-L¹-B-L²-C-L³-D (I)

in General Formula (I),
A represents (A1) a C₃₋₁₀ cycloalkyl group which is optionally substituted, (A2) a C₃₋₁₀ cycloalkenyl group which is optionally substituted, (A3) a 6- to 14-membered aromatic hydrocarbon cyclic group (aryl group) which is optionally substituted, (A4) a 5- to 14-membered aromatic heterocyclic group which is optionally substituted, (A5) a 3- to 14-membered non-aromatic heterocyclic group which is optionally substituted, or (A6) a C₄₋₆ alkyl group which is optionally substituted,
L¹ represents (L¹1) a single bond, (L¹2) a C₁₋₃ alkylene group, which is optionally linked to a divalent group (amide bond) derived from a carbamoyl group and/or is optionally linked to an ether bond or a thioether bond, (L¹3) a divalent group (amide bond) derived from a carbamoyl group, which is optionally linked to a divalent group derived from an amino group, (L¹4) a sulfonyl group, or (L¹5) a C₁₋₃ alkenylene group (a carbon-carbon double bond is optionally formed with a carbon atom of B or C adjacent to L²),
B represents (B1) a divalent group (amide bond) derived from a carbamoyl group, which is optionally substituted and/or is optionally linked to a divalent group derived from a C₁₋₃ alkyl-carbonyl group, (B2) a divalent group derived from a 5- to 14-membered aromatic heterocyclic ring, which is optionally substituted, (B3) a divalent group derived from a 3- to 14-membered non-aromatic heterocyclic ring, which is optionally substituted, (B4) a C₃₋₁₀ cycloalkyl group which is optionally substituted, (B5) a C₃₋₁₀ cycloalkenyl group which is optionally substituted, (B6) a 6- to 14-membered aromatic hydrocarbon cyclic group (aryl group) which is optionally substituted, (B7) an ester bond or a thioester bond, or (B8) a keto group or a thioketo group,
L² represents (L²1) a single bond, (L²2) a C₁₋₆ alkylene group, or (L²3) a C₁₋₃ alkenylene group (a carbon-carbon double bond is optionally formed with a carbon atom of B or C adjacent to L²),
C represents (C1) a divalent group (amide bond) derived from a carbamoyl group, which is optionally N-substituted, (C2) a divalent group derived from a 5- to 14-membered aromatic heterocyclic ring, which is optionally substituted, (C3) a divalent group derived from a 3- to 14-membered non-aromatic heterocyclic ring, which is optionally substituted, (C4) a C₃₋₁₀ cycloalkyl group which is optionally substituted, (C5) a C₃₋₁₀ cycloalkenyl group which is optionally substituted, (C6) a 6- to 14-membered aromatic hydrocarbon cyclic group (aryl group) which is optionally substituted, or (C7) an ester bond or a thioester bond,
L³ represents (L³1) a single bond, (L³2) a C₁₋₃ alkylene group, which is optionally linked to a divalent group (amide bond) derived from a carbamoyl group and/or a divalent group derived from an imino group and/or is optionally substituted, (L³3) an ether bond or a thioether bond which is optionally linked to a C₁₋₃ alkenylene group, or (L³4) a divalent group (amide bond) derived from a carbamoyl group, which is optionally linked to a divalent group derived from an amino group, and
D represents (D1) a C₃₋₁₀ cycloalkyl group which is optionally substituted, (D2) a C₃₋₁₀ cycloalkenyl group which is optionally substituted, (D3) a 6- to 14-membered aromatic hydrocarbon cyclic group (aryl group) which is optionally substituted, (D4) a 5- to 14-membered aromatic heterocyclic group which is optionally substituted, (D5) a 3- to 14-membered non-aromatic heterocyclic group which is optionally substituted, or (D6) a C₁₋₃ alkyl group which is optionally substituted.

[Item 6]
The IL-17A activity inhibitor according to Item 5, wherein the requirements according to any one of Items 1 to 4 are further satisfied.
[Item 7]
The IL-17A activity inhibitor according to Item 5 or 6, wherein the compound (I) has, as a site at which the hydrogen bonding or CH-π interaction with Cys154 is generated, at least one of:
the site A which is (A6) having a group serving as a donor or an acceptor of a hydrogen atom;
the site B which is (B1) or (B3) having a group serving as a donor or an acceptor of a hydrogen atom;
the site C which is (C1), (C2), (C3), (C6), or (C7) having a group serving as a donor or an acceptor of a hydrogen atom;
the site L¹ which is (L¹2) or (L¹4) having a group serving as a donor or an acceptor of a hydrogen atom, optionally as a substituent;
the site L² which is (L²2) having a group serving as a donor or an acceptor of a hydrogen atom, optionally as a substituent; and
the site C which is (C2) or (C6) having a π electron.

[Item 8]
The IL-17A activity inhibitor according to Item 5 or 6, wherein the compound (I) has, as a site at which the hydrogen bonding with Asp121 is generated, at least one site A which is (A3), (A4), or (A6) or at least one site L¹ which is (L¹2).
[Item 9]
The IL-17A activity inhibitor according to Item 5 or 6, wherein the compound (I) has, as a site at which the CH-π interaction or hydrogen bonding with Pro122 is generated, at least one site A which is (A4) or (A5) or at least one site B which is (B3) or (B5).
[Item 10]
The IL-17A activity inhibitor according to Item 5 or 6, wherein the compound (I) has, as a site at which the CH-π interaction or hydrogen bonding with Asp 123 is generated, at least one site A which is (A5) or at least one site C which is (C6) or (C8).
[Item 11]
The IL-17A activity inhibitor according to Item 5 or 6, wherein the compound (I) has, as a site at which the ionic bonding, hydrogen bonding, or a cation-π interaction with Lys160 is generated, at least one site D which is (D1), (D3), or (D5).
[Item 12]
The IL-17A activity inhibitor according to Item 5 or 6, wherein the compound (I) has, as a site at which the CH-π interaction with Ser170 is generated, at least one site D which is (D3) or (D5).
[Item 13]
The IL-17A activity inhibitor according to any one of Items 5 to 12, wherein the compound (I) is any one of compounds represented by the following Structural Formulas (1) to (36), respectively, (hereinafter, referred to as "compounds (1) to (36)") or derivatives thereof.

**[Table 1-1]**

| No. | Structural Formulas |
|---|---|
| (1) | 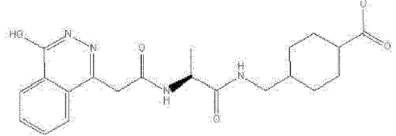 |
| (2) | 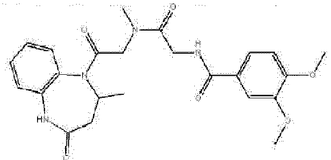 |
| (3) | 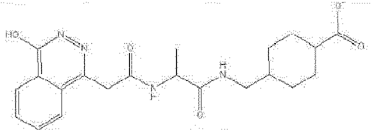 |
| (4) | 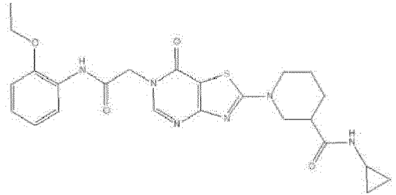 |
| (5) | 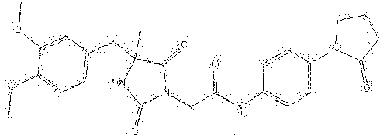 |
| (6) | 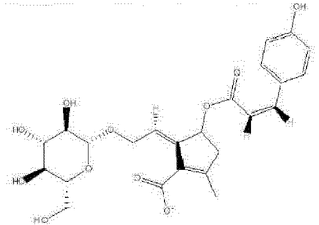 |

**[Table 1-2]**

| | |
|---|---|
| (7) | 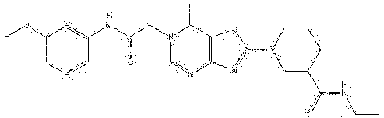 |
| (8) | 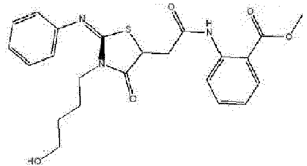 |
| (9) | 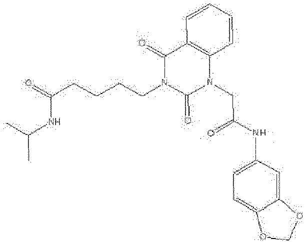 |
| (10) | 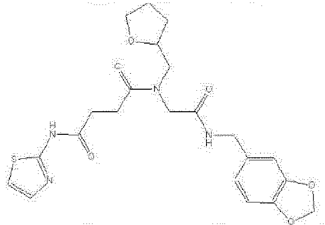 |
| (11) | 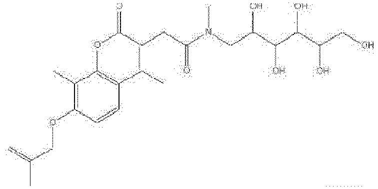 |

**[Table 1-3]**

| | |
|---|---|
| (12) | 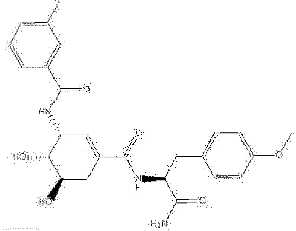 |
| (13) | 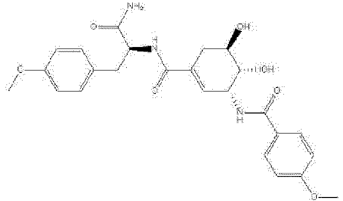 |
| (14) | 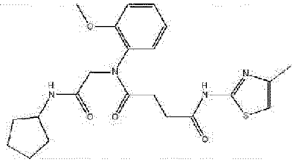 |
| (15) | 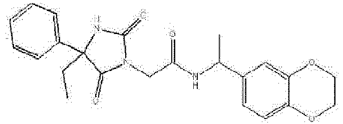 |
| (16) | 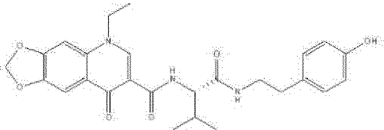 |
| (17) | 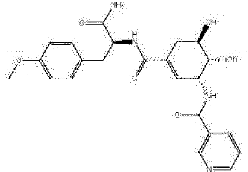 |

**[Table 1-4]**

| | |
|---|---|
| (18) | 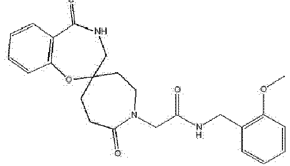 |
| (19) | 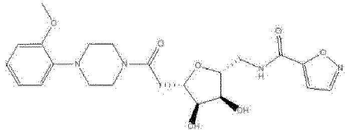 |
| (20) | 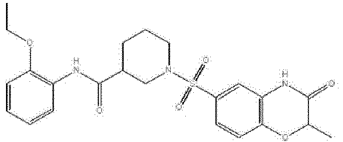 |
| (21) | 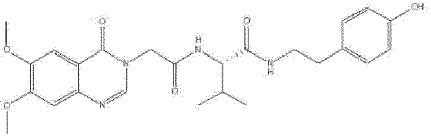 |
| (22) | 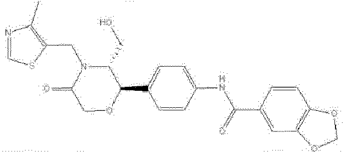 |
| (23) | 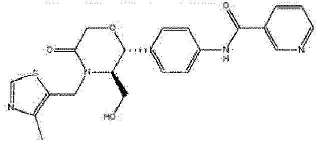 |
| (24) | 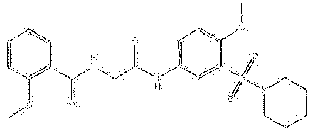 |

**[Table 1-5]**

| | |
|---|---|
| (25) | 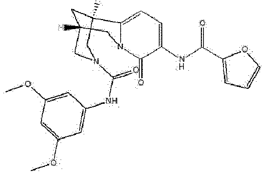 |
| (26) | 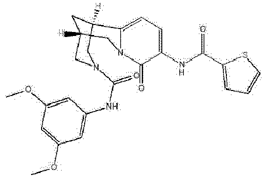 |
| (27) | 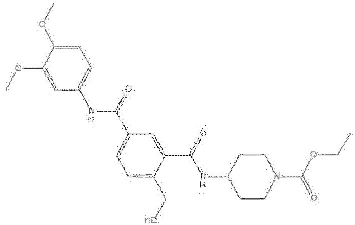 |
| (28) | 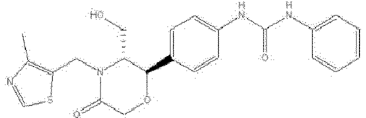 |
| (29) | 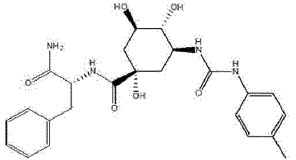 |
| (30) | 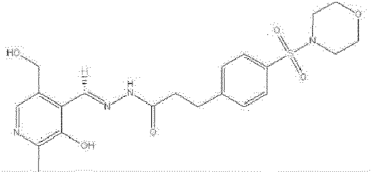 |

**[Table 1-6]**

| | |
|---|---|
| (31) | 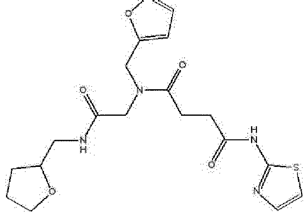 |
| (32) | 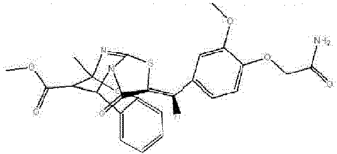 |
| (33) | 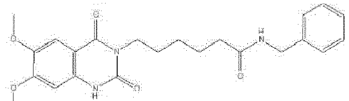 |
| (34) | 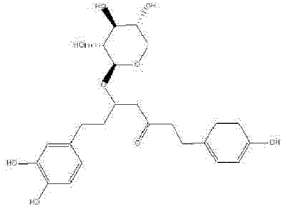 |
| (35) | 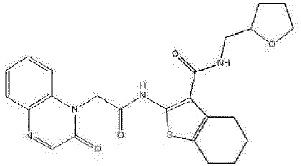 |
| (36) | 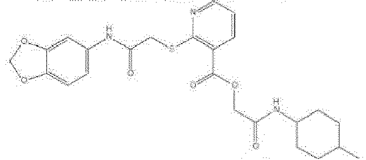 |

[Item 14]
The IL-17A activity inhibitor according to Item 13, wherein the compound (I) is the compound (1) or the derivative thereof, the compound (I) being obtained by modifying an original compound (1) so that at least one property selected from the group consisting of [X], [Y], and [Z] below is satisfied:
[X] a total van der Waals force between the compound (I) and Asp121, Pro122, Gln124, Cys154, Glu155, Lys160, Pro164, Ser168, Gly169, Ser170, Ser258, Cys259, Asp262, Cys263, and Leu264 is increased as compared with the compound (1);
[Y] the compound (I) has a site at which at least one of the CH-π interaction with Pro122, the hydrogen bonding with Cys154, and the ionic bonding with Lys160 is increased, or a site at which at least one non-covalent interaction different from the CH-π interaction with Pro122, the hydrogen bonding with Cys154, and the ionic bonding with Lys160 other than the van der Waals force is generated between the compound (I) and at least one amino acid residue selected from the group consisting of Asp121, Pro122, Gln124, Cys154, Glu155, Lys160, Pro164, Ser168, Gly169, Ser170, Ser258, Cys259, Asp262, Cys263, and Leu264, the site being included in the compound (1); and
[Z] the compound (I) has a site at which exposure of at least one amino acid residue selected from the group consisting of Asp121, Pro122, Gln124, Cys154, Glu155, Lys160, Pro164, Ser168, Gly169, Ser170, Ser258, Cys259, Asp262, Cys263, and Leu264 to a solvent is reduced as compared with the compound (1).

[Item 15]
The IL-17A activity inhibitor according to Item 13, wherein the compound (I) is the compound (2) or the derivative thereof, the compound (I) being obtained by modifying an original compound (2) so that at least one property selected from the group consisting of [X], [Y], and [Z] below is satisfied:
[X] a total van der Waals force between the compound (I) and Asp121, Pro122, Asp123, Gln124, Asp153, Cys154, Glu155, Pro164, Ser168, Gly169, Ser170, Trp172, Pro254, Phe256, Ser258, Cys259, Asp262, Leu264, and His266 is increased as compared with the compound (2);
[Y] the compound (I) has a site at which at least one of the CH-π interaction with Asp123, the hydrogen bonding with Cys154, and the CH-π interaction with Ser170 is increased, or a site at which at least one non-covalent interaction different from the CH-π interaction with Asp123, the hydrogen bonding with Cys154, and the CH-π interaction with Ser170 other than the van der Waals force is generated between the compound (I) and at least one amino acid residue selected from the group consisting of Asp121, Pro122, Asp123, Gln124, Asp153, Cys154, Glu155, Pro164, Serl68, Gly169, Ser170, Trp172, Pro254, Phe256, Ser258, Cys259, Asp262, Leu264, and His266, the site being included in the compound (2); and
[Z] the compound (I) has a site at which exposure of at least one amino acid residue selected from the group consisting of Asp121, Pro122, Asp123, Gln124, Asp153, Cys154, Glu155, Pro164, Ser168, Gly169, Ser170, Trp172, Pro254, Phe256, Ser258, Cys259, Asp262, Leu264, and His266 to a solvent is reduced as compared with the compound (2).

[Item 16]
The IL-17A activity inhibitor according to Item 13, wherein the compound (I) is the compound (5) or the derivative thereof, the compound (I) being obtained by modifying an original compound (5) so that at least one property selected from the group consisting of [X], [Y], and [Z] below is satisfied:
[X] a total van der Waals force between the compound (I) and Asp121, Pro122, Asp123, Asp153, Cys154, Glu155, Lys160, Pro164, Ser168, Gly169, Ser170, Trp172, Ser258, Cys259, Asp262, Cys263, Leu264, and His266 is increased as compared with the compound (5);
[Y] the compound (I) has a site at which at least one of the hydrogen bonding with Cys154 and the hydrogen bonding with Lys160 is increased, or a site at which at least one non-covalent interaction different from the hydrogen bonding with Cys154 and the hydrogen bonding with Lys160 other than the van der Waals force is generated between the compound (I) and at least one amino acid residue selected from the group consisting of Asp121, Pro122, Asp123, Asp153, Cys154, Glu155, Lys160, Pro164, Ser168, Gly169, Ser170, Trp172, Ser258, Cys259, Asp262, Cys263, Leu264, and His266, the site being included in the compound (5); and
[Z] the compound (I) has a site at which exposure of at least one amino acid residue selected from the group consisting of Asp121, Pro122, Asp123, Asp153, Cys154, Glu155, Lys160, Pro164, Ser168, Gly169, Ser170, Trp172, Ser258, Cys259, Asp262, Cys263, Leu264, and His266 to a solvent is reduced as compared with the compound (5).

[Item 17]
The IL-17A activity inhibitor according to Item 13, wherein the compound (I) is the compound (9) or the derivative thereof, the compound (I) being obtained by modifying an original compound (9) so that at least one property selected from the group consisting of [X], [Y], and [Z] below is satisfied:
[X] a total van der Waals force between the compound (I) and Asp121, Pro122, Asp123, Asp153, Cys154, Glu155, Lys160, Pro164, Ser167, Ser168, Gly169, Ser170, Trp172, Ser258, Cys259, Asp262, Leu264, and His266 is increased as compared with the compound (9);
[Y] the compound (I) has a site at which at least one of the CH-π interaction with Asp121, the hydrogen bonding with Cys154, and the CH-π interaction with Ser170 is increased, or a site at which at least one non-covalent interaction different from the CH-π interaction with Asp121, the hydrogen bonding with Cys154, and the CH-π interaction with Ser170 other than the van der Waals force is generated between the compound (I) and at least one amino acid residue selected from the group consisting of Asp121, Pro122, Asp123, Asp153, Cys154, Glu155, Lys160, Pro164, Ser167, Ser168, Gly169, Ser170, Trp172, Ser258, Cys259, Asp262, Leu264, and His266, the site being included in the compound (9); and
[Z] the compound (I) has a site at which exposure of at least one amino acid residue selected from the group consisting of Asp121, Pro122, Asp123, Asp153, Cys154, Glu155, Lys160, Pro164, Ser167, Ser168, Gly169, Ser170, Trp172, Ser258, Cys259, Asp262, Leu264, and His266 to a solvent is reduced as compared with the compound (9).

[Item 18]
The IL-17A activity inhibitor according to Item 13, wherein the compound (I) is the compound (11) or the derivative thereof, the compound (I) being obtained by modifying an original compound (11) so that at least one property selected from the group consisting of [X], [Y], and [Z] below is satisfied:
[X] a total van der Waals force between the compound (I) and Asp121, Pro122, Gln124, Asp153, Cys154, Glu155, Pro164, Cys165, Ser168, Gly169, Ser170, Trp172, Ser258, Cys259, Asp262, Leu264, and His266 is increased as compared with the compound (11);
[Y] the compound (I) has a site at which at least one of the CH-π interaction or hydrogen bonding with Cys154 is increased, or a site at which at least one non-covalent interaction different from the CH-π interaction or hydrogen bonding with Cys154 other than the van der Waals force is generated between the compound (I) and at least one amino acid residue selected from the group consisting of Asp121, Pro122, Gln124, Asp153, Cys154, Glu155, Pro164, Cys165, Ser168, Gly169, Ser170, Trp172, Ser258, Cys259, Asp262, Leu264, and His266, the site being included in the compound (11); and
[Z] the compound (I) has a site at which exposure of at least one amino acid residue selected from the group consisting of Asp121, Pro122, Gln124, Asp153, Cys154, Glu155, Pro164, Cys165, Ser168, Gly169, Ser170, Trp172, Ser258, Cys259, Asp262, Leu264, and His266 to a solvent is reduced as compared with the compound (11).

[Item 19]
An expression regulator containing the IL-17A activity inhibitor according to any one of Items 1 to 18, wherein the expression regulator is used for regulating an expression level of a gene whose expression level is changed by binding of IL-17Ato IL-17RA in a cell expressing IL-17RA
[Item 20]
The expression regulator according to Item 19, wherein the gene is a gene whose expression is enhanced by binding of IL-17A to IL-17RA, and the expression regulator is used for suppressing the expression of the gene.
[Item 21]
The expression regulator according to Item 20, wherein the gene is at least one selected from the group consisting of IL-6, COX-2, mPGES1, MMP-3, MMP-13, and CXCL1.
[Item 22]
The expression regulator according to Item 20, wherein the gene is a gene whose expression is enhanced by phosphorylation of p38, and the expression regulator is used for suppressing the expression of the gene.
[Item 23]
The expression regulator according to any one of Items 19 to 22, wherein the cell expressing IL-17RA is an intervertebral disc nucleus pulposus cell.
[Item 24]
The expression regulator according to Item 23, wherein the intervertebral disc nucleus pulposus cell is an intervertebral disc nucleus pulposus cell cultured under a low oxygen condition or an intervertebral disc nucleus pulposus cell present in an intervertebral disc tissue.
[Item 25]
The expression regulator according to any one of Items 19 to 24, wherein the cell expressing IL-17RA is a keratinocyte or another epidermal cell.
[Item 26]
A medicament for treating or prophylaxis of a disease with a symptom associated with binding of IL-17A to IL-17RA, the medicament containing the IL-17A activity inhibitor according to any one of Items 1 to 18, or the expression regulator according to any one of Items 19 to 25, as an active ingredient.
[Item 27]
The medicament according to Item 26, wherein the disease with a symptom associated with binding of IL-17A to IL-17RA is a lumbar or cervical intervertebral disc disease, intervertebral disc hernia, spondylolysis and spondylolisthesis, lumbar spinal canal stenosis, lumbar degenerative spondylolisthesis, or lumbar degenerative scoliosis.
[Item 28]
The medicament according to Item 26, wherein the disease with a symptom associated with binding of IL-17Ato IL-17RA is psoriasis vulgaris, articular psoriasis, pustular psoriasis, or psoriatic erythroderma.
[Item 29]
A screening method for an IL-17A activity inhibitor, including:
from a three-dimensional molecular model of a space surrounded by 28 amino acid residues of Phe60, Gln87, Asp121, Pro122, Asp123, Gln124, Asp153, Cys154, Glu155, Lys160, Pro164, Cys165, Ser167, Ser168, Gly169, Ser170, Leu171, Trp172, Asp173, Pro174, Pro254, Phe256, Ser258, Cys259, Asp262, Cys263, Leu264, and His266 that are contained in an extracellular domain of human IL-17RA, or a three-dimensional molecular model of a space surrounded by amino acid residues (where homology between the amino acid residues is 80% or more) corresponding to the 28 amino acid residues contained in an extracellular domain of non-human animal IL-17RA, and a three-dimensional molecular model of a candidate compound,
evaluating binding stability between the candidate compound and IL-17RA through a non-covalent interaction including a van der Waals force generated between an atom or an atomic group included in at least 13 amino acid residues among the amino acid residues and an atom or an atomic group included in the candidate compound, to determine whether the candidate compound has an action of inhibiting binding of IL-17A to IL-17RA by binding to IL-17RA competitively with IL-17A.

[Item 30]
The screening method according to Item 29, further including:
comparing binding stability of the candidate compound with binding stability of each of the compounds (1) to (36).
[Item 31]
A method of inhibiting binding of IL-17A to IL-17RA, the method including:
bringing the IL-17A activity inhibitor according to any one of Items 1 to 16 into contact with IL-17RA outside a living body of a human or another animal.
[Item 32]
A method of regulating expression of a gene whose expression level is changed by binding of IL-17A to IL-17RA, the method including: bringing the expression regulator according to any one of Items 17 to 22 into contact with a cell expressing IL-17RA outside a living body of a human or another animal.

In another aspect, the present invention provides: a method for treating and prophylaxis of a predetermined disease, the method including administering the compound of the present invention in an effective amount; the compound of the present invention used as an IL-17 activity inhibitor to be administered as an active ingredient; the use of the compound of the present invention as an IL-17 activity inhibitor; the use of the compound of the present invention in production of a medicament for treating or prophylaxis of a predetermined disease; and other inventions derived from the use of the compound of the present invention.

### [Advantages of the Invention]

The low-molecular-weight compound provided by the present invention has excellent IL-17A activity inhibiting ability as compared to that of the low-molecular-weight compound according to the related art, and thus, the compound of the present invention is expected to be used as an active ingredient for a medicament for treating or prophylaxis of intervertebral disc degeneration or psoriasis or alleviating pain.

### [Brief Description of Drawings]

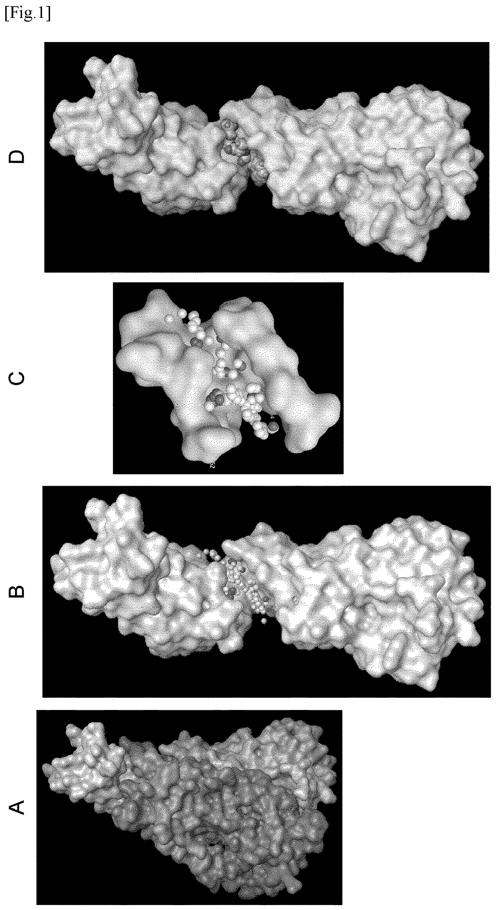

Fig. 1 illustrates molecular structures drawn by software in in silico analysis. [A] illustrates a molecular structure of a complex of human IL-17A and human IL-17RA. [B] illustrates a molecular structure of human IL-17RA. An aggregate of small balls seen in a "groove" in the central portion is a group of pseudo-atoms showing a predicted position of atoms of a candidate compound of a human IL-17A activity inhibitor when the candidate compound binds to human IL-17RA. It is presumed that a non-covalent interaction including a van der Waals force acts between an amino acid residue within 3.5 Å from these pseudo-atoms and the candidate compound. [C] illustrates a partially enlarged molecular structure of the "groove" of human IL-17RA and the pseudo-atomic group in the groove. When represented in color, a hydrophilic pseudo-atom is represented in red and a hydrophobic pseudo-atom is represented in white. [D] illustrates a molecular structure in a state where, as an example of the candidate compound, a compound (1) of the present invention binds to the "groove" of human IL-17RA. When represented in color, a carbon atom, an oxygen atom, a nitrogen atom, and a hydrogen atom are represented in green, red, blue, and white, respectively.

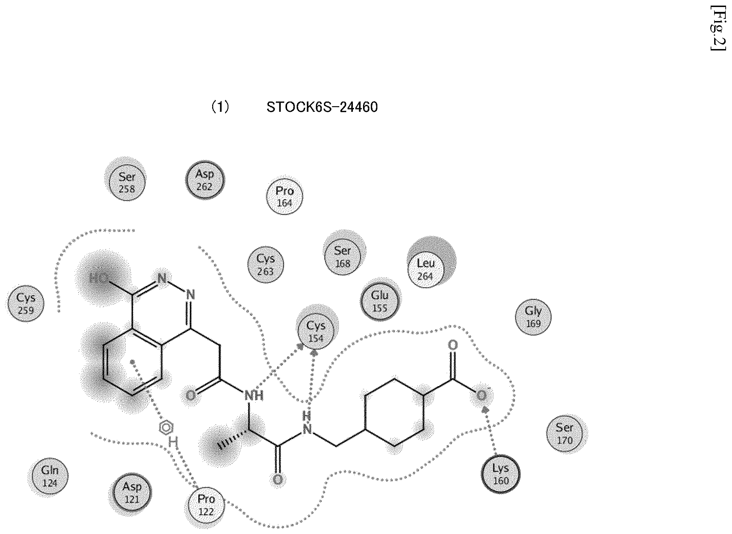

Fig. 2 is a schematic view illustrating a mode of a non-covalent interaction between the compound (1) of the present invention and amino acid residues in an extracellular domain of human IL-17RA. A curved dotted line surrounding a molecule represents a binding interface of the compound of the present invention and human IL-17RA (predetermined amino acid residues in an interaction region). A linear dotted line represents an intermolecular interaction such as a hydrogen bonding or a CH-π interaction. A cloud surrounding atoms of the compound of the present invention represents exposure on a molecular surface to a solvent, and as a size of the cloud is large, the exposure becomes large. An amino acid residue having a thick circle outline indicates an acidic or basic residue. In addition, a disk-like shadow around the circle shows a magnitude of a degree of exposure of the amino acid residue to the solvent when the compound of the present invention is absent, and the degree of exposure of the amino acid residue to the solvent is reduced by binding of the compound. (The same applies to drawings related to other compounds of the present invention described below.)
Fig. 3 is a schematic view illustrating a mode of a non-covalent interaction between a compound (2) of the present invention and amino acid residues in an extracellular domain of human IL-17RA.
Fig. 4 is a schematic view illustrating a mode of a non-covalent interaction between a compound (4) of the present invention and amino acid residues in an extracellular domain of human IL-17RA.
Fig. 5 is a schematic view illustrating a mode of a non-covalent interaction between a compound (5) of the present invention and amino acid residues in an extracellular domain of human IL-17RA.
Fig. 6 is a schematic view illustrating a mode of a non-covalent interaction between a compound (6) of the present invention and amino acid residues in an extracellular domain of human IL-17RA.
Fig. 7 is a schematic view illustrating a mode of a non-covalent interaction between a compound (7) of the present invention and amino acid residues in an extracellular domain of human IL-17RA.
Fig. 8 is a schematic view illustrating a mode of a non-covalent interaction between a compound (8) of the present invention and amino acid residues in an extracellular domain of human IL-17RA.
Fig. 9 is a schematic view illustrating a mode of a non-covalent interaction between a compound (9) of the present invention and amino acid residues in an extracellular domain of human IL-17RA.
Fig. 10 is a schematic view illustrating a mode of a non-covalent interaction between a compound (10) of the present invention and amino acid residues in an extracellular domain of human IL-17RA.
Fig. 11 is a schematic view illustrating a mode of a non-covalent interaction between a compound (11) of the present invention and amino acid residues in an extracellular domain of human IL-17RA.
Fig. 12 is a schematic view illustrating a mode of a non-covalent interaction between a compound (12) of the present invention and amino acid residues in an extracellular domain of human IL-17RA.
Fig. 13 is a schematic view illustrating a mode of a non-covalent interaction between a compound (13) of the present invention and amino acid residues in an extracellular domain of human IL-17RA.
Fig. 14 is a schematic view illustrating a mode of a non-covalent interaction between a compound (14) of the present invention and amino acid residues in an extracellular domain of human IL-17RA.
Fig. 15 is a schematic view illustrating a mode of a non-covalent interaction between a compound (15) of the present invention and amino acid residues in an extracellular domain of human IL-17RA.
Fig. 16 is a schematic view illustrating a mode of a non-covalent interaction between a compound (16) of the present invention and amino acid residues in an extracellular domain of human IL-17RA.
Fig. 17 is a schematic view illustrating a mode of a non-covalent interaction between a compound (17) of the present invention and amino acid residues in an extracellular domain of human IL-17RA.
Fig. 18 is a schematic view illustrating a mode of a non-covalent interaction between a compound (18) of the present invention and amino acid residues in an extracellular domain of human IL-17RA.
Fig. 19 is a schematic view illustrating a mode of a non-covalent interaction between a compound (19) of the present invention and amino acid residues in an extracellular domain of human IL-17RA.
Fig. 20 is a schematic view illustrating a mode of a non-covalent interaction between a compound (20) of the present invention and amino acid residues in an extracellular domain of human IL-17RA.
Fig. 21 is a schematic view illustrating a mode of a non-covalent interaction between a compound (21) of the present invention and amino acid residues in an extracellular domain of human IL-17RA.
Fig. 22 is a schematic view illustrating a mode of a non-covalent interaction between a compound (22) of the present invention and amino acid residues in an extracellular domain of human IL-17RA.
Fig. 23 is a schematic view illustrating a mode of a non-covalent interaction between a compound (23) of the present invention and amino acid residues in an extracellular domain of human IL-17RA.
Fig. 24 is a schematic view illustrating a mode of a non-covalent interaction between a compound (24) of the present invention and amino acid residues in an extracellular domain of human IL-17RA.
Fig. 25 is a schematic view illustrating a mode of a non-covalent interaction between a compound (25) of the present invention and amino acid residues in an extracellular domain of human IL-17RA.
Fig. 26 is a schematic view illustrating a mode of a non-covalent interaction between a compound (26) of the present invention and amino acid residues in an extracellular domain of human IL-17RA.
Fig. 27 is a schematic view illustrating a mode of a non-covalent interaction between a compound (27) of the present invention and amino acid residues in an extracellular domain of human IL-17RA.
Fig. 28 is a schematic view illustrating a mode of a non-covalent interaction between a compound (28) of the present invention and amino acid residues in an extracellular domain of human IL-17RA.
Fig. 29 is a schematic view illustrating a mode of a non-covalent interaction between a compound (29) of the present invention and amino acid residues in an extracellular domain of human IL-17RA.
Fig. 30 is a schematic view illustrating a mode of a non-covalent interaction between a compound (30) of the present invention and amino acid residues in an extracellular domain of human IL-17RA.
Fig. 31 is a schematic view illustrating a mode of a non-covalent interaction between a compound (31) of the present invention and amino acid residues in an extracellular domain of human IL-17RA.
Fig. 32 is a schematic view illustrating a mode of a non-covalent interaction between a compound (32) of the present invention and amino acid residues in an extracellular domain of human IL-17RA.
Fig. 33 is a schematic view illustrating a mode of a non-covalent interaction between a compound (33) of the present invention and amino acid residues in an extracellular domain of human IL-17RA.
Fig. 34 is a schematic view illustrating a mode of a non-covalent interaction between a compound (34) of the present invention and amino acid residues in an extracellular domain of human IL-17RA.
Fig. 35 is a schematic view illustrating a mode of a non-covalent interaction between a compound (35) of the present invention and amino acid residues in an extracellular domain of human IL-17RA.
Fig. 36 is a schematic view illustrating a mode of a non-covalent interaction between a compound (36) of the present invention and amino acid residues in an extracellular domain of human IL-17RA.
Fig. 37 illustrates results relating to "Reference Example 1". [A] and [B] illustrate tissue immunostaining images of IL-17A in a degenerated intervertebral disc tissue (degeneration) of a human and a normal intervertebral disc tissue (normal) of a human, respectively. Scale bar: 10 µm. [C] illustrates a graph showing a percentage of IL-17A positive cells in the degenerated intervertebral disc tissue (degeneration) and the normal intervertebral disc tissue (normal). n = 3. *: p < 0.05.

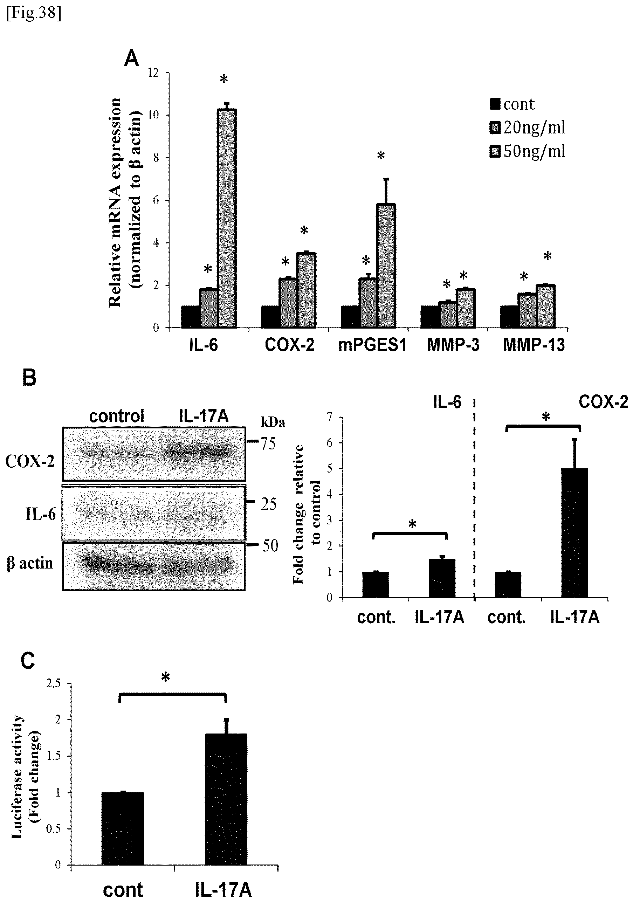

Fig. 38 illustrates results relating to "Reference Example 2". [A] illustrates a graph showing an expression level of mRNA of a gene of each of IL-6, COX-2, mPGES1 (prostaglandin E synthase 1), MMP-3, and MMP-13 when a group in which recombinant mouse IL-17A with a concentration of 20 or 50 ng/ml is administered to a rat NP cell and a non-treated group are cultured under a 1% oxygen condition for 24 hours. *: p < 0.05, n = 5. [B] illustrates an electropherogram (left) and a graph (right) showing an expression level of a protein of each of COX-2, IL-6, and, as an internal control, β actin, when IL-17A with a concentration of 50 ng/ml is administered to a rat NP cell, and the cell is cultured under a 1% oxygen condition for 24 hours. *: p < 0.05, n = 3. [C] illustrates a graph showing transcriptional activity of COX-2 when IL-17A with a concentration of 50 ng/ml is administered to a rat NP cell, and the cell is cultured under a 1% oxygen condition for 24 hours (evaluation by promoter assay method). *: p < 0.05, n = 3.

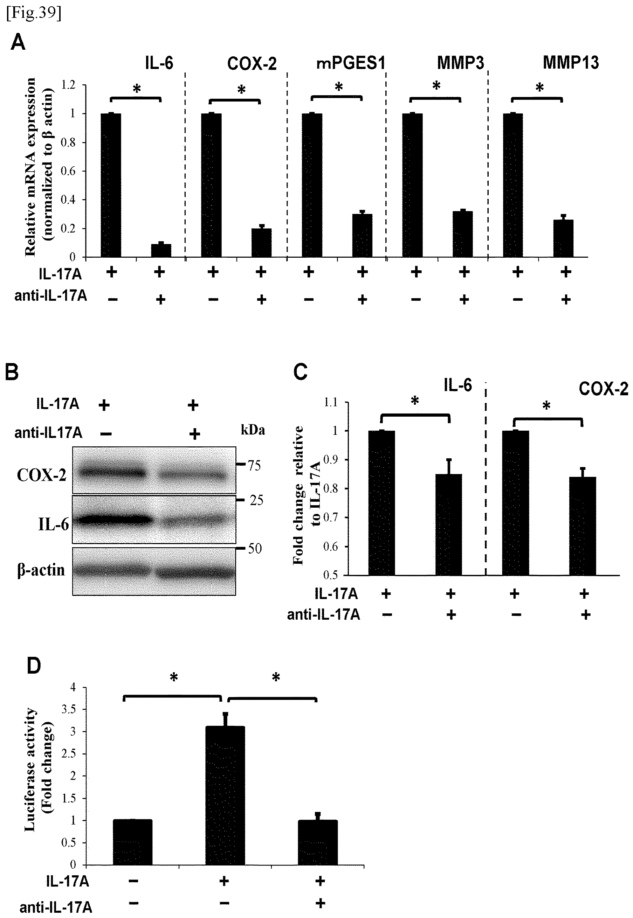

Fig. 39 illustrates results relating to "Reference Example 3". [A] illustrates a graph showing an expression level of mRNA of a gene of each of IL-6, COX-2, mPGES1, MMP-3, and MMP-13 when each of a group in which only recombinant mouse IL-17A with a concentration of 50 ng/ml is administered to a rat NP cell (IL-17A single administration group: "IL-17A" is "+", and "anti-IL-17A" is "-") and a group in which a mixed solution of IL-17A with a concentration of 50 ng/ml and an anti-IL-17A antibody with a concentration of 0.5 µg/ml is administered to a rat NP cell (anti-IL-17A neutralizing antibody combination group: both "IL-17A" and "anti-IL-17A" are "+") is cultured under a 1% oxygen condition for 24 hours. *: p < 0.05, n = 3. [B] illustrates an electropherogram showing an expression level of a protein of each of COX-2, IL-6, and as an internal control, β actin, when each of the IL-17A single administration group and the IIL-17A single administration group is cultured under a 1% oxygen condition for 24 hours. *: p < 0.05, n = 3. [C] illustrates a graph corresponding to [B]. [D] illustrates a graph showing transcriptional activity of COX-2 when each of a group in which both IL-17A and an anti-EL-17A antibody are not administered to a rat NP cell (non-administration group: both "IL-17A" and "anti-IL-17A" are "-"), the IL-17A single administration group, and the IL-17A single administration group is cultured under a 1% oxygen condition for 24 hours (evaluation by promoter assay method). *: p < 0.05, n = 3.

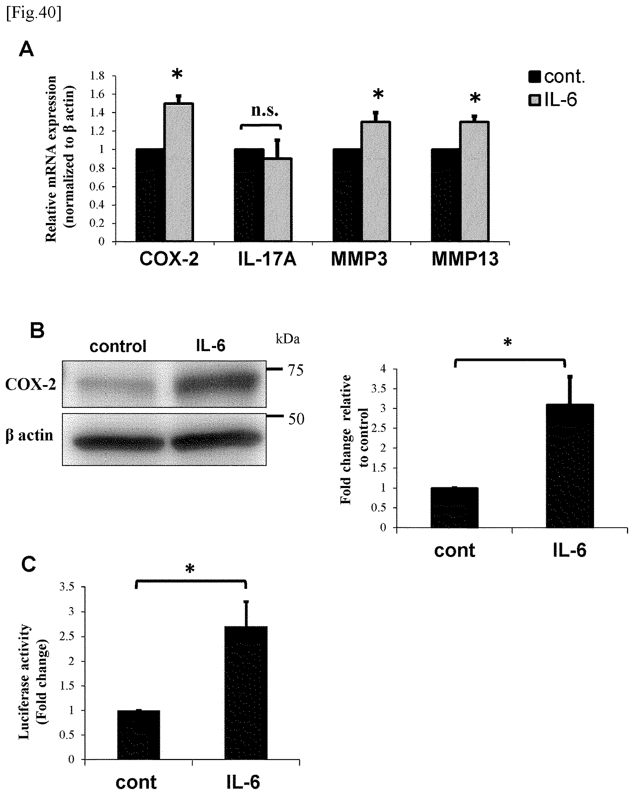

Fig. 40 illustrates results relating to "Reference Example 4". [A] illustrates a graph showing an expression level of mRNA of a gene of each of COX-2, IL-17A, MMP-3, and MMP-13 when a group in which IL-6 with a concentration of 50 ng/ml is administered to a rat NP cell and a non-treated group are cultured under a 1% oxygen condition for 24 hours. *: p < 0.05, n = 3. [B] illustrates an electropherogram (left) and a graph (right) showing an expression level of a protein of each of COX-2 and, as an internal control, β actin, when IL-6 with a concentration of 50 ng/ml is administered to a rat NP cell, and the cell is cultured under a 1% oxygen condition for 24 hours. *: p < 0.05, n = 3. [C] illustrates a graph showing transcriptional activity of COX-2 when IL-6 with a concentration of 50 ng/ml is administered to a rat NP cell, and the cell is cultured under a 1% oxygen condition for 24 hours (evaluation by promoter assay method). *: p < 0.05, n = 3.

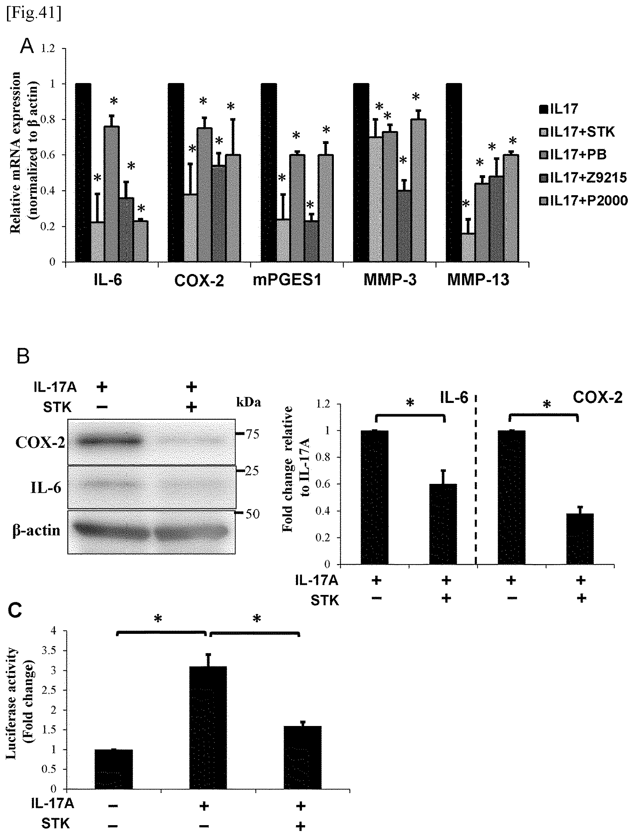

Fig. 41 illustrates results relating to "Example 1". [A] illustrates a graph showing an expression level of mRNA of a gene of each of IL-6, COX-2, mPGES 1, MMP-3, and MMP-13 when each of a group in which only recombinant mouse IL-17A with a concentration of 50 ng/ml is administered to a rat NP cell (IL-17 group) and a group in which recombinant mouse IL-17A with a concentration of 50 ng/ml and any one of the compounds (3), (2), (5), and (11) with a concentration of 50 µg/ml are administered to a rat NP cell (IL17+STK group, IL17+PB group, IL17+Z9215 group, and IL17+P2000 group, respectively) is cultured under a 1% oxygen condition for 24 hours. *: p < 0.05, n = 3. [B] illustrates an electropherogram (left) and a graph (right) showing an expression level of a protein of each of COX-2 and IL-6 when each of the IL-17 group and the IL-17+STK group is cultured under a 1% oxygen condition for 24 hours. *: p < 0.05, n = 3. [C] illustrates a graph showing transcriptional activity of COX-2 when each of a group in which both IL-17A and the compound (1) are not administered to a rat NP cell (non-administration group: both "IL-17A" and "STK" are "-"), the IL-17 group, and the IL-17+STK group is cultured under a 1% oxygen condition for 24 hours (evaluation by promoter assay method). *: p < 0.05, n = 3.
Fig. 42 illustrates results relating to "Example 2". [A] illustrates a graph showing an expression level of mRNA of IL-6 in a rat NP cell (normalized to β actin). *: p < 0.05, n = 3. [B] illustrates a graph showing an expression level of mRNA of COX-2 (normalized to β actin). *: p < 0.05, n = 3.

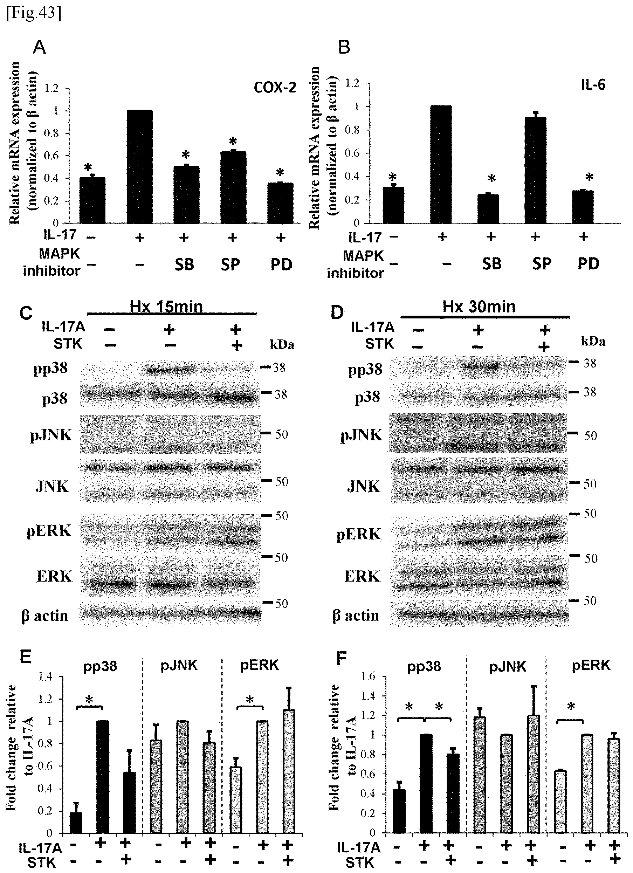

Fig. 43 illustrates results relating to "Example 3". [A] illustrates a graph showing an expression level of mRNA of COX-2 when a group in which recombinant mouse IL-17A with a concentration of 50 ng/ml is administered to a rat NP cell ("IL-17"+/"Inhibitor"-), a group in which IL-17A with a concentration of 50 ng/ml and a p38 phosphorylation inhibitor SB203580, a JNK phosphorylation inhibitor SP600125, or an ERK phosphorylation inhibitor PD98059 with a concentration of 10 µM are administered to a rat NP cell ("IL-17"+/"Inhibitor" SB, SP, or PD, respectively), and a non-treated group ("IL-17"-/"Inhibitor"-) are cultured under a 1% oxygen condition for 24 hours. *: p < 0.05, n = 3. [B] illustrates a group showing an expression level of mRNA of IL-6 in the same groups as those in [A]. *: p < 0.05, n = 3. [C] illustrates an electropherogram showing an expression level of a protein of each of phosphorylated p38 (pp38), p38, phosphorylated JNK (pJNK), JNK, phosphorylated ERK (pERK), and ERK when a group in which IL-17A with a concentration of 50 ng/ml is administered to a rat NP cell ("IL-17"+/"STK"-), a group in which IL-17A with a concentration of 50 ng/ml and the compound (1) of the present invention with a concentration of 50 µg/ml are administered to a rat NP cell ("IL-17"+/"STK"+), and a non-treated group ("IL-17"-/"STK"-) are cultured under a 1% oxygen condition for 15 minutes. [D] illustrates an electropherogram showing an expression level of each protein when the same groups as those in [C] are cultured under a 1% oxygen condition for 30 minutes. [E] illustrates a graph corresponding to the electropherogram of [C]. *: p < 0.05, n = 4. [F] illustrates a graph corresponding to the electropherogram of [D]. *: p < 0.05, n = 4.

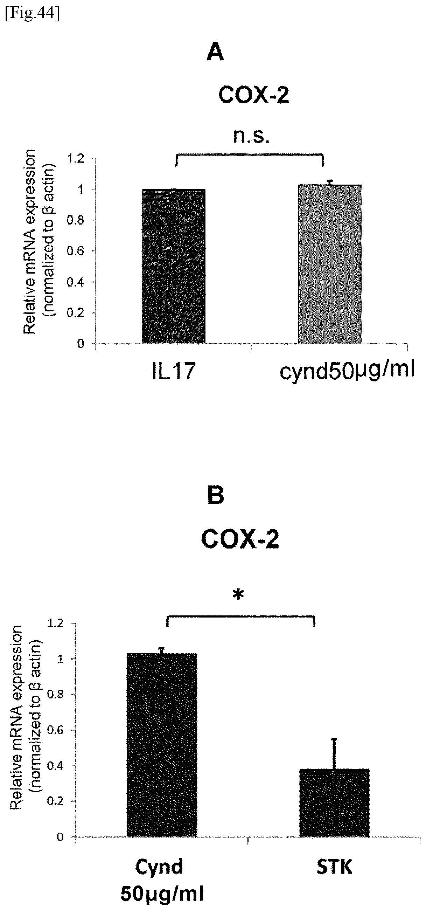

Fig. 44 illustrates results relating to "Comparative Example 1". [A] illustrates a graph showing an expression level of mRNA of COX-2 when each of a group in which only recombinant mouse IL-17A with a concentration of 50 ng/ml is administered to a rat NP cell (IL-17 group) and a group in which IL-17A with a concentration of 50 ng/ml and the compound of Non-Patent Document 3 with a concentration of 50 µg/ml are administered to a rat NP cell (cynd 50 µg/ml group) is cultured under a 1% oxygen condition for 24 hours. n = 3. [B] illustrates a graph obtained by comparing the expression level of mRNA of COX-2 of the cynd 50 µg/ml group of [A] and the expression level of mRNA of COX-2 of the IL-17+STK group obtained in [Example 1] (relative value of the latter when the former is 1). *: p < 0.05, n = 3.
Fig. 45 is a schematic view illustrating a reaction pathway in which interleukin-17 family (A, B, C, D, E, and F) is involved.

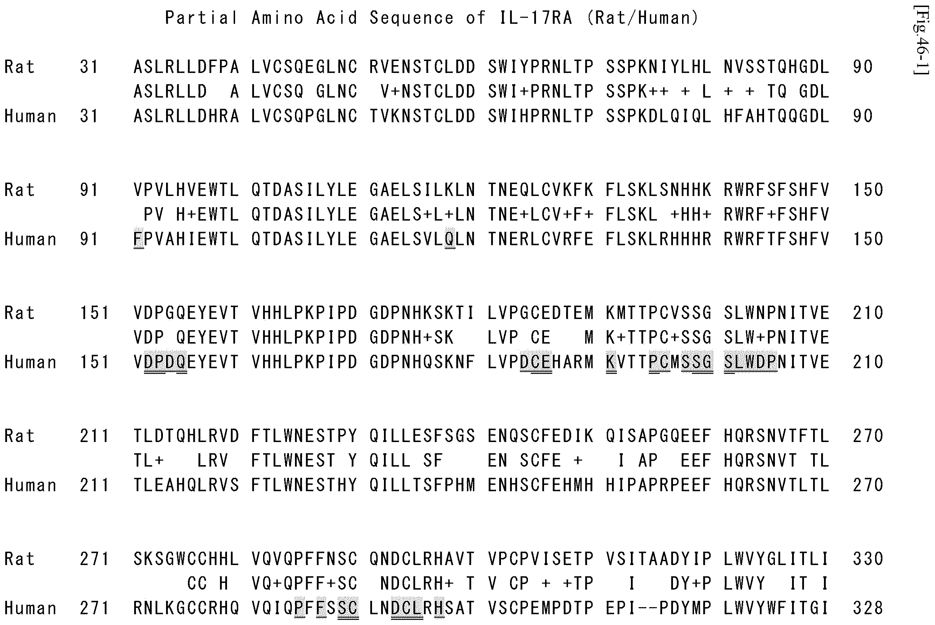

Fig. 46-1 is a view illustrating a result of comparing partial amino acid sequences of human and rat IL-17RAs by BLAST (https://blast.ncbi.nlm.nih.gov/Blast.cgi). Single underlines represent 28 predetermined amino acid residues in an interaction region, and each double underline represents an amino acid residue at which a non-covalent interaction (intermolecular interaction) with the representative compound (any one of the compounds (1) to (36)) of the present invention other than a van der Waals force is generated. The amino acid residue numbers indicated on the right and left of the sequences in the present drawing are the same as the amino acid residue numbers of SEQ ID NO: 1 and SEQ ID NO: 2. For example, Cys154 included in a predetermined amino acid residue in an interaction region corresponds to C representing the 185^{th} amino acid residue in the present drawing.

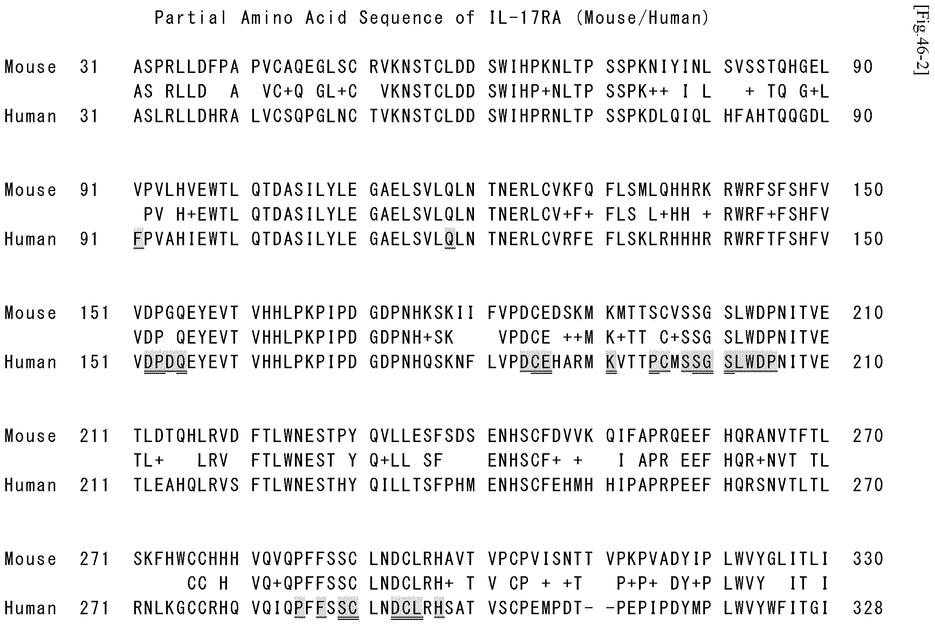

Fig. 46-2 is a view illustrating a result of comparing partial amino acid sequences of human and mouse IL-17RAs by BLAST (https://blast.ncbi.nlm.nih.gov/Blast.cgi). Single underlines represent 28 predetermined amino acid residues in an interaction region, and each double underline represents an amino acid residue at which a non-covalent interaction (intermolecular interaction) with the representative compound (any one of the compounds (1) to (36)) of the present invention other than a van der Waals force is generated. The amino acid residue numbers indicated on the right and left of the sequences in the present drawing are the same as the amino acid residue numbers of SEQ ID NO: 1 and SEQ ID NO: 2. For example, Cys154 included in the predetermined amino acid residues in an interaction region corresponds to C representing the 185^{th} amino acid residue in the present drawing.
Fig. 47 illustrates results relating to "Example 4". [A] illustrates optical microscope photographs of HE-stained samples of a mouse skin. [B] illustrates a graph showing a thickness of an epidermis layer based on the optical microscope photographs. Normal: normal group, IMQ: IMQ group (mice with psoriasis-like dermatitis caused by imiquimod cream), DMSO: Sham group (mice with an affected area to which DMSO is applied), and STK: STK group (mice with an affected area to which a DMSO solution of the compound (3) is applied)
Fig. 48 illustrates results relating to "Example 4". [A] illustrates fluorescent microscope photographs of immunofluorescent stained samples obtained by using an anti-CXCL1 antibody of mouse skin. [B] illustrates a graph showing expression areas of CXCL1 based on the fluorescent microscope photographs. Normal: normal group, IMQ: IMQ group (diseased mice with psoriasis-like dermatitis caused by imiquimod cream), DMSO: Sham group (mice with an affected area to which DMSO is applied), and STK: STK group (mice with an affected area to which a DMSO solution of the compound (3) is applied)

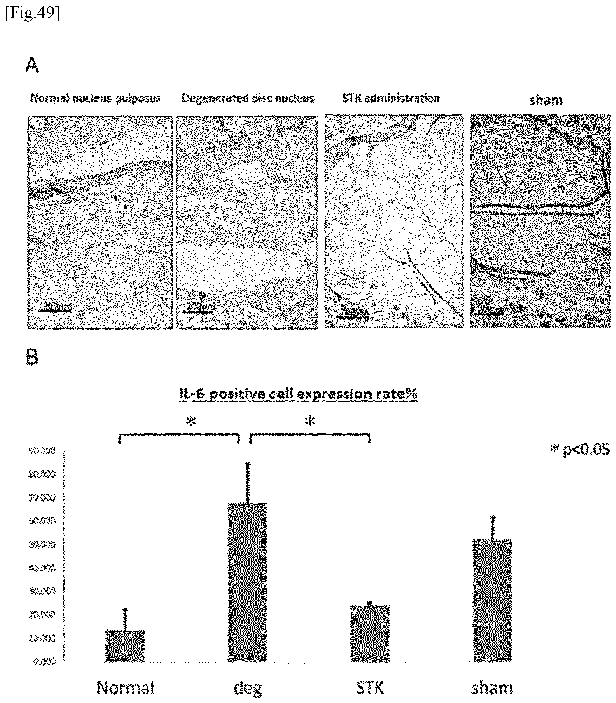

Fig. 49 illustrates results relating to "Example 5". [A] illustrates optical microscope photographs of immunostained samples obtained by using an anti-IL-6 antibody of a rat caudal vertebra. [B] illustrates a graph showing expression rates of IL-6 positive cells based on the optical microscope photographs. Normal: normal group, deg: degeneration group (rat subjected to intervertebral disc degeneration), STK: STK group (mice to which a DMSO solution of the compound (3) is injected, after being subjected to the intervertebral disc degeneration), and sham: Sham group (mice to which DMSO is injected, after being subjected to the intervertebral disc degeneration).

### [Mode for Carrying Out the Invention]

In a plurality of aspects, the present invention includes inventions belonging to different categories (agents, medicaments, methods, and the like). Matters described in the present specification can be in common in the inventions different from each other in accordance with the context, unless specifically noted.

Unless otherwise noted, each substituent used in the present specification is defined as follows.

A "C₁₋₃ alkyl group" refers to a linear or branched saturated hydrocarbon group having 1 to 3 carbon atoms. Examples thereof can include methyl, ethyl, propyl, and isopropyl.

A "C₄₋₆ alkyl group" refers to a linear or branched saturated hydrocarbon group having 4 to 6 carbon atoms. Examples thereof can include butyl, isobutyl, sec-butyl, tert-butyl, pentyl, isopentyl, neopentyl, 1-ethylpropyl, hexyl, isohexyl, 1,1-dimethylbutyl, 2,2-dimethylbutyl, 3,3-dimethylbutyl, and 2-ethylbutyl.

A "C₃₋₁₀ cycloalkyl group" refers to a cyclic saturated hydrocarbon group having 3 to 10 carbon atoms. Examples thereof can include cyclopropyl, cyclobutyl, cyclopentyl, cyclohexyl, cycloheptyl, and cyclooctyl.

A "C₃₋₁₀ cycloalkenyl group" refers to a cyclic unsaturated hydrocarbon group having 3 to 10 carbon atoms and one carbon-carbon double bond. Examples thereof can include cyclopropenyl, cyclobutenyl, cyclopentenyl, cyclohexenyl, cycloheptenyl, and cyclooctenyl.

A "6- to 14-membered aromatic hydrocarbon cyclic group (aryl group)" refers to a group derived from a 6- to 14-membered (preferably, 6- to 10-membered) aromatic cyclic compound having a carbon atom as a ring-constituting atom. Examples thereof can include phenyl, 1-naphthyl, 2-naphthyl, 1-anthryl, 2-anthryl, and 9-anthryl.

A "5- to 14-membered aromatic heterocyclic ring" refers to a 5- to 14-membered (preferably, 5- to 10-membered) aromatic cyclic compound having at least one (preferably, 1 to 4) heteroatom selected from the group consisting of a nitrogen atom, a sulfur atom, and an oxygen atom in addition to a carbon atom as a ring-constituting atom. Examples thereof can include the following:
a 5- or 6-membered monocyclic aromatic heterocyclic ring such as thiophene, furan, pyrrole, imidazole, pyrazole, thiazole, isothiazole, oxazole, isoxazole, pyridine, pyrazine, pyrimidine, pyridazine, 1,2,4-oxadiazole, 1,3,4-oxadiazole, 1,2,4-thiadiazole, 1,3,4-thiadiazole, triazole, tetrazole, or triazine; and
a 8- to 14-membered condensed polycyclic (preferably, bi- or tri-cyclic) aromatic heterocyclic ring such as benzothiophene, benzofuran, benzimidazole, benzoxazole, benzisoxazole, benzothiazole, benzisothiazole, benzotriazole, imidazopyridine, thienopyridine, furopyridine, pyrrolopyridine, pyrazolopyridine, oxazolopyridine, thiazolopyridine, imidazopyrazine, imidazopyrimidine, thienopyrimidine, furopyrimidine, pyrrolopyrimidine, pyrazolopyrimidine, oxazolopyrimidine, thiazolopyrimidine, pyrazolopyrimidine, pyrazolotriazine, naphtho[2,3-b]thiophene, phenoxathiin, indole, isoindole, 1H-indazole, purine, isoquinoline, quinoline, phthalazine, naphthyridine, quinoxaline, quinazoline, cinnoline, carbazole, β-carboline, phenanthridine, acridine, phenazine, phenothiazine, or phenoxazine.

A "3- to 14-membered non-aromatic heterocyclic ring" refers to a 3- to 14-membered (preferably, 4- to 10-membered) non-aromatic cyclic compound having at least one (preferably, 1 to 4) heteroatom selected from the group consisting of a nitrogen atom, a sulfur atom, and an oxygen atom in addition to a carbon atom as a ring-constituting atom. Examples thereof can include the following:
a 3- to 8-membered monocyclic non-aromatic heterocyclic ring such as aziridine, oxirane, thiirane, azetidine, oxetane, thietane, tetrahydrothiophene, tetrahydrofuran, pyrroline, pyrrolidine, imidazoline, imidazolidine, oxazoline, oxazolidine, pyrazoline, pyrazolidine, thiazoline, thiazolidine, tetrahydroisothiazole, tetrahydrooxazole, tetrahydroisoxazole, piperidine, piperazine, tetrahydropyridine, dihydropyridine, dihydrothiopyran, tetrahydropyrimidine, tetrahydropyridazine, dihydropyran, tetrahydropyran, tetrahydrothiopyran, morpholine, thiomorpholine, azepanin, diazepane, azepine, azocane, diazocane, or oxepane; and
a 9- to 14-membered condensed polycyclic (preferably, bi- or tri-cyclic) non-aromatic heterocyclic ring such as dihydrobenzofuran, dihydrobenzimidazole, dihydrobenzoxazole, dihydrobenzothiazole, dihydrobenzisothiazole, dihydronaphtho[2,3-b]thiophene, tetrahydroisoquinoline, tetrahydroquinoline, 4H-quinolizine, indoline, isoindoline, tetrahydrothieno[2,3-c]pyridine, tetrahydrobenzazepine, tetrahydroquinoxaline, tetrahydrophenanthridine, hexahydrophenothiazine, hexahydrophenoxazine, tetrahydrophthalazine, tetrahydronaphthyridine, tetrahydroquinazoline, tetrahydrocinnoline, tetrahydrocarbazole, tetrahydro-β-carboline, tetrahydroacridine, tetrahydrophenazine, tetrahydrothioxanthene, or octahydroisoquinoline.

Examples of a substituent that a "C₃₋₁₀ cycloalkyl group which is optionally substituted", a "C₃₋₁₀ cycloalkenyl group which is optionally substituted", a "6- to 14-membered aromatic hydrocarbon cyclic group (aryl group) which is optionally substituted", a "5- to 14-membered aromatic heterocyclic group which is optionally substituted", a "3- to 14-membered non-aromatic heterocyclic group which is optionally substituted", a "C₁₋₃ alkyl group which is optionally substituted", or a "C₄₋₆ alkyl group which is optionally substituted" may have can include substituents included in the following "substituent group A":

### [Substituent group A]

(1) a halogen atom;
(2) a nitro group;
(3) a cyano group;
(4) an oxo group;
(5) a hydroxyl group;
(6) a C₁₋₆ alkoxy group which is optionally halogenated;
(7) a C₆₋₁₄ aryloxy group (for example, phenoxy or naphthoxy);
(8) a C₇₋₁₆ aralkyloxy group (for example, benzyloxy);
(9) a 5- to 14-membered aromatic heterocyclic oxy group (for example, pyridyloxy);
(10) a 3- to 14-membered non-aromatic heterocyclic oxy group (for example, morpholinyloxy or piperidinyloxy);
(11) a C₁₋₆ alkyl-carbonyloxy group (for example, acetoxy or propanoyloxy), or C₁₋₆ alkyl-thiocarbonyloxy group (for example, thioacetoxy or thiopropanoyloxy);
(12) a C₆₋₁₄ aryl-carbonyloxy group (for example, benzoyloxy, 1-naphthoyloxy, or 2-naphthoyloxy);
(13) a C₁₋₆ alkoxy-carbonyloxy group (for example, methoxycarbonyloxy, ethoxycarbonyloxy, propoxycarbonyloxy, or butoxycarbonyloxy);
(14) a mono- or di-C₁₋₆ alkyl-carbamoyloxy group (for example, methylcarbamoyloxy, ethylcarbamoyloxy, dimethylcarbamoyloxy, or diethylcarbamoyloxy);
(15) a C₆₋₁₄ aryl-carbamoyloxy group (for example, phenylcarbamoyloxy or naphthylcarbamoyloxy);
(16) a 5- to 14-membered aromatic heterocyclic carbonyloxy group (for example, nicotinoyloxy);
(17) a 3- to 14-membered non-aromatic heterocyclic carbonyloxy group (for example, morpholinylcarbonyloxy or piperidinylcarbonyloxy);
(18) a C₁₋₆ alkylsulfonyloxy group which is optionally halogenated (for example, methylsulfonyloxy or trifluoromethylsulfonyloxy);
(19) a C₆₋₁₄ arylsulfonyloxy group which is optionally substituted with a C₁₋₆ alkyl group (for example, phenylsulfonyloxy or toluenesulfonyloxy);
(20) a C₁₋₆ alkylthio group which is optionally halogenated;
(21) a 5- to 14-membered aromatic heterocyclic group which is optionally substituted;
(22) a 3- to 14-membered non-aromatic heterocyclic group which is optionally substituted;
(23) a formyl group;
(24) a carboxyl group or a thiocarboxyl group;
(25) a C₁₋₆ alkyl-carbonyl group which is optionally halogenated;
(26) a C₆₋₁₄ aryl-carbonyl group;
(27) a 5- to 14-membered aromatic heterocyclic carbonyl group;
(28) a 3- to 14-membered non-aromatic heterocyclic carbonyl group;
(29) a C₁₋₆ alkoxy-carbonyl group;
(30) a C₆₋₁₄ aryloxy-carbonyl group (for example, phenyloxycarbonyl, 1-naphthyloxycarbonyl, or 2-naphthyloxycarbonyl);
(31) a C₇₋₁₆ aralkyloxy-carbonyl group (for example, benzyloxycarbonyl or phenethyloxycarbonyl);
(32) a carbamoyl group;
(33) a thiocarbamoyl group;
(34) a mono- or di-C₁₋₆ alkyl-carbamoyl group;
(35) a C₆₋₁₄ aryl-carbamoyl group (for example, phenylcarbamoyl);
(36) a 5- to 14-membered aromatic heterocyclic carbamoyl group (for example, pyridylcarbamoyl or thienylcarbamoyl);
(37) a 3- to 14-membered non-aromatic heterocyclic carbamoyl group (for example, morpholinylcarbamoyl or piperidinylcarbamoyl);
(38) a C₁₋₆ alkylsulfonyl group which is optionally halogenated;
(39) a C₆₋₁₄ aryl-sulfonyl group;
(40) a 5- to 14-membered aromatic heterocyclic sulfonyl group (for example, pyridylsulfonyl or thienylsulfonyl);
(41) a C₁₋₆ alkylsulfinyl group which is optionally halogenated;
(42) a C₆₋₁₄ arylsulfinyl group (for example, phenylsulfinyl, 1-naphthylsulfinyl, or 2-naphthylsulfinyl);
(43) a 5- to 14-membered aromatic heterocyclic sulfinyl group (for example, pyridylsulfinyl or thienylsulfinyl);
(44) an amino group or an imino group;
(45) a mono- or di-C₁₋₆ alkylamino group (for example, methylamino, ethylamino, propylamino, isopropylamino, butylamino, dimethylamino, diethylamino, dipropylamino, dibutylamino, or N-ethyl-N-methylamino);
(46) a mono- or di-C₆₋₁₄ arylamino group (for example, phenylamino);
(47) a 5- to 14-membered aromatic heterocyclic amino group (for example, pyridylamino);
(48) a C₇₋₁₆ aralkylamino group (for example, benzylamino);
(49) a formylamino group;
(50) a C₁₋₆ alkyl-carbonylamino group (for example, acetylamino, propanoylamino, or butanoylamino);
(51) a (C₁₋₆ alkyl) (C₁₋₆ alkyl-carbonyl)amino group (for example, N-acetyl-N-methylamino);
(52) a C₆₋₁₄ aryl-carbonylamino group (for example, phenylcarbonylamino or naphthylcarbonylamino);
(53) a C₁₋₆ alkoxy-carbonylamino group (for example, methoxycarbonylamino, ethoxycarbonylamino, propoxycarbonylamino, butoxycarbonylamino, or tert-butoxycarbonylamino);
(54) a C₇₋₁₆ aralkyloxy-carbonylamino group (for example, benzyloxycarbonylamino);
(55) a C₁₋₆ alkylsulfonylamino group (for example, methylsulfonylamino or ethylsulfonylamino);
(56) a C₆₋₁₄ arylsulfonylamino group which is optionally substituted with a C₁₋₆ alkyl group (for example, phenylsulfonylamino or toluenesulfonylamino);
(57) a C₁₋₆ alkyl group which is optionally halogenated;
(58) a C₂₋₆ alkenyl group;
(59) a C₂₋₆ alkynyl group;
(60) a C₃₋₁₀ cycloalkyl group;
(61) a C₃₋₁₀ cycloalkenyl group; and
(62) a C6-14 aryl group.

A "divalent group (amide bond) derived from a carbamoyl group" may have an -NH-CO- orientation or a -CO-NH- orientation.

A "divalent group (amide bond) derived from a carbamoyl group, which is optionally N-substituted and/or is optionally linked to a divalent group derived from a C₁₋₆ alkyl-carbonyl group" indicates that in the amide bond (-NH-CO- or -CO-NH-), the nitrogen atom (N) may have a substituent, the divalent group derived from the C₁₋₆ alkyl-carbonyl group may be linked to one end or each of both ends (preferably, one end) of the amide bond, or both of two features may be provided.N-substituted also includes the case where two bonds of N form a ring structure (for example, piperazine).

Examples of the substituent that the nitrogen atom of the amide bond may have can include substituents selected from the substituent group A.

A "divalent group derived from a C₁₋₃ alkyl-carbonyl group" refers to a group to which a divalent group (-CₙH₂ₙ-; n = 1 to 3) derived from a linear or branched hydrocarbon group (C1-3 alkyl group) having 1 to 3 carbon atoms and a carbonyl group (-CO-) are linked, and may have a -CₙH₂ₙ-CO- orientation or a -CO-CₙH₂ₙ- orientation.

A "C₁₋₃ alkylene group" refers to a divalent group derived from a linear or branched saturated hydrocarbon (C₁₋₃ alkyl group) having 1 to 3 carbon atoms. Examples thereof can include -CH₂-, -(CH₂)₂-, -(CH₂)₃-, -CH(CH₃)-, -C(CH₃)₂-, - CH(C₂H₅)-, and -CH(CH₃)-CH₂-. A "C₁₋₆ alkylene group" refers to a divalent group derived from a linear or branched hydrocarbon (C₁₋₆ alkyl group) having 1 to 6 carbon atoms. Examples thereof can include -(CH₂)₄-, -(CH₂)₅-, -(CH₂)₆-, -CH(CH(CH₃)₂))-, - CH(C₂H₄(CH₃)₂)-, -CH(C₃H₆(CH₃)₂)-, -CH(C(CH₃)₃)-, and -CH(CH(CH₃)₂))-CH-, in addition to the "C₁₋₃ alkylene group".

A "C₁₋₃ alkenylene group" refers to a divalent group derived from a linear or branched unsaturated hydrocarbon (C₁₋₃ alkenyl group) having 1 to 3 carbon atoms and one carbon-carbon double bond. Examples thereof can include -CH₂=CH₂-, - CH₂=CH₂-CH₂-, and -CH₂-CH₂=CH₂-. However, in a case where the carbon-carbon double bond is formed between a carbon atom at a terminal of the C₁₋₃ alkenyl group and a carbon atom adjacent thereto (for example, in the compound of the present invention, between a carbon atom at a terminal of the "C₁₋₃ alkenylene group" corresponding to a site L2 and a carbon atom at a site B adjacent thereto), for example, =CH-, =CH-CH₂-, and =CH-CH₂-CH₂ are also included in the "C₁₋₃ alkenylene group". Either a cis- or trans-position may be acceptable due to an unsaturated bond.

A "C₁₋₃ alkylene group which is optionally linked to a divalent group (amide bond) derived from a carbamoyl group" indicates that a divalent group (amide bond) derived from a carbamoyl group may be linked to one end or each of both ends (preferably, one end) of the C₁₋₃ alkylene group in the -NH-CO- orientation or the -CO-NH- orientation. Examples of the C₁₋₃ alkylene group linked to the divalent group (amide bond) derived from the carbamoyl group can include -(CH₂)ₙ-NH-CO-, -(CH₂)ₙ-CO-NH-, -NH-CO-(CH₂)ₙ-, and -CO-NH-(CH₂)ₙ- (n is an integer of 1 to 3).

### -IL-17 Activity Inhibitor-

An "IL-17 activity inhibitor" provided in an aspect of the present invention contains a compound (a compound according to a first embodiment of the present invention) having an action of inhibiting binding of interleukin-17A (IL-17A) to interleukin-17 receptor A (IL-17RA) by binding to IL-17RA competitively with IL-17A through a van der Waals force or a non-covalent interaction other than the van der Waals force that acts between the compound and some amino acid residues among 28 amino acid residues of Phe60, Gln87, Asp121, Pro122, Asp123, Gln124, Asp153, Cys154, Glu155, Lys160, Pro164, Cys165, Ser167, Ser168, Gly169, Ser170, Leu171, Trp172, Asp173, Pro174, Pro254, Phe256, Ser258, Cys259, Asp262, Cys263, Leu264, and His266 (in the present specification, these 28 amino acid residues are collectively called "predetermined amino acid residues constituting an interaction region") that are contained in an extracellular domain of human IL-17RA in a space (interaction region) surrounded by the 28 amino acid residues, or a pharmaceutically acceptable salt, solvate, or prodrug thereof.

Since the "IL-17 activity inhibitor" inhibits activation of IL-17RA caused by binding of IL-17A to IL-17RA, the IL-17 activity inhibitor can be also referred to as an "IL-17RA activation inhibitor" (an "IL-17 activity inhibitor" in the present specification is replaced with an "IL-17RA activation inhibitor").

An amino acid sequence of human IL-17RA is shown in SEQ ID NO: 1 (GenBank: AAH11624.1, https://www.ncbi.nlm.nih.gov/protein/AAH11624.1). In the present specification, the 1^{st} amino acid residue in the extracellular domain of human IL-17RA corresponds to the 32^{nd} amino acid residue in SEQ ID NO: 1 (Ser). Therefore, among the predetermined amino acid residues constituting the interaction region, for example, Phe60 (phenylalanine which is the 60^{th} amino acid residue in the extracellular domain), Cys154 (cysteine that is the 154^{th} amino acid residue in the extracellular domain), and His266 (histidine that is the 266^{th} amino acid residue in the extracellular domain) correspond to the 91^{st} amino acid residue in SEQ ID NO: 1 (Phe), the 185^{th} amino acid residue in SEQ ID NO: 1 (Cys), and the 297^{th} amino acid residue in SEQ ID NO: 1 (His), respectively. If necessary, the amino acid residue number in the "extracellular domain" dealt with in the present specification (and the drawings) as described above can be replaced with the amino acid residue number in SEQ ID NO: 1 (including a signal peptide, an extracellular domain, a transmembrane region (α helix), and a cytoplasmic domain of IL-17RA). It is clear that the invention defined by the amino acid residues with the replaced numbers is not actually altered at all from the invention defined by the amino acid residues with the numbers before the replacement.

For the sake of comparison, an amino acid sequence of rat IL-17RA is shown in SEQ ID NO: 2 (NCBI Reference Sequence: NP_001101353.2, https://www.ncbi.nlm.nih.gov/protein/NP_001101353.2). In addition, Fig. 46-1 illustrates a result of comparing portions including the predetermined amino acid residues constituting the interaction regions in amino acid sequences of human and rat IL-17RAs. Between human and rat IL-17RAs, homology of the interaction region including the predetermined amino acid residues is high (23 amino acid residues among the 28 predetermined amino acid residues are identical, and sequence homology is 82.1%). Therefore, in the present specification, from the result obtained by using human cells (with respect to human IL-17RA) in Example 2, the result obtained by using rat cells (with respect to rat IL-17RA) in Examples 1 and 3, and the result of an in vivo test using rats in Example 5, those skilled in the art can understand that the compound of the present invention has an activity inhibiting action with respect to human IL-17RA and an action regulating expression of a predetermined gene, and is effective in prophylaxis or treating a predetermined disease in a human.

For the sake of comparison, an amino acid sequence of mouse IL-17RA is shown in SEQ ID NO: 3 (NCBI Reference Sequence: NP_032385.1, https://www.ncbi.nlm.nih.gov/protein/NP_032385.1). In addition, Fig. 46-2 illustrates a result of comparing portions including the predetermined amino acid residues constituting the interaction regions among amino acid sequences of human and mouse IL-17RAs. Between human and rat IL-17RAs, homology of the interaction region including the predetermined amino acid residues is high (25 amino acid residues among the 28 predetermined amino acid residues are identical, and sequence homology is 89.3%). Therefore, from the result obtained by using human cells (with respect to human IL-17RA) shown in Example 2, and the result of an in vivo test using mice in Example 4 in the present specification, those skilled in the art can understand that the compound of the present invention has an activity inhibiting action with respect to human IL-17RA and an action regulating expression of a predetermined gene, and is effective in prophylaxis or treating a predetermined disease in a human.

In an aspect of the present invention, the IL-17A activity inhibitor of the present invention is determined by a van der Waals force and other non-covalent interactions with the predetermined amino acid residues contained in the extracellular domain of human IL-17RA (interaction region). Those skilled in the art can understand that even in a case where the IL-17A activity inhibitor is used for non-human animals, and preferably non-human animal IL-17RA, for example, even in a case where the IL-17A activity inhibitor is used for IL-17RA in which full-length sequence homology of IL-17RA, preferably sequence homology in the extracellular domain, or particularly preferably sequence homology in the interaction region (the predetermined 28 amino acid residues) is 50% or more, 60% or more, 70% or more, or 75% or more, and preferably 80% or more, 85% or more, 90% or more, or 95% or more, the same activity inhibiting ability is exhibited. That is, the IL-17A activity inhibitor of the present invention is a typical human IL-17A activity inhibitor, but is not limited thereto, and includes non-human mammalian IL-17A (preferably, having the above sequence homology) activity inhibitor.

On the contrary, in an aspect of the present invention, the IL-17A activity inhibitor of the present invention is determined by a van der Waals force and other non-covalent interactions with the predetermined amino acid residues contained in the extracellular domain of non-human animal IL-17RA (interaction region). Those skilled in the art can understand that even in a case where the IL-17A activity inhibitor is used for IL-17RA of a human or another animal (preferably, non-human mammal), for example, even in a case where the IL-17A activity inhibitor is used for IL-17RA in which full-length sequence homology of IL-17RA, preferably sequence homology in the extracellular domain, or particularly preferably sequence homology in the interaction region (the predetermined 28 amino acid residues) is 50% or more, 60% or more, 70% or more, or 75% or more and preferably 80% or more, 85% or more, 90% or more, or 95% or more, the same activity inhibiting ability is exhibited. The sequence homology in the present specification can be calculated by using a general method (tool), for example, a basic local alignment search tool (BLAST), or the like.

The compound of the present invention binds to the interaction region by an action of a van der Waals force with at least 13, and preferably 14 or more, 15 or more, 16 or more, 17 or more, or 18 or more amino acid residues among the predetermined (28) amino acid residues constituting the interaction region.

In an embodiment of the present invention, the compound of the present invention binds to the interaction region by an action of a van der Waals force with at least 13, and preferably 14 or more, 15 or more, 16 or more, 17 or more, or 18 or more amino acid residues among 19 amino acid residues of Asp121, Pro122, Asp123, Gln124, Asp153, Cys154, Glu155, Lys160, Pro164, Ser168, Gly169, Ser170, Trp172, Ser258, Cys259, Asp262, Cys263, Leu264, and His266, among the predetermined (28) amino acid residues constituting the interaction region.

The expression of "the van der Waals force acts" in the present invention means that at least one atom included in the compound of the present invention and at least one atom included in the amino acid residue are distant from each other within 3.5 Å in the interaction region. When such a result is obtained using a simulator (for example, software "ASEDock") having a molecular structure used in in silico analysis, it can be considered that "the van der Waals force acts". Those skilled in the art can estimate the van der Waals force and other non-covalent interactions that are generated between a target compound and the amino acid residues of IL-17RA (in the interaction region) by "ASEDock" or other software (in silico analysis means) under appropriate conditions.

Further, it is preferable that a non-covalent interaction other than the van der Waals force (in the present specification, simply referred to as an "intermolecular interaction") acts between the compound of the present invention and at least one of the predetermined amino acid residues constituting the interaction region. Examples of the intermolecular interaction can include an ionic bonding, a hydrogen bonding, a hydrophobic interaction, an OH-π interaction, a cation-π interaction, a CH-π interaction (also is a hydrophobic interaction), and a π-π interaction (also is a hydrophobic interaction). The number of amino acid residues at which the intermolecular interaction acts is preferably 2 or more, and more preferably 3 or more. The intermolecular interaction may be one kind or two kinds or more.

Those skilled in the art can understand that, what kind of atom, atomic group, and other molecular structures the compound of the present invention and the predetermined amino acid residues constituting the interaction region basically have allow each of the intermolecular interactions to act, by taking into consideration the common technical knowledge and known matters together with the disclosure in the present specification. In this case, in silico analysis can be appropriately utilized. In addition, those skilled in the art can exclude compounds not having IL-17A inhibitory activity at a desired level among compounds having a molecular structure designed based on such a basic principle to select a compound that can be used in the present invention, thereby implementing the invention without excessive trial and error.

In an embodiment of the present invention, at least one intermolecular interaction (the non-covalent interaction other than the van der Waals force) selected from the group consisting of an ionic bonding, a hydrogen bonding, a CH-π interaction, a cation-π interaction, and a hydrophobic interaction acts between the compound of the present invention and the predetermined amino acid residues constituting the interaction region, preferably, at least one amino acid selected from the group consisting of Asp121, Pro122, Asp123, Gln124, Asp153, Cys154, Glu155, Lys160, Ser168, Ser170, Ser258, Asp262, Leu264, and His266. More preferably, at least one intermolecular interaction (the non-covalent interaction other than the van der Waals force) selected from the group consisting of an ionic bonding, a hydrogen bonding, a CH-π interaction, and a hydrophobic interaction acts between the compound of the present invention and at least one amino acid selected from the group consisting of Pro122, Cys154, Lys160, Ser170, and Leu264.

In such an embodiment, in a case where the predetermined intermolecular interaction acts between the compound of the present invention and at least one amino acid residue selected from the group consisting of Asp121, Gln124, Ser168, and Asp262 that are targeted by the compound described in Non-Patent Document 3, it is more preferable that the predetermined intermolecular interaction acts between the compound of the present invention and amino acid residues other than the amino acid residues described above among the predetermined amino acid residues constituting the interaction region, that is, at least one amino acid selected from the group consisting of Pro122, Asp123, Asp153, Cys154, Glu155, Lys160, Ser170, Ser258, Leu264, and His266.

An "IL-17 activity inhibitor" provided in another aspect of the present invention contains a compound represented by General Formula (I) (compound (I), a compound according to a second embodiment of the present invention), or a pharmaceutically acceptable salt, solvate, or prodrug thereof.
[Chem. 3]

A-L¹-B-L²-C-L³-D (I)

Details of each symbol in General Formula (I) are as follows.

A represents (A1) a C₃₋₁₀ cycloalkyl group which is optionally substituted, (A2) a C₃₋₁₀ cycloalkenyl group which is optionally substituted, (A3) a 6- to 14-membered aromatic hydrocarbon cyclic group (aryl group) which is optionally substituted, (A4) a 5- to 14-membered aromatic heterocyclic group which is optionally substituted, (A5) a 3- to 14-membered non-aromatic heterocyclic group which is optionally substituted, or (A6) a C₄₋₆ alkyl group which is optionally substituted.

L¹ represents (L¹1) a single bond, (L¹2) a C₁₋₃ alkylene group, which is optionally linked to a divalent group (amide bond) derived from a carbamoyl group and/or is optionally linked to an ether bond or a thioether bond, (L¹3) a divalent group (amide bond) derived from a carbamoyl group, which is optionally linked to a divalent group derived from an amino group, (L¹4) a sulfonyl group, or (L¹5) a C₁₋₃ alkenylene group (a carbon-carbon double bond is optionally formed with a carbon atom of B or C adjacent to L²).

B represents (B1) a divalent group (amide bond) derived from a carbamoyl group, which is optionally substituted and/or is optionally linked to a divalent group derived from a C₁₋₃ alkyl-carbonyl group, (B2) a divalent group derived from a 5- to 14-membered aromatic heterocyclic ring, which is optionally substituted, (B3) a divalent group derived from a 3- to 14-membered non-aromatic heterocyclic ring, which is optionally substituted, (B4) a C₃₋₁₀ cycloalkyl group which is optionally substituted, (B5) a C₃₋₁₀ cycloalkenyl group which is optionally substituted, (B6) a 6- to 14-membered aromatic hydrocarbon cyclic group (aryl group) which is optionally substituted, (B7) an ester bond or a thioester bond, or (B8) a keto group or a thioketo group.

L² represents (L²1) a single bond, (L²2) a C₁₋₆ alkylene group, or (L²3) a C₁₋₃ alkenylene group (a carbon-carbon double bond is optionally formed with a carbon atom of B or C adjacent to L²).

C represents (C1) a divalent group (amide bond) derived from a carbamoyl group, which is optionally N-substituted, (C2) a divalent group derived from a 5- to 14-membered aromatic heterocyclic ring, which is optionally substituted, (C3) a divalent group derived from a 3- to 14-membered non-aromatic heterocyclic ring, which is optionally substituted, (C4) a C₃₋₁₀ cycloalkyl group which is optionally substituted, (C5) a C₃₋₁₀ cycloalkenyl group which is optionally substituted, (C6) a 6- to 14-membered aromatic hydrocarbon cyclic group (aryl group) which is optionally substituted, or (C7) an ester bond or a thioester bond.

L³ represents (L³1) a single bond, (L³2) a C₁₋₃ alkylene group, which is optionally linked to a divalent group (amide bond) derived from a carbamoyl group and/or a divalent group (-N=) derived from an imino group and/or is optionally substituted, (L³3) an ether bond or a thioether bond which is optionally linked to a C₁₋₃ alkenylene group, or (L³4) a divalent group (amide bond) derived from a carbamoyl group, which is optionally linked to a divalent group derived from an amino group.

D represents (D1) a C₃₋₁₀ cycloalkyl group which is optionally substituted, (D2) a C₃₋₁₀ cycloalkenyl group which is optionally substituted, (D3) a 6- to 14-membered aromatic hydrocarbon cyclic group (aryl group) which is optionally substituted, (D4) a 5- to 14-membered aromatic heterocyclic group which is optionally substituted, (D5) a 3- to 14-membered non-aromatic heterocyclic group which is optionally substituted, or (D6) a C₁₋₃ alkyl group which is optionally substituted.

In an embodiment of the present invention, the compound of the present invention is represented by General Formula (I) (the requirement for the second embodiment is satisfied), and has a van der Waals force or a non-covalent interaction other than the van der Waals force with the "predetermined amino acid residues constituting the interaction region" as described in the present specification (the requirement for the first embodiment is satisfied). Meanwhile, the compound of the present invention may satisfy the requirement for the second embodiment, but may not satisfy the requirement for the first embodiment, or the compound of the present invention may satisfy the requirement for the first embodiment, but may not satisfy the requirement for the second embodiment, as long as the action effect of the present invention are achieved.

In General Formula (I), preferred specific examples of A, L¹, B, L², C, L³, and D can include those represented by a structural formula of any one of the compounds (1) to (36) of the present invention, and more preferred specific examples thereof can include those represented by a structural formula of any one of the compounds (1), (2), (5), (9), and (11) of the present invention.

It should be noted that, among the compounds (1) to (36) shown in Table 2 below, the compounds (18), (32), and (33) are not compounds that completely comply with the definition of the General Formula (I).

In the compound (18), a specific ring structure (spiro ring) (having a substituent) is formed by integration of A, L¹, and B, but the definition of General Formula (I) can be applied to L², C, L³, and D.

In the compound (32), a specific ring structure (having a substituent) is formed by integration of A, L¹, and B, but the definition of General Formula (I) can be applied to L², C, L³, and D.

In the compound (33), a specific structure (an alkylene group) is formed by integration of L¹, B, and L², but the definition of General Formula (I) can be applied to A, C, L³, and D.

In an embodiment of the present invention, the compound (I) has at least a site at which a hydrogen bonding or CH-π interaction with Cys154 acts. The site is preferably at least one location selected from the group consisting of the sites L², A, B, and C in the compound (I). For example, two locations of L² and B are preferably included, or two locations of B and C are preferably included. A (δ+) hydrogen atom serving as a proton donor may be included in the compound (I) or in Cys154.

For example, as a site at which a hydrogen bonding or CH-π interaction with Cys154 acts, the compound (I) may have at least one of:
the site A which is (A6) having a group serving as a donor or an acceptor of a hydrogen atom, optionally as a substituent;
the site L¹ which is (L¹2) having a group serving as a donor or an acceptor of a hydrogen atom, optionally as a substituent;
the site B which is (B1) or (B3) having a group serving as a donor or an acceptor of a hydrogen atom, optionally as a substituent;
the site C which is (C1), (C2), (C3), (C6), or (C7) having a group serving as a donor or an acceptor of a hydrogen atom, optionally as a substituent;
the site L¹ which is (L¹2) or (L¹4) having a group serving as a donor or an acceptor of a hydrogen atom, optionally as a substituent;
the site L² which is (L²2) having a group serving as a donor or an acceptor of a hydrogen atom, optionally as a substituent;
the site L³ which is (L³2) having a group serving as a donor or an acceptor of a hydrogen atom, optionally as a substituent; and
the site C which is (C2) or (C6) having a π electron (which may be included in some rings in a non-aromatic condensed ring as a whole).

Specific examples of the hydrogen bonding that acts between the compound (I) and Cys154 can include:
a hydrogen bonding between a nitrogen atom (lone electron pair) of -NH-, an oxygen atom (lone electron pair) of -CO-, or a sulfur atom (lone electron pair) of -S-included in the site B, C, or L¹ and a hydrogen atom of -SH included in a side chain of Cys154 (for example, the compound (1), (2), (5), (9), (11), or (36));
a hydrogen bonding between an oxygen atom (lone electron pair) of =O included in the site B, L¹, or L³ and a hydrogen atom of -SH included in the side chain of Cys154 (for example, the compound (7), (14), (15), (24), (25), (26), (31), or (35));
a hydrogen bonding between a hydrogen atom of -OH included in the site A and a sulfur atom (lone electron pair) of -SH included in the side chain of Cys154 (for example, the compound (11)); and
a hydrogen bonding between a hydrogen atom of =CH-, -CH₂-, or -CH(R)-included in the site B, L¹, or L² or a hydrogen atom of -NH- included in the site B and a sulfur atom (lone electron pair) of -SH included in the side chain of Cys154 (for example, the compound (6), (8), (10), (16), (27), or (35)).
In addition, specific examples of the CH-π interaction that acts between the compound (I) and Cys154 can include:
   a CH-π interaction between a π electron of an aromatic heterocyclic ring (C2) or an aromatic hydrocarbon group (C6) included in the site C and a hydrogen atom of - SH included in the side chain of Cys154 (for example, the compound (11), (22), (23), or (27)).

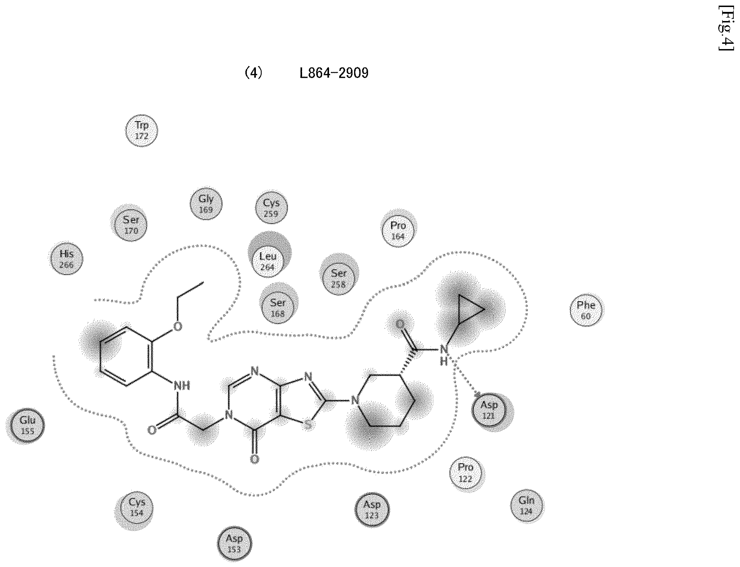

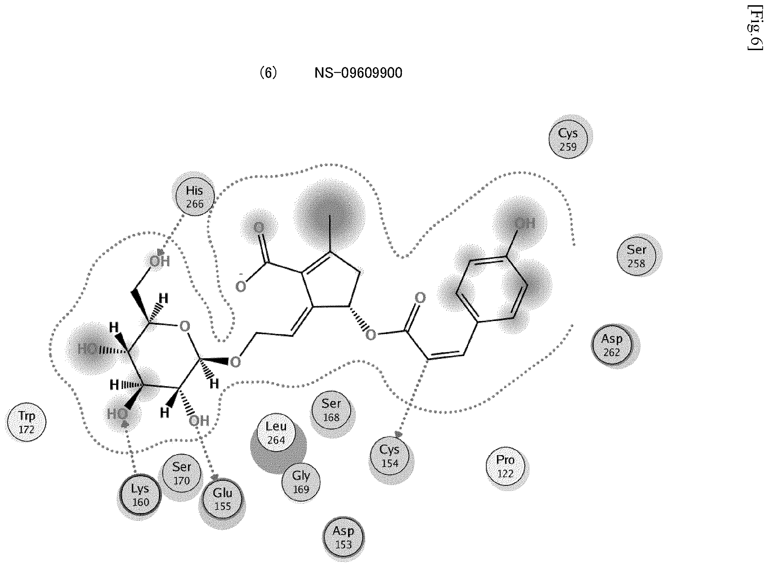

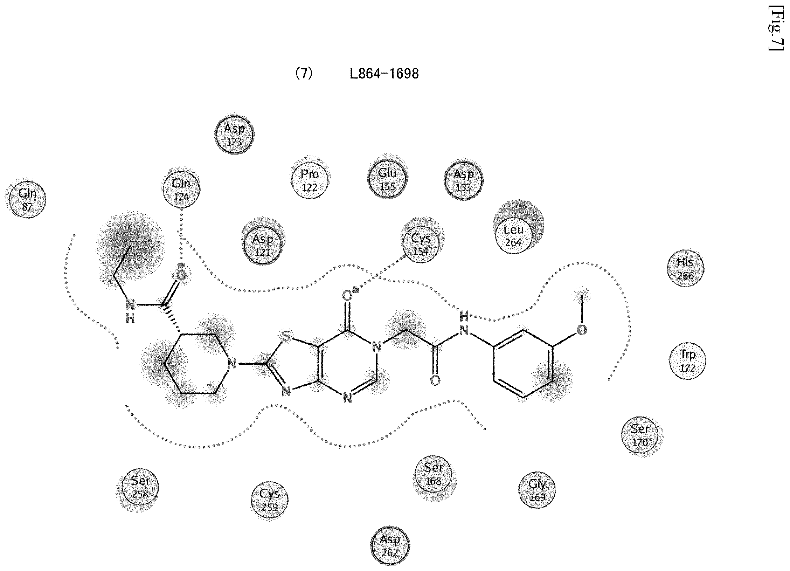

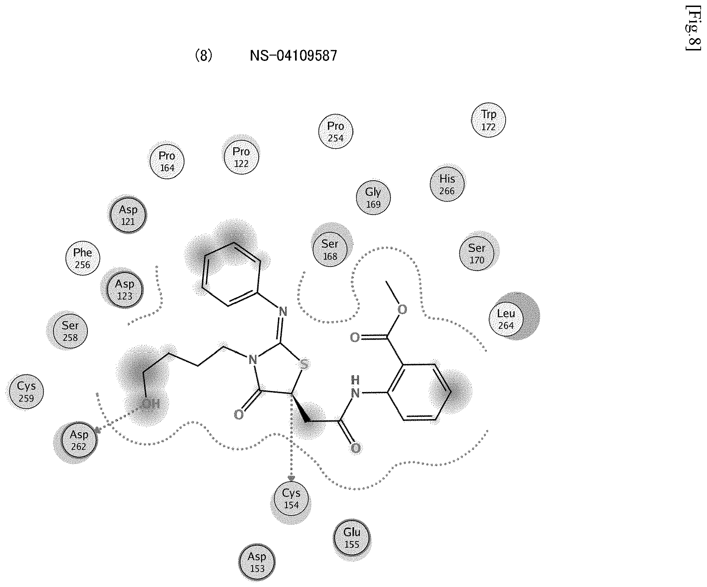

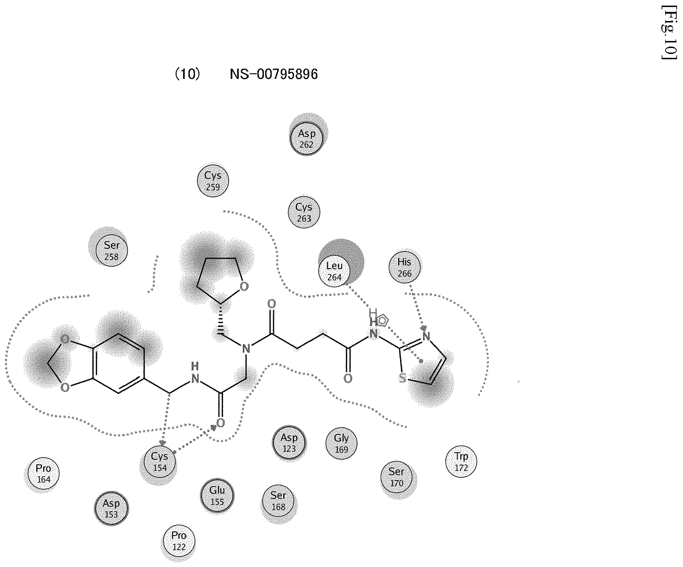

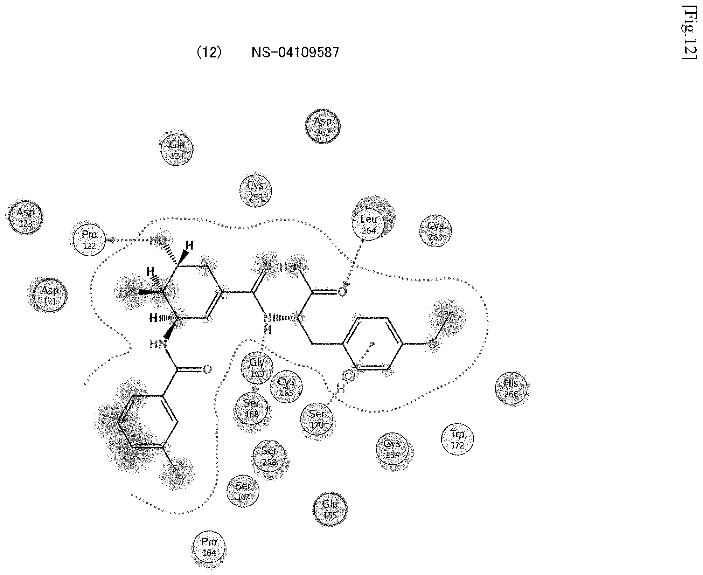

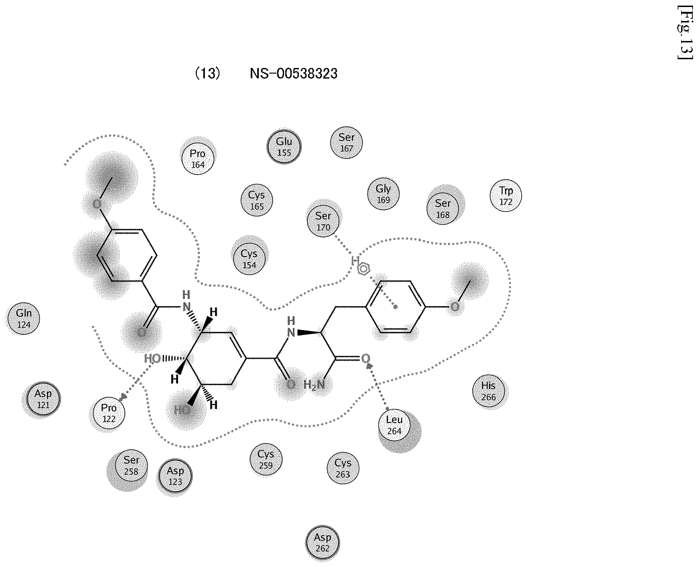

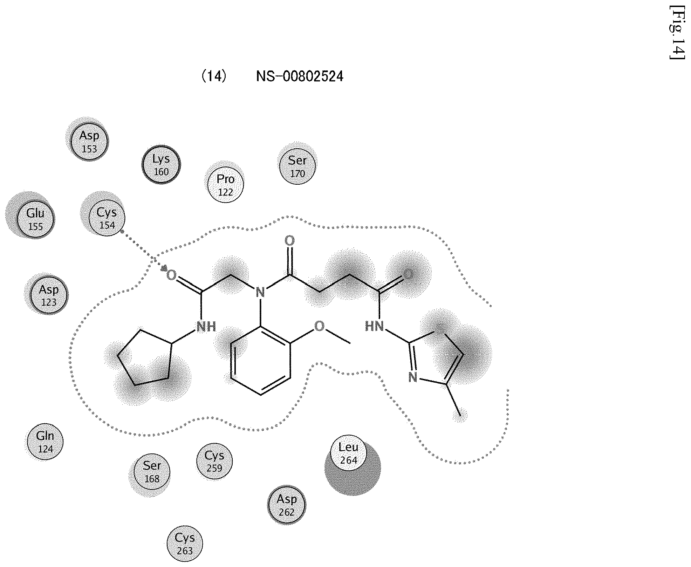

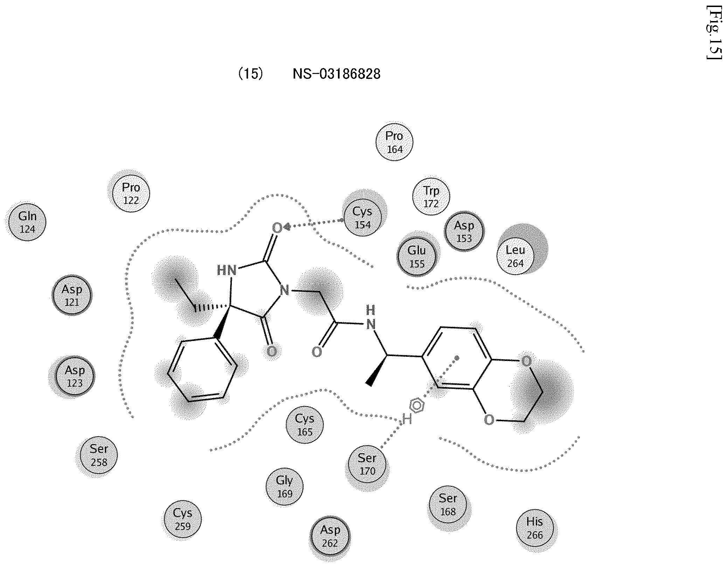

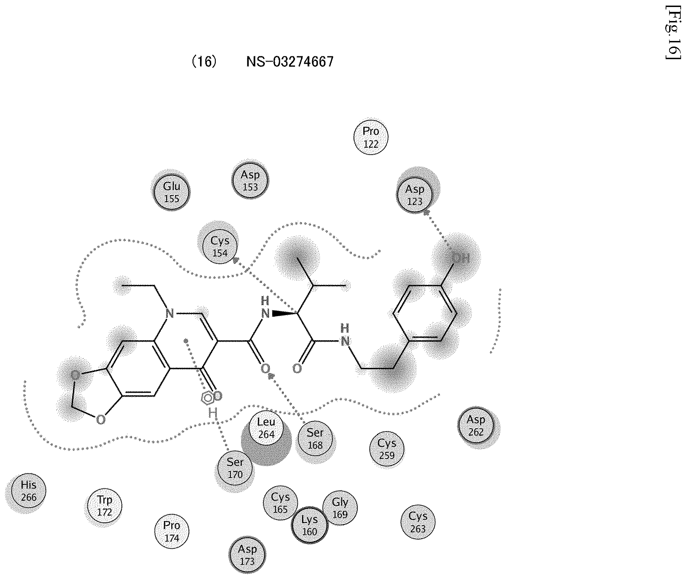

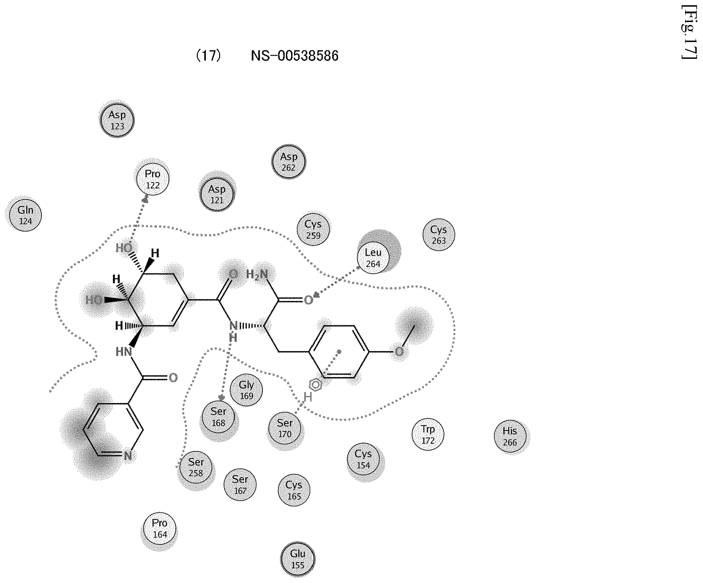

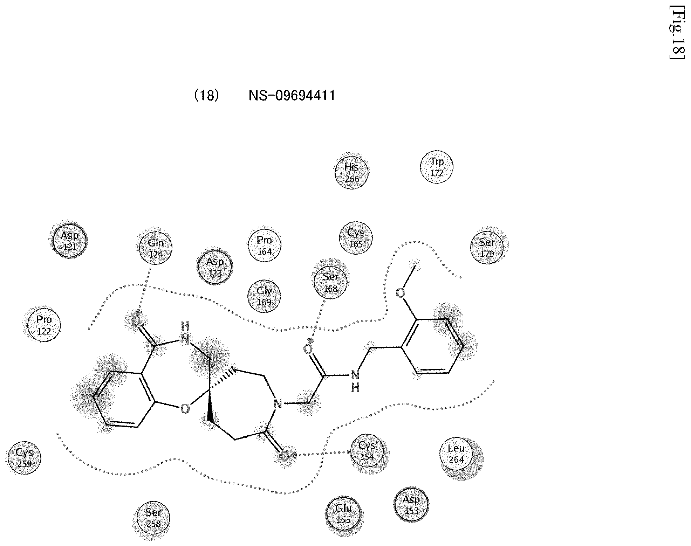

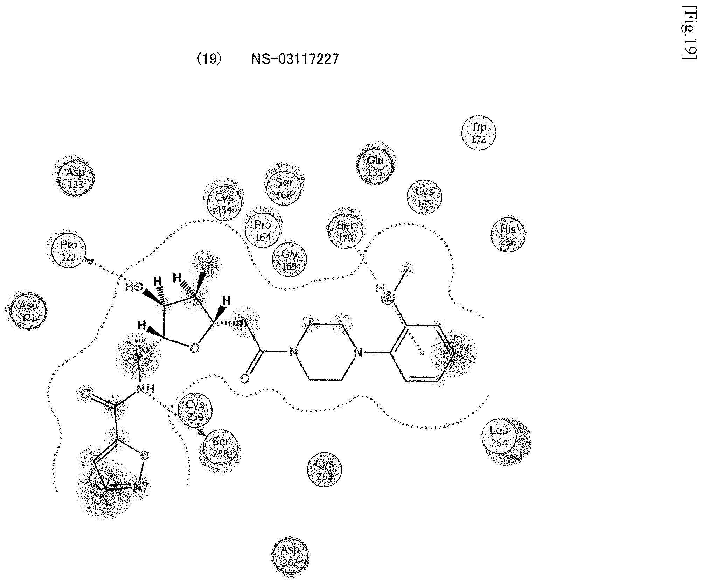

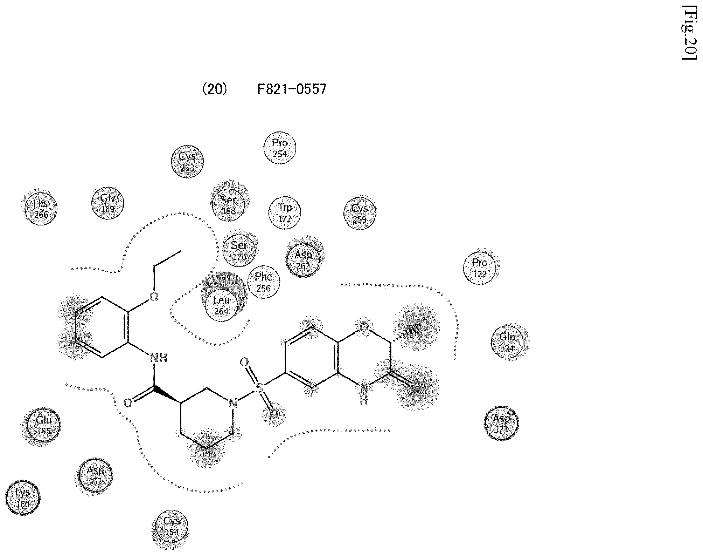

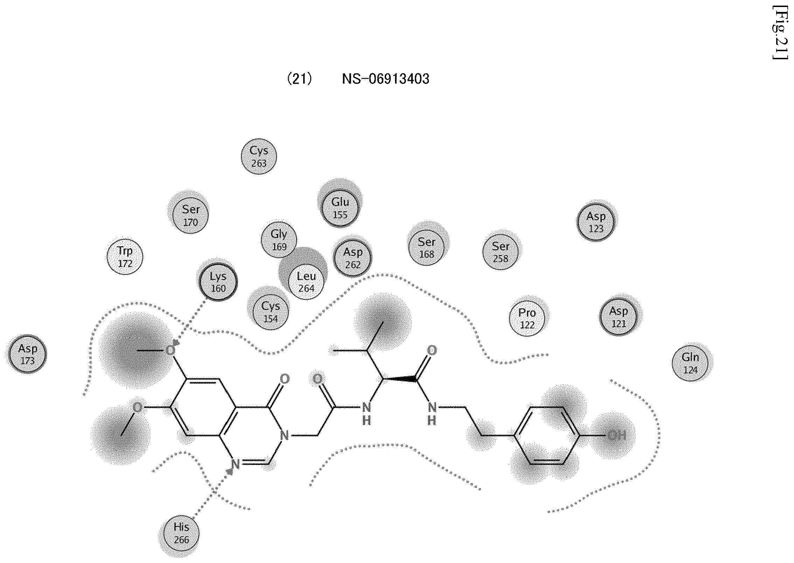

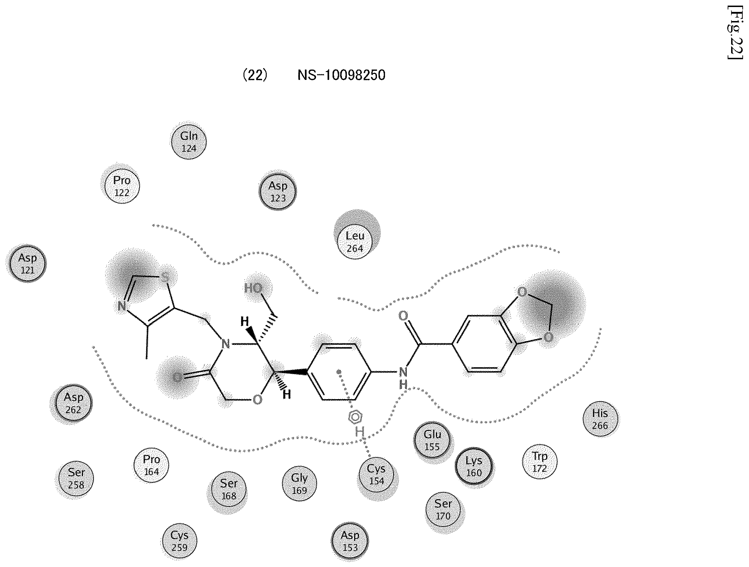

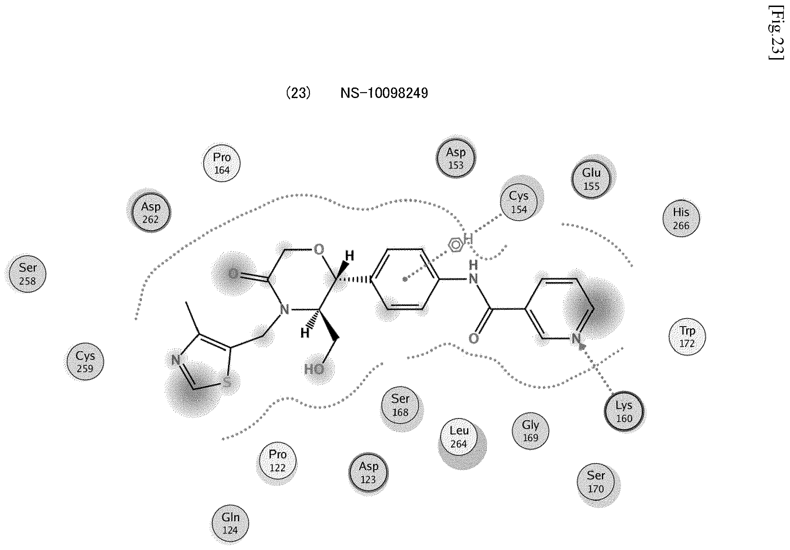

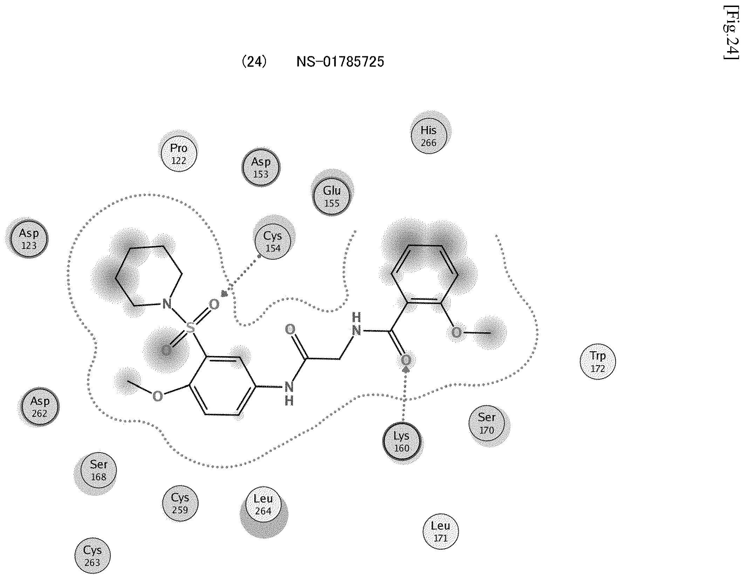

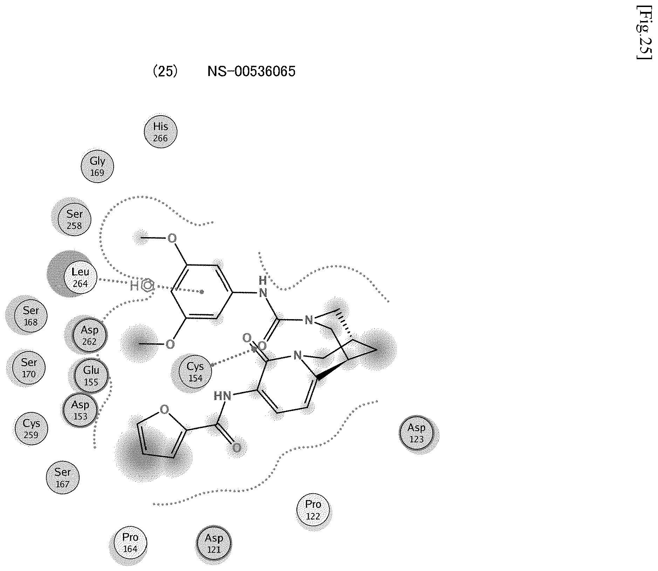

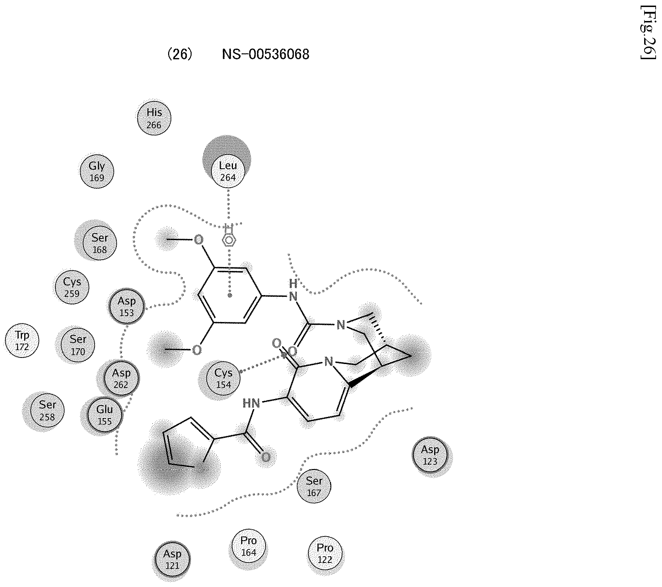

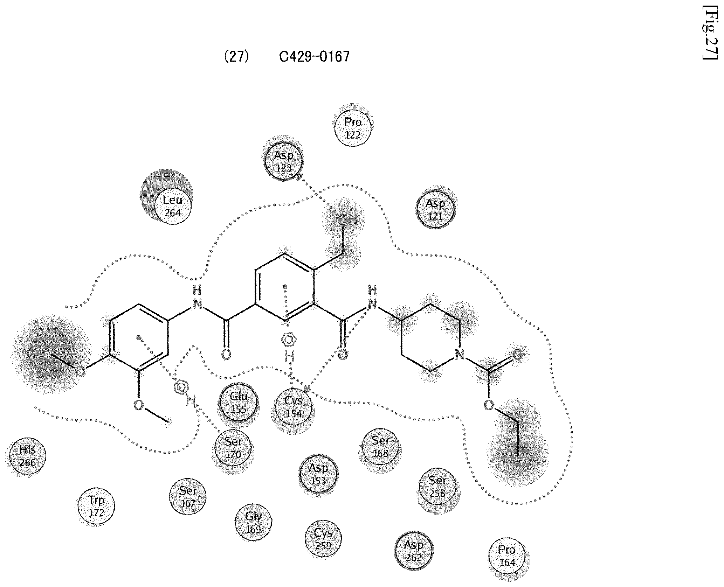

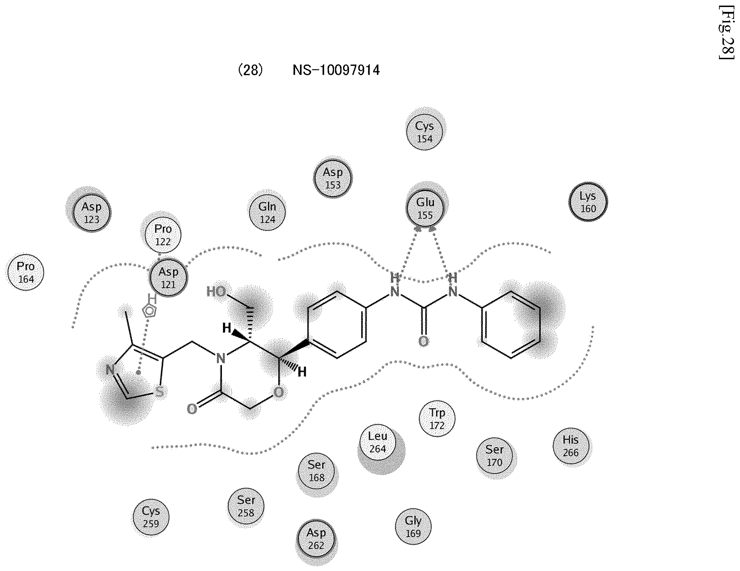

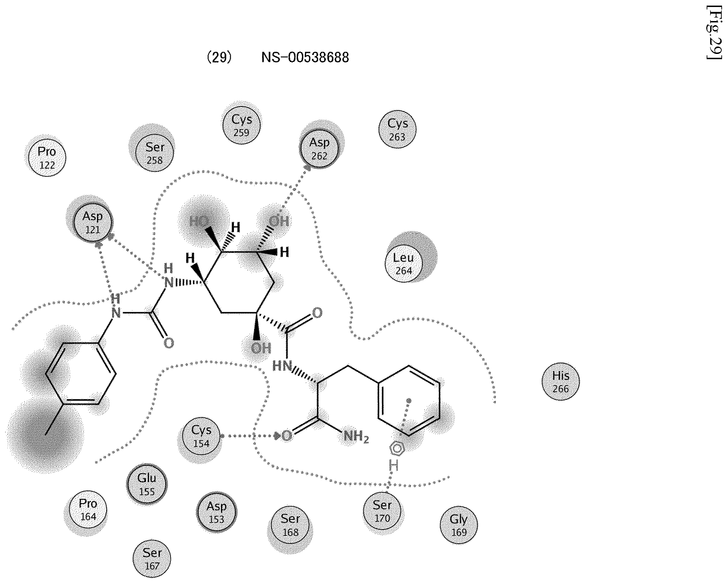

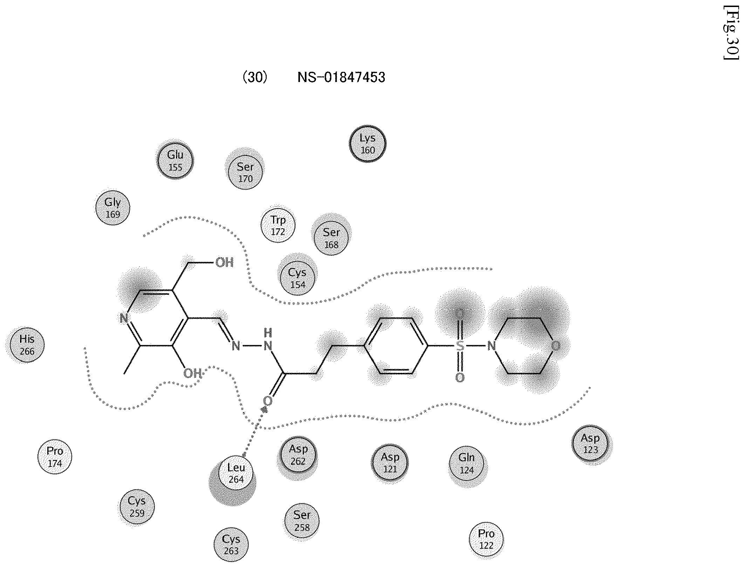

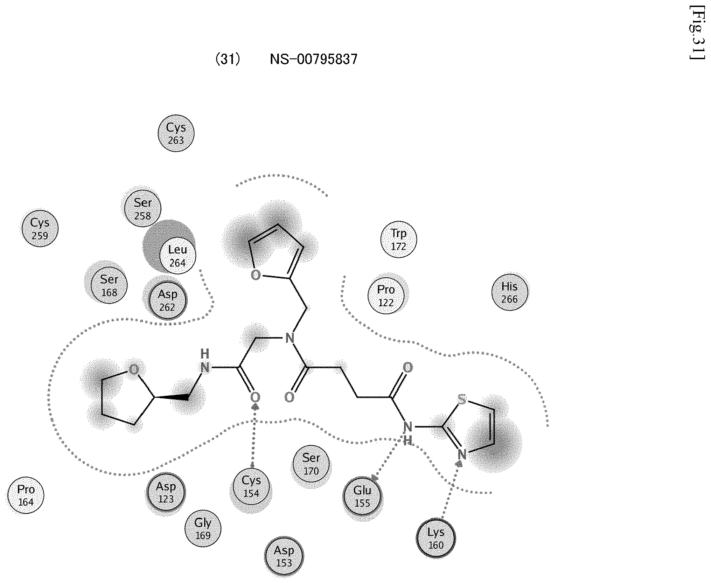

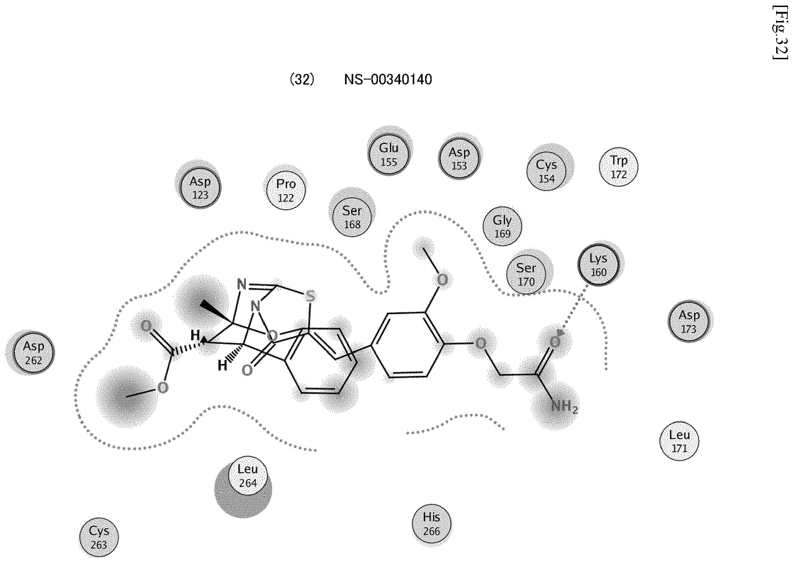

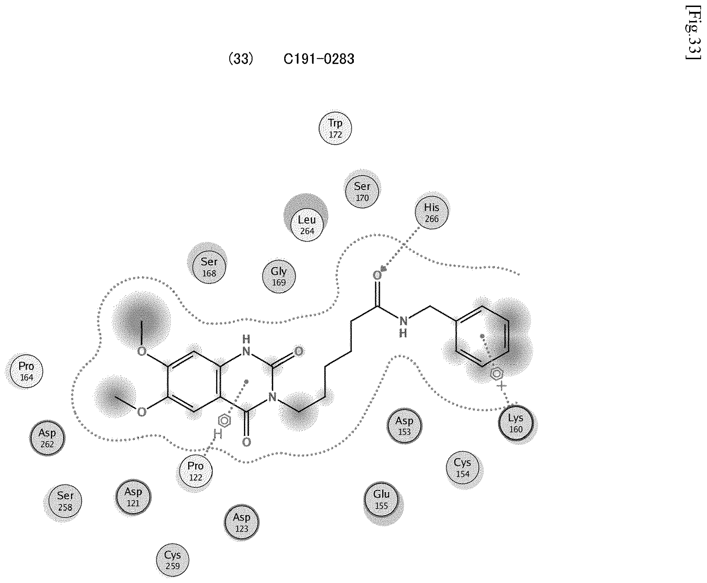

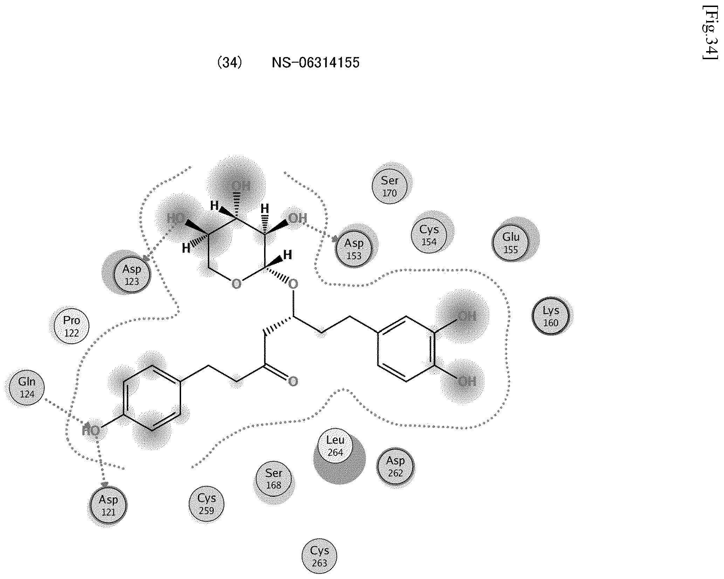

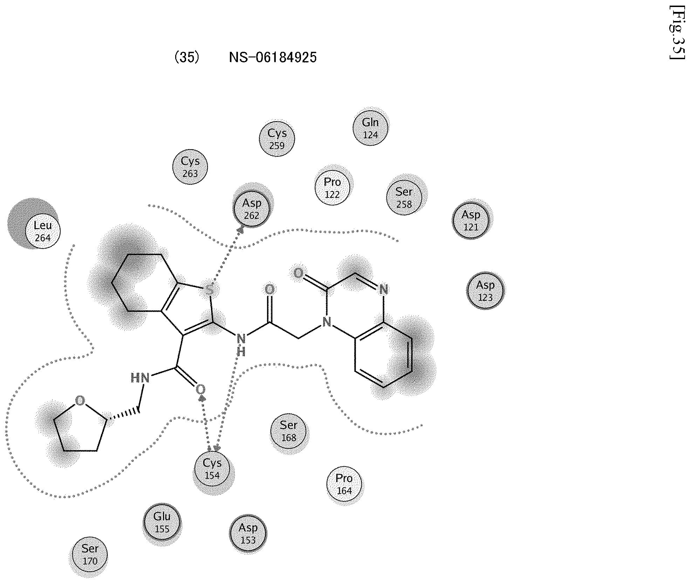

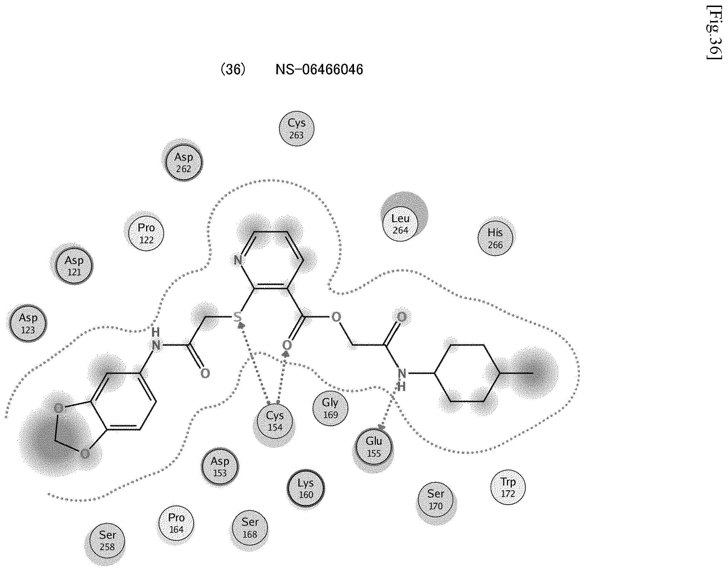

The hydrogen bonding or CH-π interaction that acts between the compound (I) and Cys154 may be an intermolecular interaction illustrated in Figs. 2 to 36 in addition to the above interactions.

The compound (I) may have a site at which a hydrogen bonding, a CH-π interaction, an ionic bonding, or other intermolecular interactions with an amino acid residue other than Cys154 among the predetermined amino acid residues constituting the interaction region are generated. Representative examples of the intermolecular interaction can include a site at which a hydrogen bonding with Asp121 is generated, a site at which a CH-π interaction with Pro122 is generated, a site at which a CH-π interaction with Asp123 is generated, a site at which an ionic bonding or hydrogen bonding with Lys160 is generated, a site at which a CH-π interaction with Ser170 is generated, and other intermolecular interactions illustrated in Figs. 2 to 36.

Representative examples of the site at which the hydrogen bonding with Asp121 is generated can include the site A which is (A6), for example, a substituted C₄₋₆ alkyl group included in the compound (9). In the embodiment, it is preferable that a substituent of the C₄₋₆ alkyl group has an atom serving as a donor or an acceptor for forming a hydrogen bond with an asparagine residue, and examples thereof can include an amino group which is optionally substituted. In addition, among (A1) to (A6) defined as the site A, in addition to the substituted C₄₋₆ alkyl group (corresponding to A6), for example, each of groups of (A1) to (A5) including a group having an atom serving as a donor or an acceptor in a hydrogen bonding as a substituent, such as -NH-of (A4) included in the compound (4), -NH- of (L¹2) included in the compound (29), and -OH of (A3) included in the compound (34) can be defined as a site at which the hydrogen bonding with Asp 121 is generated.

Representative examples of the site at which the CH-π interaction with Pro122 is generated can include the site A which is (A4), for example, a divalent group that is included in the compound (1) or (28) and is derived from an aromatic heterocyclic ring, which is optionally substituted, or (A5), for example, a divalent group that is included in the compound (33) and is derived from a non-aromatic heterocyclic ring, which is optionally substituted (where an aromatic ring (π electron) is included as a part of a condensed ring). In the embodiment, it is preferable that an aromatic heterocyclic ring or a non-aromatic heterocyclic ring is a group having a π electron that can form a CH-π interaction with a proline residue. In addition, among (A1) to (A6) defined as the site A, in addition to (A4) and (A5), for example, a cyclic group of (A3) having a π electron can be defined as a site at which the CH-π interaction with Pro122 is generated.

A hydrogen bonding may be generated between the compound (I) of the present invention and Pro122, and examples of a site at which such a hydrogen bonding is generated can include the site B which is (B5) included in the compound (12), (13), or (17), that is, a divalent group derived from a substituted cycloalkenyl group, or (B3) included in the compound (19), that is, a divalent group derived from a substituted non-aromatic heterocyclic ring. In the embodiment, it is preferable that a substituent of the cycloalkenyl group or the non-aromatic heterocyclic ring has an atom serving as a donor or an acceptor for forming a hydrogen bond with a proline residue, and examples thereof can include a hydroxyl group. In addition, among (B1) to (B8) defined as the site B, in addition to (B3) and (B5), for example, a group of (B1), (B2), (B4), (B6) to (B8) including a group having an atom serving as a donor or an acceptor in a hydrogen bonding as a substituent can be defined as a site at which a hydrogen bonding with Pro122 is generated.

Representative examples of the site at which the CH-π interaction with Asp 123 is generated can include the site A which is (A5), for example, a non-aromatic heterocyclic group which is included in the compound (2) and is optionally substituted (where an aromatic ring (π electron) is included as a part of a condensed ring). In the embodiment, the non-aromatic heterocyclic group is optionally a group having a π electron that can form a CH-π interaction with an aspartic acid residue, and examples thereof can include a condensed ring of an aromatic ring and a non-aromatic ring (although it is non-aromatic as a whole, since the π electron is included in an aromatic ring part, the CH-π interaction with the aspartic acid residue can be formed at the part). In addition, among (A1) to (A6) defined as the site A, in addition to (A5), for example, a cyclic group of (A3) or (A4) having a π electron can be defined as a site at which the CH-π interaction with Asp 123 is generated.

A hydrogen bonding may be generated between the compound (I) of the present invention and Asp 123, and examples of the site at which such a hydrogen bonding is generated can include the site C which is (C6) included in the compound (27), that is, an aromatic hydrocarbon group which is optionally substituted, or (C8) included in the compound (34), that is, a methylene group substituted with a hydroxyl group, which is optionally substituted. In the embodiment, it is preferable that a substituent of the aromatic hydrocarbon group or the methylene group has an atom serving as a donor or an acceptor for forming a hydrogen bond with a proline residue, and examples thereof can include a hydroxyl group (or a substituent having a hydroxyl group at a terminal thereof). In addition, among (C1) to (C8) defined as the site C, in addition to (C6) and (C8), for example, a group of (C1) to (C5), or (C7) including a group having an atom serving as a donor or an acceptor in a hydrogen bonding as a substituent can be defined as a site at which a hydrogen bonding with Pro122 is generated.

Representative examples of the site at which an ionic bonding or hydrogen bonding with Lys160 is generated can include the site D which is (D1) included in the compound (1), that is, a substituted cycloalkyl group, (D3) included in the compound (5), that is, a substituted aromatic hydrocarbon cyclic group, (D5) included in the compound (6), that is, a substituted non-aromatic heterocyclic group, (D4) included in the compound (21), (23), or (31), that is, an aromatic heterocyclic group which is optionally substituted, or (D6) included in the compound (32), that is an alkyl group which is optionally substituted, or a substituted alkyl group, and (L³2) included in the compound (24), that is, an alkylene group which is optionally linked to a predetermined group or is optionally substituted with a predetermined group. In the embodiment, it is preferable that a substituent of the cycloalkyl group or the aromatic hydrocarbon cyclic group has an atom producing an anion for forming an ionic bonding with a lysine residue or an atom serving as a donor or an acceptor for forming a hydrogen bond with a lysine residue. Examples of the former can include a carboxyl group, and examples of the latter can include a keto group (an oxo group). In addition, among (D1) to (D6) defined as the site D, in addition to (D1), (D3), and (D5), a group of (D2), (D4), or (D6) having such a substituent can be defined as a site at which the ionic bonding or hydrogen bonding with Lys160 is generated.

A cation-π interaction may be generated between the compound (I) of the present invention and Lys160, and examples of a site at which such a cation-π interaction is generated can include the site D which is (D3) included in the compound (33), that is, an aromatic hydrocarbon group (phenyl group) which is optionally substituted. In the embodiment, the aromatic hydrocarbon group is a group having a π electron that can form a cation-π interaction with a lysine residue. In addition, among (D1) to (D8) defined as the site D, in addition to (D3), (D4) having a π electron, or (D5) of the embodiment which is non-aromatic as a whole but has a π electron in an aromatic ring part thereof can be defined as a site at which the cation-π interaction with Lys160 is generated.

Examples of a site at which a CH-π interaction with Ser170 can include the site D which is (D3) included in the compound (2), (12), (13), (17), (19), (27), or (29), that is, an aromatic hydrocarbon group which is optionally substituted, or (D5) included in the compound (9), (15), or (16), that is, a non-aromatic heterocyclic group which is optionally substituted (where an aromatic ring (π electron) is included as a part of a condensed ring). In the embodiment, the aromatic hydrocarbon group may be a group having a π electron that can form a CH-π interaction with a serine residue. In addition, in the embodiment, the non-aromatic heterocyclic group may be a group having a π electron that can form a CH-π interaction with a serine residue, and examples thereof can include a condensed ring of an aromatic ring and a non-aromatic ring (although it is non-aromatic as a whole, since the π electron is included in an aromatic ring part, the CH-π interaction with the serine residue can be formed at the part). In addition, among (D1) to (D6) defined as the site D, in addition to (D3) and (D5), for example, a cyclic group of (D4) having a π electron can be defined as a site at which the CH-π interaction with Ser170 is generated.

In addition, the compound (I) may have at least one selected from the group consisting of a hydrogen bonding with Gln124, a hydrogen bonding with Asp153, a hydrogen bonding with Glu155, a hydrogen bonding with Ser168, a hydrogen bonding with Ser258, a hydrogen bonding with Asp262, a hydrogen bonding or CH-π interaction with Leu264, and a hydrogen bonding with His266. A site at which a predetermined interaction with the predetermined amino acid residue is generated can be defined in the same manner as in the above embodiment from the drawing or the tables.

The compound (I) may include a stereoisomer, that is, an enantiomer and/or a diastereomer (a stereoisomer other than an enantiomer). In the present invention, as the compound (I), a mixture of stereoisomers (for example, a racemic form which is a mixture of enantiomers) may be used, and a purified product in which purity of a specific stereoisomer useful for pharmacological activity is increased, for example, a purified product ideally substantially formed of only the stereoisomer whose purity is 90% or higher, preferably 95% or higher, and more preferably 99% or higher, may be used.

The compound (I) may include a tautomer. Examples of the tautomer can include a keto-enol tautomer having the following interconvertible structures. Regardless of a structure represented by General Formula (I), all the tautomers can be included in the compound (I).

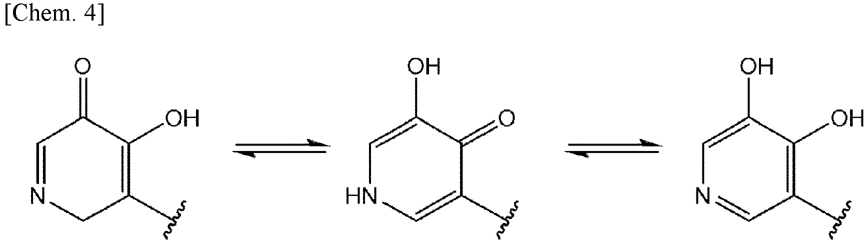

Each site of the compound (I) may be ionized under a condition in which the compound (I) is used, typically, under a physiological property. For example, a carboxyl group (-COOH) may be present in a carboxylate ion (-COO⁻) state.

In an embodiment of the present invention, the compound (I) is any one of the compounds (1) to (36) shown in Table 2. The compound (3) represents a racemic form which is a mixture of an S form and an R form, and the compound (1) represents only the S form. The smaller the docking score "GBVIWSA_dG" (negative value, unit: kcal/mol), the more stable the binding of the compound to IL-17RA. Regarding the "total number" indicated in the parentheses in "Number of amino acid residues at which non-covalent interaction other than van der Waals force acts", for example, in a case where two non-covalent interactions (intermolecular interactions) other than the van der Waals force act with respect to one amino acid residue, the total number is "2", which represents "a total number of non-covalent interactions (intermolecular interactions) other than the van der Waals force". Among compounds (1) to (36) excluding compound (3), those which interact among predetermined amino acid residues constituting the interaction region are shown in Table 3.

For reference, in a case where the cyanidin compound (A18, see Chem. 1) described in Non-Patent Document 3 is disposed to interact with Asp121, Gln124, Serl68, or Asp262 as described in Non-Patent Document 3, a GBVIWSA_dG value is - 5.3894 kcal/mol which is larger than those of any one of the compounds (1) to (36) shown in the following table (the maximum is -7.5007 kcal/mol of the compound (36)), which suggests that binding stability is inferior to that of the compound of the present invention.

**[Table 2-1]**

| Comp. No. | Structural Formulas | Database registration name | GBVIWSA_dG | Number of amino acid residues on which van der Waals force works | Number of amino acid residues for non-cov alent interactions oth er than van der Waals force (total number in parentheses) | Remarks |
|---|---|---|---|---|---|---|
| (1) | 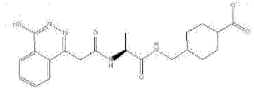 | STOCK6S-2446 0 | -7.9600 | 15 | 3 (4) | S comp. Example Fig2 |
| (2) | 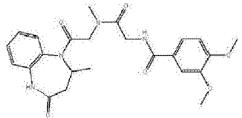 | NS-03822525 PB203263256 | -7.9519 | 19 | 3 | Example Fig3 |
| (3) | 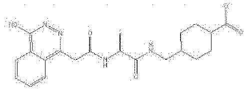 | NS-03973940 STK630921 | -7.9359 | - | - | Racemic body of (1) |
| (4) | 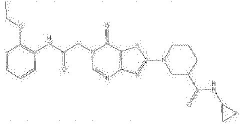 | L864-2909 | -7.8579 | 17 | 1 | Fig4 |
| (5) | 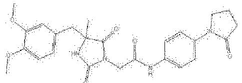 | NS-03184715 Z9215 | -7.7817 | 18 | 2 | Example Fig5 |

**[Table 2-2]**

| | | | | | | |
|---|---|---|---|---|---|---|
| (6) | 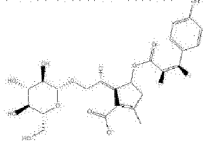 | NS-09609900 | -7.7486 | 14 | 4 | Fig6 |
| (7) | 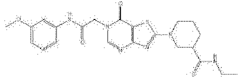 | L864-1698 STL093038 NS-09373913 | -7.7046 | 17 | 2 | Fig7 |
| (8) | 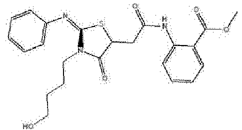 | NS-04109587 | -7.7022 | 18 | 2 | Fig8 |
| (9) | 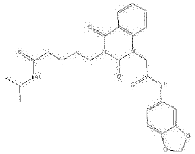 | NS-03184715 NS-06067257 F3382 F142_0244 | -7.6956 | 18 | 3 | Example Fig9 |
| (10) | 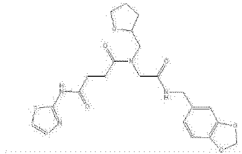 | NS-09609900 NS_00795896 ASN05396949 | -7.6893 | 16 | 3 (4) | Fig10 |

**[Table 2-3]**

| | | | | | | |
|---|---|---|---|---|---|---|
| (11) | 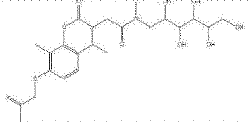 | L864-1698 STL093038 NS-09373913 NS-06910446 P2000N-53454 | -7.6880 | 17 | 1 (3) | Example Fig11 |
| (12) | 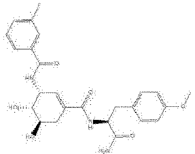 | NS-04109587 NS-00538321 | -7.6681 | 19 | 4 | Fig12 |
| (13) | 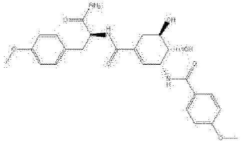 | NS-00538323 | -7.6618 | 19 | 3 | Fig13 |
| (14) | 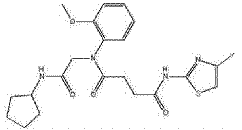 | NS-00802524 | -7.6572 | 13 | 1 | Fig14 |
| (15) | 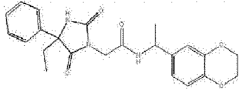 | NS-03186828 | -7.6493 | 18 | 2 | Fig15 |

**[Table 2-4]**

| | | | | | | |
|---|---|---|---|---|---|---|
| (16) | 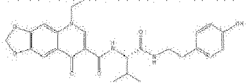 | NS-03274667 STOCK6S-2449 7 STK606232 | -7.6200 | 18 | 4 | Fig16 |
| (17) | 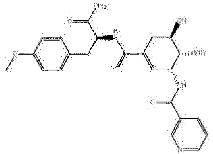 | NS-00538586 | -7.6042 | 19 | 4 | Fig11 |
| (18) | 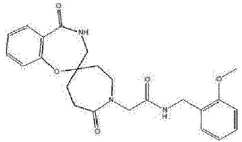 | NS-09694411 | -7.5987 | 17 | 3 | Fig18 |
| (19) | 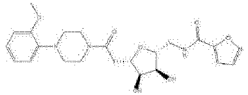 | NS-03117227 | -7.5772 | 17 | 3 | Fig19 |
| (20) | 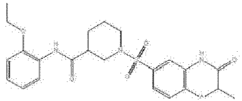 | F821-0557 | -7.5765 | 18 | 0 | Fig20 |
| (21) | 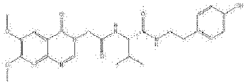 | NS-06913403 STOCK1M-72S8 1 | -7.5754 | 17 | 2 | Fig21 |

**[Table 2-5]**

| | | | | | | |
|---|---|---|---|---|---|---|
| (22) | 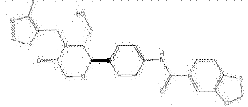 | NS-10098250 | -7.5647 | 18 | 1 | Fig22 |
| (23) | 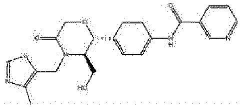 | NS-10098249 | -7.5592 | 17 | 2 | Fig23 |
| (24) | 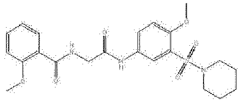 | NS-01785725 PB33391959 | -7.5562 | 15 | 2 | Fig24 |
| (25) | 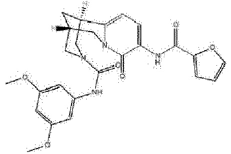 | NS-00536065 | -7.5463 | 16 | 2 | Fig25 |
| (26) | 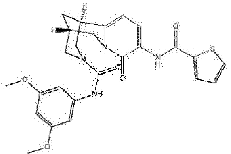 | NS-00536068 | -7.5458 | 17 | 2 | Fig26 |

**[Table 2-6]**

| | | | | | | |
|---|---|---|---|---|---|---|
| (27) | 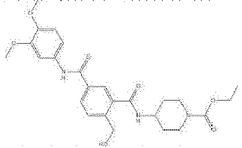 | C429-0167 | -7.5412 | 17 | 3 (4) | Fig27 |
| (28) | 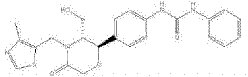 | NS-10097914 | -7.5315 | 18 | 2 (3) | Fig28 |
| (29) | 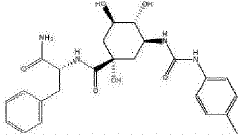 | NS-00538688 | -7.5221 | 16 | 4 (5) | Fig29 |
| (30) | 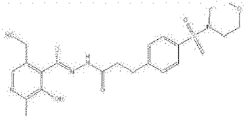 | NS-01847453 | -7.5208 | 18 | 1 | Fig30 |
| (31) | 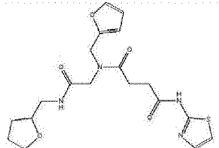 | NS-00795837 | -7.5147 | 17 | 3 | Fig31 |

**[Table 2-7]**

| | | | | | | |
|---|---|---|---|---|---|---|
| (32) | 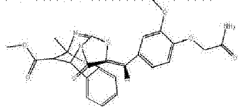 | NS-00340140 | -7.5142 | 16 | 1 | Fig32 |
| (33) | 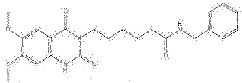 | C191-0283 | -7.5098 | 17 | 3 | Fig33 |
| (34) | 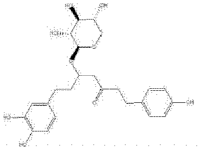 | NS-06314155 | -7.5089 | 14 | 4 | Fig34 |
| (35) | 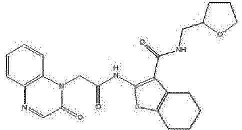 | NS-06184925 | -7.5055 | 15 | 2 (3) | Fig35 |
| (36) | 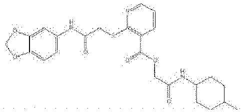 | NS-06466046 | -7.5007 | 17 | 2 (3) | Fig36 |

**[Table 3-1]**

| **Comp. No.** | **1** | **2** | **4** | **5** | **6** | **7** | **8** | **9** |
|---|---|---|---|---|---|---|---|---|
| Phe60 | | | ✔ | | | | | |
| Gln87 | | | | | | ✔ | | |
| Asp121* | ✔ | ✔ | ✔ H | ✔ | | ✔ | ✔ | ✔ H |
| Pro 122* | ✔ CH-π | ✔ | ✔ | ✔ | ✔ | ✔ | ✔ | ✔ |
| Asp123* | | ✔ CH-π | ✔ | ✔ | | ✔ | ✔ | ✔ |
| Gln124* | ✔ | ✔ | ✔ | | | ✔ H | | |
| Asp153* | | ✔ | ✔ | ✔ | ✔ | ✔ | ✔ | ✔ |
| Cys154* | ✔ H ×2 | ✔ H | ✔ | ✔ H | ✔ H | ✔ H | ✔ H | ✔ H |
| Gu155* | ✔ | ✔ | ✔ | ✔ | ✔ ion | ✔ | ✔ | ✔ |
| Lys160* | ✔ ion | | | ✔ H | ✔ H | | | ✔ |
| Pre164* | ✔ | ✔ | ✔ | ✔ | | | ✔ | ✔ |
| Cys165 | | | | | | | | |
| Ser167 | | | ✔ | | | | | ✔ |
| Ser168* | ✔ | ✔ | ✔ | ✔ | ✔ | ✔ | ✔ | ✔ |
| Gly169* | ✔ | ✔ | ✔ | ✔ | ✔ | ✔ | ✔ | ✔ |
| Sar170* | ✔ | ✔ CH-π | | ✔ | ✔ | ✔ | ✔ | ✔ CH-π |
| Leu171 | | | ✔ | | | | | |
| Trp172* | | ✔ | | ✔ | ✔ | ✔ | ✔ | ✔ |
| Asp173 | | | | | | | | |
| Pro174 | | | | | | | | |
| Pro254 | | ✔ | | | | | ✔ | |
| Phe256 | | ✔ | | | | | ✔ | |
| Ser258* | ✔ | ✔ | ✔ | ✔ | ✔ | ✔ | ✔ | ✔ |
| Cys259* | ✔ | ✔ | ✔ | ✔ | ✔ | ✔ | ✔ | ✔ |
| Asp262* | ✔ | ✔ | | ✔ | ✔ | ✔ | ✔ H | ✔ |
| Cys263* | ✔ | | | ✔ | | | | |
| Leu264* | ✔ | ✔ | ✔ | ✔ | ✔ | ✔ | ✔ | ✔ |
| His266* | | ✔ | ✔ | ✔ | ✔ H | ✔ | ✔ | ✔ |
| Of the given 28 amino acids | 15 | 19 | 17 | 18 | 14 | 17 | 18 | 18 |
| *Of the preferred 19 amino acids | 15 | 17 | 16 | 18 | 14 | 16 | 16 | 17 |

**[Table 3-2]**

| **Comp. No.** | **10** | **11** | **12** | **13** | **14** | **15** | **16** | **17** |
|---|---|---|---|---|---|---|---|---|
| Phe60 | | | | | | | | |
| Gln87 | | | | | | | | |
| Asp121* | | ✔ | ✔ | ✔ | | ✔ | | ✔ |
| Pro122* | ✔ | ✔ | ✔ H | ✔ H | ✔ | ✔ | ✔ | ✔ H |
| Aspt23* | ✔ | | ✔ | ✔ | ✔ | ✔ | ✔ H | ✔ |
| Gln124* | | ✔ | ✔ | ✔ | ✔ | ✔ | | ✔ |
| Asp153* | ✔ | ✔ | | | ✔ | ✔ | ✔ | |
| Cys154* | ✔ H ×2 | ✔ H ×2 CH-*π* | ✔ | ✔ | ✔ H | ✔ H | ✔ H | ✔ |
| Glu155* | ✔ | ✔ | ✔ | ✔ | ✔ | ✔ | ✔ | ✔ |
| Lys160* | | | | | ✔ | | ✔ | |
| Pro164* | ✔ | ✔ | ✔ | ✔ | | ✔ | | ✔ |
| Cys165 | | ✔ | ✔ | ✔ | | ✔ | ✔ | ✔ |
| Ser167 | | | ✔ | ✔ | | | | ✔ |
| Ser168* | ✔ | ✔ | ✔ H | ✔ | ✔ | ✔ | ✔ H | ✔ H |
| Gly169* | ✔ | ✔ | ✔ | ✔ | | ✔ | ✔ | ✔ |
| Ser170* | ✔ | ✔ | ✔ CH-*π* | ✔ CH-*π* | ✔ | ✔ CH-*π* | ✔ CH-*π* | ✔ CH-*π* |
| Leu171 | | | | | | | | |
| Trp172* | ✔ | ✔ | ✔ | ✔ | ✔ | ✔ | ✔ | ✔ |
| Asp172 | | | | | | | ✔ | |
| Pro174 | | | | | | | ✔ | |
| Pro254 | | | | | | | | |
| Phe256 | | | | | | | | |
| Ser258* | ✔ | ✔ | ✔ | ✔ | | ✔ | | ✔ |
| Cys259* | ✔ | ✔ | ✔ | ✔ | ✔ | ✔ | ✔ | ✔ |
| Asp262* | ✔ | ✔ | ✔ | ✔ | ✔ | ✔ | ✔ | ✔ |
| Cys263* | ✔ | | ✔ | ✔ | ✔ | | ✔ | ✔ |
| Leu264* | ✔ CH-*π* | ✔ | ✔ H | ✔ H | ✔ | ✔ | ✔ | ✔ H |
| His266* | ✔ H | ✔ | ✔ | ✔ | | ✔ | ✔ | ✔ |
| Of the given 28 amino acids | 16 | 17 | 19 | 19 | 13 | 18 | 18 | 19 |
| *Of the preferred 19 amino acids | 16 | 16 | 17 | 17 | 13 | 17 | 15 | 17 |

**[Table 3-3]**

| **Comp. No.** | **18** | **19** | **20** | **21** | **22** | **23** | **24** | **25** |
|---|---|---|---|---|---|---|---|---|
| Phe60 | | | | | | | | |
| Gln87 | | | | | | | | |
| Asp121* | ✔ | ✔ | ✔ | ✔ | ✔ | | | ✔ |
| Pro122* | ✔ | ✔ H | ✔ | ✔ | ✔ | ✔ | ✔ | ✔ |
| Asp123* | ✔ | ✔ | ✔ | ✔ | ✔ | ✔ | ✔ | ✔ |
| Gln124* | ✔ H | | | ✔ | ✔ | ✔ | | |
| Asp153* | ✔ | | ✔ | | ✔ | ✔ | ✔ | ✔ |
| Cys154* | ✔ H | ✔ | ✔ | ✔ | ✔ CH-*π* | ✔ CH-*π* | ✔ H | ✔ H |
| Glu155* | ✔ | ✔ | ✔ | ✔ | ✔ | ✔ | ✔ | ✔ |
| Lys160* | | | ✔ | ✔ H | ✔ | ✔ H | ✔ H | |
| Pro164* | ✔ | ✔ | | | ✔ | ✔ | | ✔ |
| Cys165 | ✔ | ✔ | | | | | | |
| Ser167 | | | | | | | | ✔ |
| Ser168* | ✔ H | ✔ | ✔ | ✔ | ✔ | ✔ | ✔ | ✔ |
| Gly169* | ✔ | ✔ | ✔ | ✔ | ✔ | ✔ | | ✔ |
| Ser170* | ✔ | ✔ CH-*π* | ✔ | ✔ | ✔ | ✔ | ✔ | ✔ |
| Leu171 | | | | | | | ✔ | |
| Trp172* | ✔ | ✔ | ✔ | ✔ | ✔ | ✔ | ✔ | |
| Asp173 | | | | ✔ | | | | |
| Pro174 | | | | | | | | |
| Pro254 | | | ✔ | | | | | |
| Phe256 | | | ✔ | | | | | |
| Ser258* | ✔ | ✔ H | | ✔ | ✔ | ✔ | | ✔ |
| Cys259* | ✔ | ✔ | ✔ | | ✔ | ✔ | ✔ | ✔ |
| Asp262* | | ✔ | ✔ | ✔ | ✔ | ✔ | ✔ | ✔ |
| Cys263* | | ✔ | ✔ | ✔ | | | ✔ | |
| Leu264* | ✔ | ✔ | ✔ | ✔ | ✔ | ✔ | ✔ | ✔ CH-*π* |
| His266* | ✔ | ✔ | ✔ | ✔ H | ✔ | ✔ | ✔ | ✔ |
| Of the given 28 amino acids | 17 | 17 | 18 | 17 | 18 | 17 | 15 | 16 |
| *Of the preferred 19 amino acids | 16 | 16 | 16 | 16 | 18 | 17 | 14 | 15 |

**[Table 3-4]**

| **Comp. No.** | **26** | **27** | **28** | **29** | **30** | **31** | **32** | **33** |
|---|---|---|---|---|---|---|---|---|
| Phe60 | | | | | | | | |
| Gln87 | | | | | | | | |
| Asp121* | ✔ | ✔ | ✔ | ✔ CH ×2 | ✔ | | | ✔ |
| Pro 122* | ✔ | ✔ | ✔ CH-*π* | ✔ | ✔ | ✔ | ✔ | ✔ CH-*π* |
| Asp123* | ✔ | ✔ H | ✔ | | ✔ | ✔ | ✔ | ✔ |
| Gln124* | | | ✔ | | ✔ | | | |
| Asp153* | ✔ | ✔ | ✔ | ✔ | | ✔ | ✔ | ✔ |
| Cys154* | ✔ H | ✔ H CH-*π* | ✔ | ✔ H | ✔ | ✔ H | ✔ | ✔ |
| Glu155* | ✔ | ✔ | ✔ H ×2 | ✔ | ✔ | ✔ H | ✔ | ✔ |
| Lys160* | | | ✔ | | ✔ | ✔ H | ✔ H | ✔ cation-π |
| Pro164* | ✔ | ✔ | ✔ | ✔ | | ✔ | | ✔ |
| Cys165 | | | | | | | | |
| Ser167 | ✔ | ✔ | | ✔ | | | | |
| Ser168* | ✔ | ✔ | ✔ | ✔ | ✔ | ✔ | ✔ | ✔ |
| Gly169* | ✔ | ✔ | ✔ | ✔ | ✔ | ✔ | ✔ | ✔ |
| Sar170* | ✔ | ✔ CH-*π* | ✔ | ✔ CH-*π* | ✔ | ✔ | ✔ | ✔ |
| Leu171 | | | | | | | ✔ | |
| Trp172* | ✔ | ✔ | ✔ | | ✔ | ✔ | ✔ | ✔ |
| Asp173 | | | | | | | ✔ | |
| Pro 174 | | | | | ✔ | | | |
| Pro254 | | | | | | | | |
| Phe256 | | | | | | | | |
| Ser258* | ✔ | ✔ | ✔ | ✔ | ✔ | ✔ | | ✔ |
| Cys259* | ✔ | ✔ | ✔ | ✔ | ✔ | ✔ | | ✔ |
| Asp262* | ✔ | ✔ | ✔ | ✔ H | ✔ | ✔ | ✔ | ✔ |
| Cys263* | | | | ✔ | ✔ | ✔ | ✔ | |
| Leu264* | ✔ CH-*π* | ✔ | ✔ | ✔ | ✔ H | ✔ | ✔ | ✔ |
| His266* | ✔ | ✔ | ✔ | ✔ | ✔ | ✔ | ✔ | ✔ H |
| Of the given 28 amino acids | 17 | 17 | 18 | 16 | 18 | 17 | 16 | 17 |
| *Of the preferred 19 amino acids | 16 | 16 | 18 | 15 | 17 | 17 | 14 | 17 |

**[Table 3-5]**

| **Comp. No.** | 34 | 35 | 36 |
|---|---|---|---|
| Phe60 | | | |
| Gln87 | | | |
| Asp121* | ✔ H | ✔ | ✔ |
| Pro122* | ✔ | ✔ | ✔ |
| Asp123* | ✔ H | ✔ | ✔ |
| Gln124* | ✔ H | ✔ | |
| Asp153* | ✔ H | ✔ | ✔ |
| Cys154* | ✔ | ✔ H ×2 | ✔ H ×2 |
| Glu155* | ✔ | ✔ | ✔ H |
| Lys160* | ✔ | | ✔ |
| Pro 164* | | ✔ | ✔ |
| Cys165 | | | |
| Ser167 | | | |
| Ser168* | ✔ | ✔ | ✔ |
| Gly169* | | | ✔ |
| Ser170* | ✔ | ✔ | ✔ |
| Leu171 | | | |
| Trp172* | | | ✔ |
| Aspl73 | | | |
| Pro174 | | | |
| Pro254 | | | |
| Phe256 | | | |
| Ser258* | | ✔ | ✔ |
| Cys259* | ✔ | ✔ | |
| Asp262* | ✔ | ✔ H | ✔ |
| Cys263* | ✔ | ✔ | ✔ |
| Leu264* | ✔ | ✔ | ✔ |
| His266* | | | ✔ |
| Of the given 28 amino acids | 14 | 15 | 17 |
| *Of the preferred 19 amino acids | 14 | 15 | 17 |

In the present invention, derivatives of the compounds (1) to (36) can also be used as the IL-17A activity inhibitor. Those skilled in the art can prepare the derivatives of the compounds (1) to (36) and select derivatives having a desired IL-17A activity inhibiting ability, thereby implementing the present invention without excessive trial and error. Derivatives of the other compounds that can be used in the present invention can also be prepared, for example, by referring to descriptions of the derivatives of the compounds (1), (5), (9), and (11) to be described below, or by referring to contents shown in each of the schematic views that are illustrated in the drawings and illustrate modes of the non-covalent interactions between each of the compounds and the amino acid residues contained in the extracellular domain of IL-17RA

When preparing the derivatives, groups, bonds, and other structures to be replaced from an original compound may be selected from the same types as those of the original compound or may be selected from the types different from those of the original compound. In the present specification, 6 types of (Al) to (A6) as the site A, 8 types of (B1) to (B8) as the site B, 7 types of (C1) to (C7) as the site C, 6 types of (D1) to (D6) as the site D, 5 types of (L¹1) to (L¹5) as L¹, 3 types of (L²1) to (L²3) as L², and 4 types of (L³1) to (L³4) as L³ in Structural Formula (I) are exemplified, and specific examples thereof are also provided. For example, in a case where the original compound has a group of (Al) as the site A, a derivative thereof may be any one of a derivative having another group selected from (A1) (the same types), a derivative having a group selected from (A2) to (A6) (different types), and a derivative having a group selected from the types other than (A1) to (A6), as a site corresponding to the site A. The same applies to other sites. In addition, when preparing the derivatives, when a substituent is different from that of the original compound or a substituent that is absent in the original compound is introduced, a substituent of a derivative can be selected from the "substituent group A" exemplified in the present specification.

In an embodiment of the present invention, 4, 5, or 6 sites among 7 sites A, L¹, B, L², C, L³, and D in a derivative of a certain compound are the same groups as those in the original group, and remaining sites in the derivative of the certain compound are groups selected from the same types as those in the original compound (for example, having different substituents) or other groups selected from the types different from those in the original compound. In an embodiment of the present invention, 4, 5, 6, or 7 sites among the 7 sites A, L¹, B, L², C, L³, and D in a derivative of a certain compound are the same groups as those in the original compound or other groups selected from the same types as those in the original compound (where a case in which all of the 7 sites are the same groups as those in the original compound is excluded), and remaining sites in the derivative of the certain compound are groups selected from the types different from those in the original compound. In an embodiment of the present invention, the "group selected from the same types as those in the original compound" or the "other groups selected from the type different from those in the original compound" are groups included in compounds other than the original group among the compounds (1) to (36) at the corresponding site.

In an embodiment of the present invention, in a case where a cyclic structure is present at a certain site in an original compound, a derivative of the compound has the same cyclic structure at the corresponding site. In an embodiment of the present invention, in a case where a chain structure is present at a certain site in an original compound, a derivative of the compound has the same chain structure at the corresponding site.

In an embodiment of the present invention, in a case where a cyclic or chain structure is present at a certain site in an original compound, a derivative of the compound has a cyclic or chain structure according to an interconversion between the cyclic structure and the chain structure that are pharmaceutically used at the corresponding site. In an embodiment of the present invention, in a case where a cyclic or chain structure having a substituent is present at a certain site in the original compound, a derivative of the compound has a cyclic or chain structure having a substituent with the same or similar chemical properties at the corresponding site.

In general, it is preferable that a non-covalent interaction that is generated between each of the derivatives of the compounds (1) to (36) and IL-17RA is more stable (stronger) than a non-covalent interaction that is generated between each of original compounds (1) to (36) and IL-17RA in all (total). For an index of stability (strength) of the interaction, for example, the score (unit: kcal/mol) shown as "GBVIWSA_dG" in Table 2 can be referred to. If necessary, a structure to be introduced into the derivative can be selected with reference to the index of the stability (strength) of the interaction such as the van der Waals force and/or the non-covalent interaction other than the van der Waals force.

However, in the preparation of the derivatives of the compounds (1) to (36), a structure of each of the compounds (1) to (36) is preferably modified so that the compound becomes more similar to a compound having desired properties, while considering not only an increase of the binding stability to IL-17RA but also solubility in a solvent or disposition which are important in the use as an active ingredient of a medicament. In the preparation of the derivative, various methods known in the technical field to which the present invention relates can be used.

Regarding the compounds (1) to (36) excluding the compound (3), the sites corresponding to the structures A, L1, B, L2, C, L3 and D in the general formula (I) of each compound are shown in Table 4. Shown in. In a preferred embodiment of the present invention, the compound (I) is the compound (1), (2), (5), (9), or (11), or a derivative thereof. For example, 4, 5, or 6 sites among the sites A, L¹, B, L², C, L³, and D in the derivative of the compound (1), (2), (5), (9), or (11) may be the same groups as those in the original compound, and remaining sites may be groups selected from the same types as those in the original compound or other groups selected from the types different from those in the original compound. In addition, 4, 5, 6, or 7 sites among the sites A, L¹, B, L², C, L³, and D in the derivative of the compound (1), (2), (5), (9), or (11) may be the same groups as those in the original compound or other groups selected from the same types as those in the original compound (where a case in which all of the 7 sites are the same groups as those in the original compound is excluded), and remaining sites may be selected from the types different from those in the original compound. The same applies to compounds other than the compounds (1), (2), (5), (9) and (11).

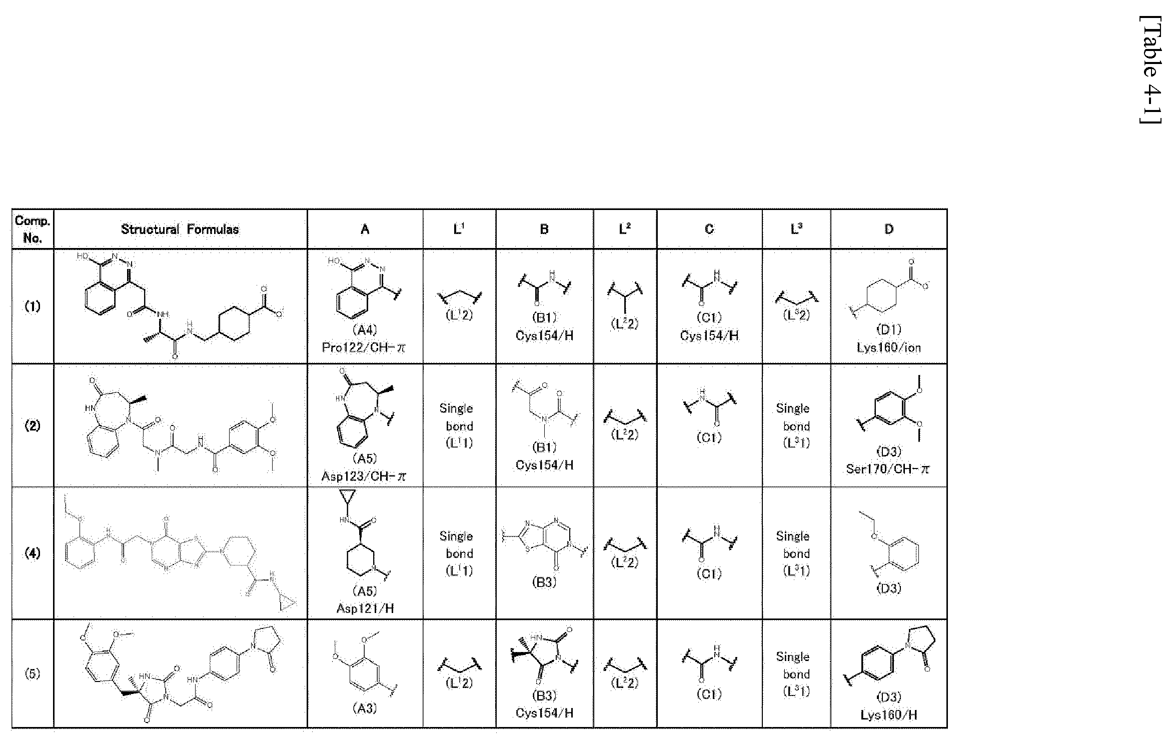

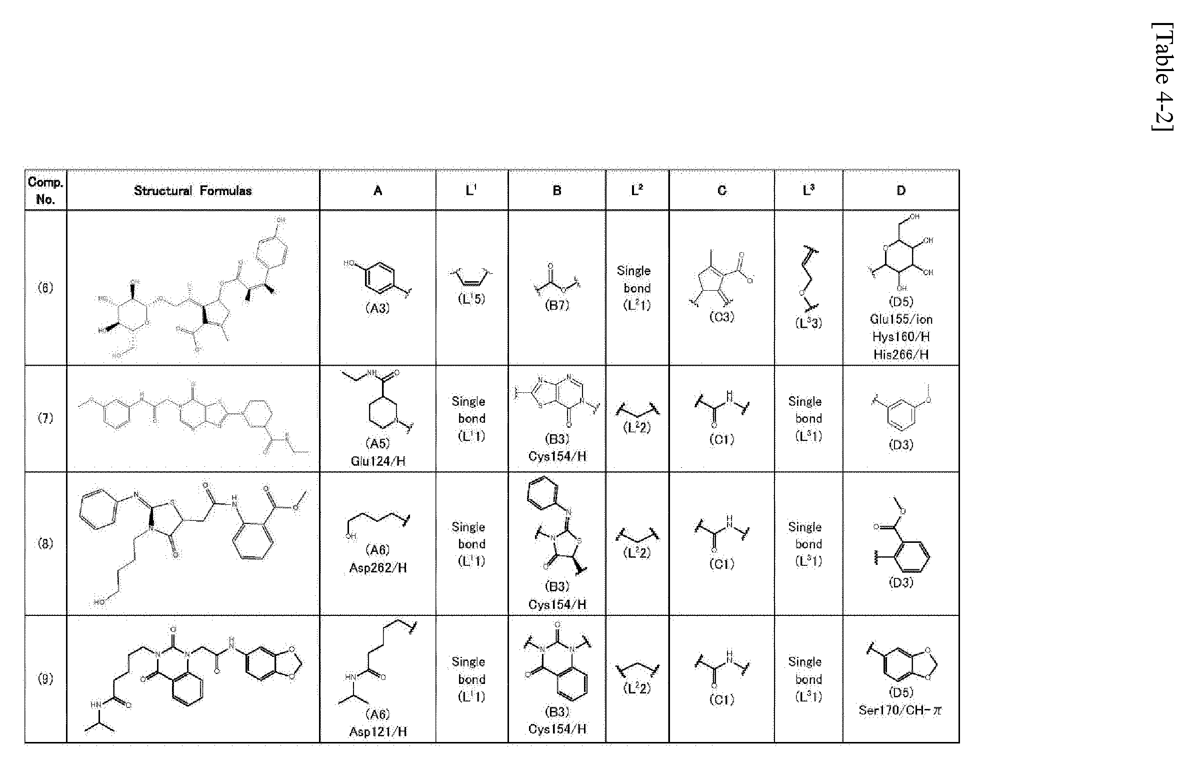

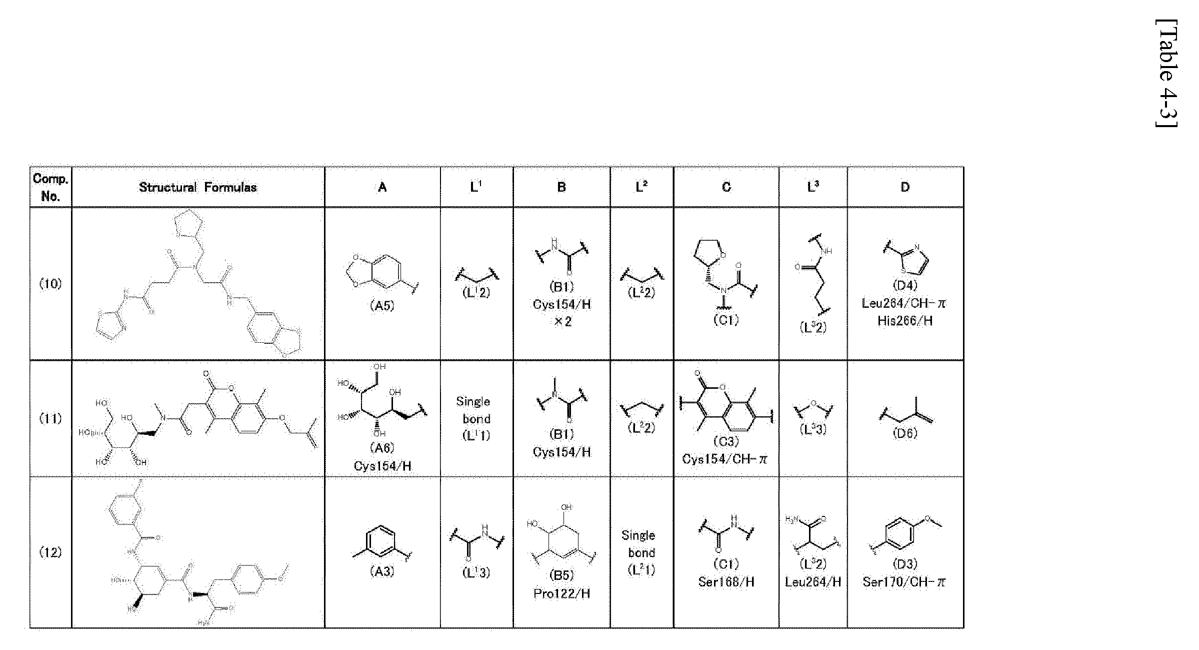

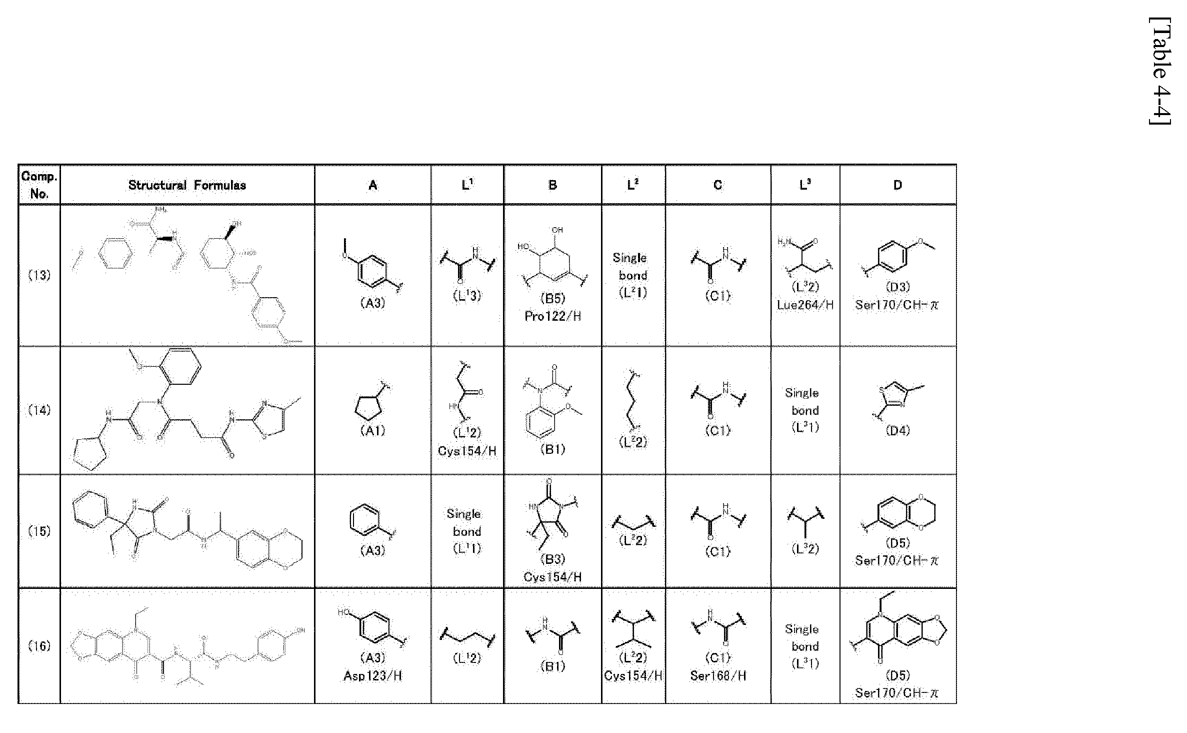

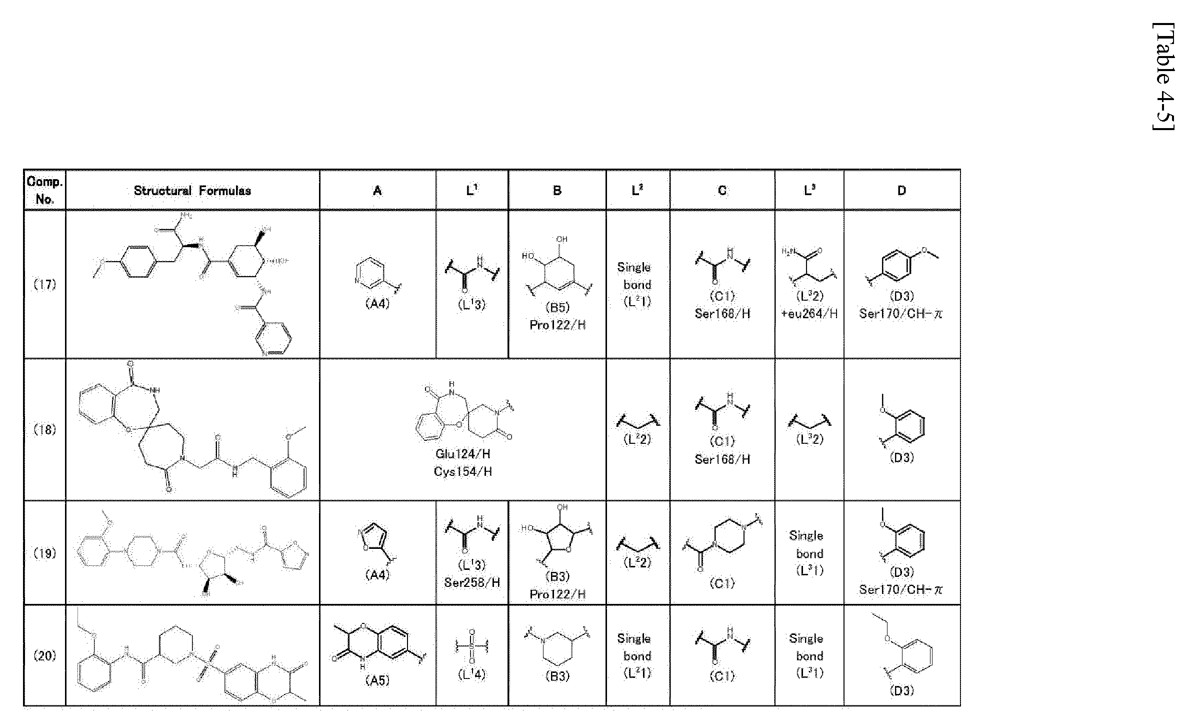

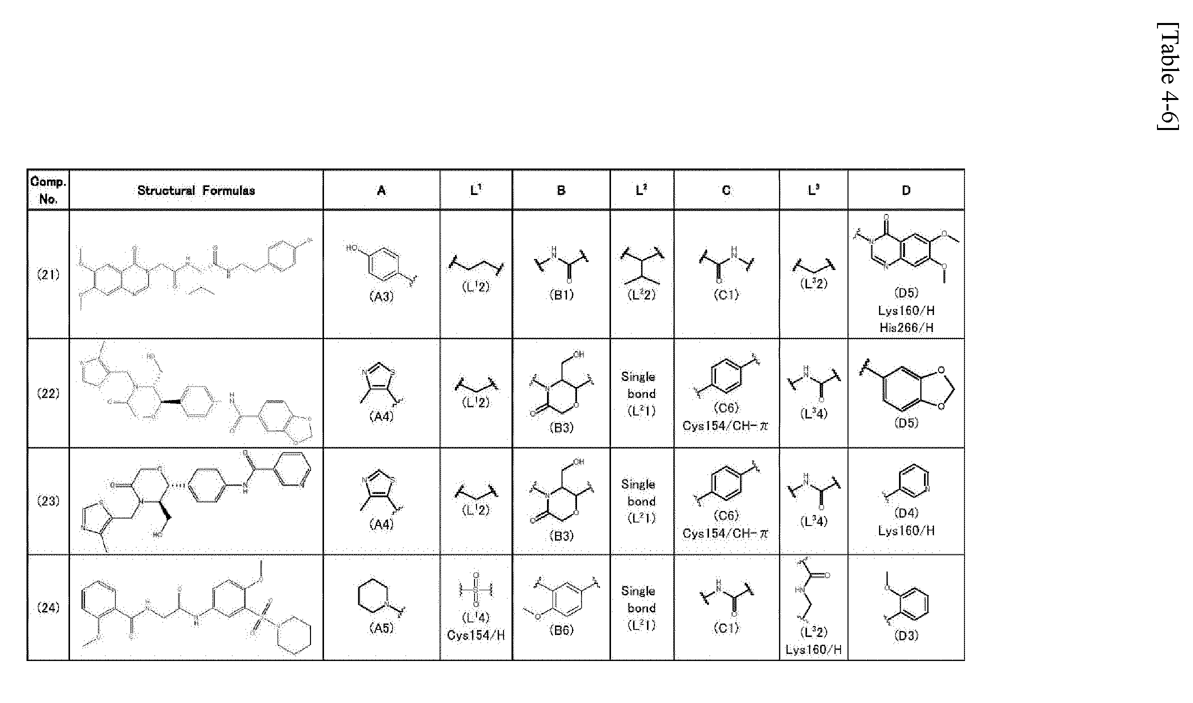

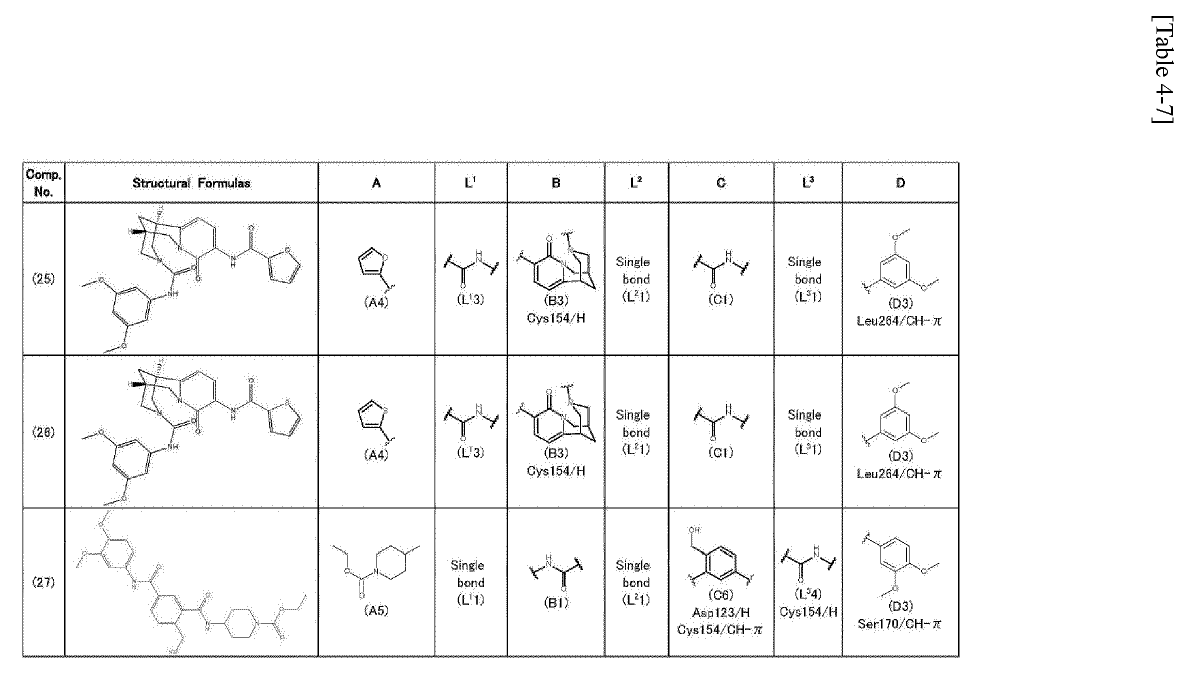

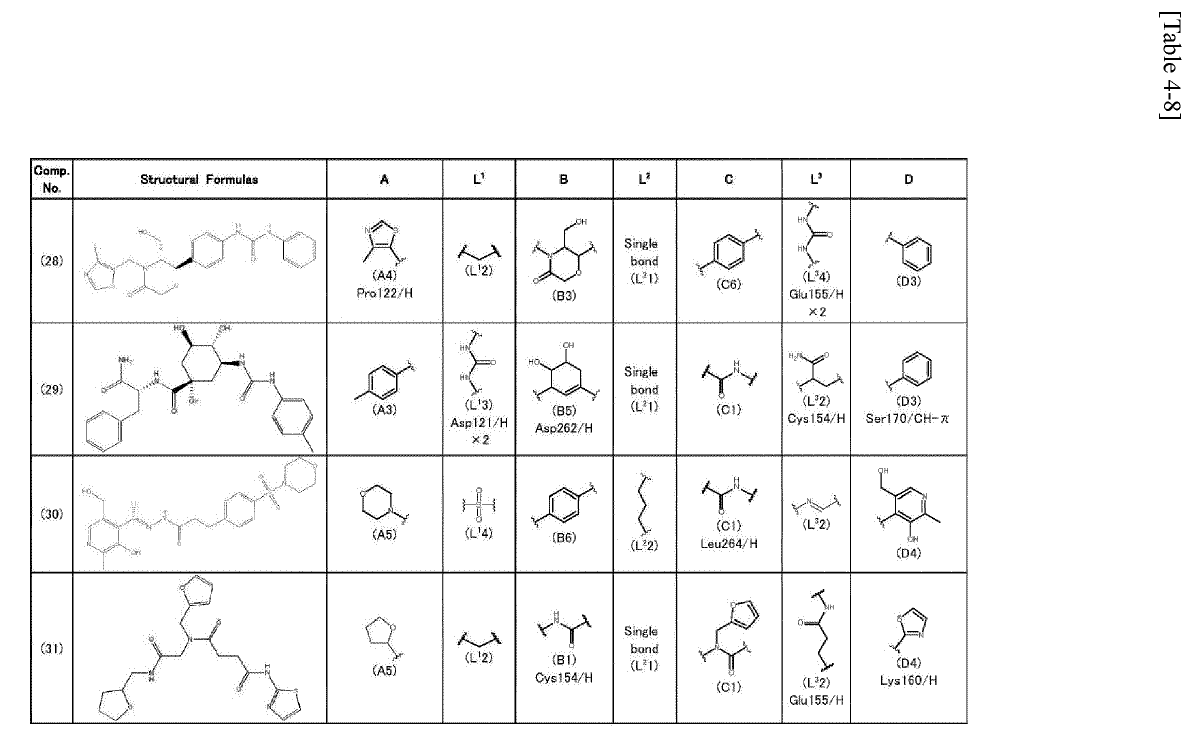

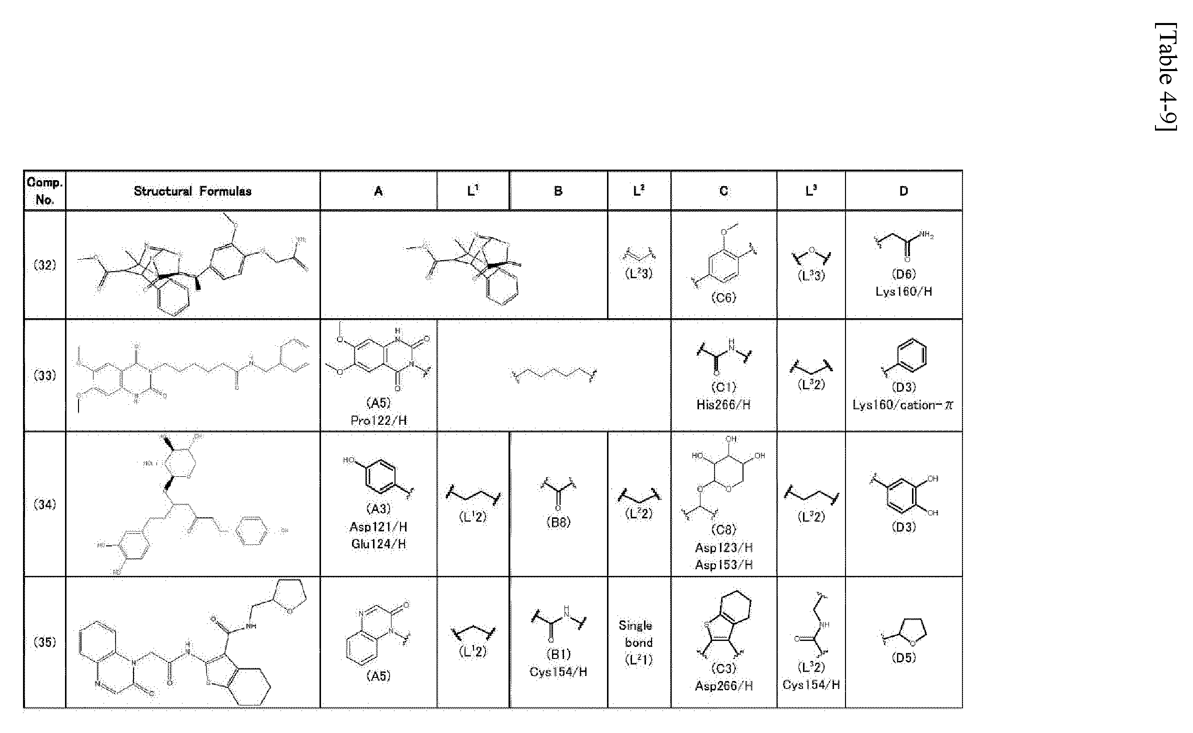

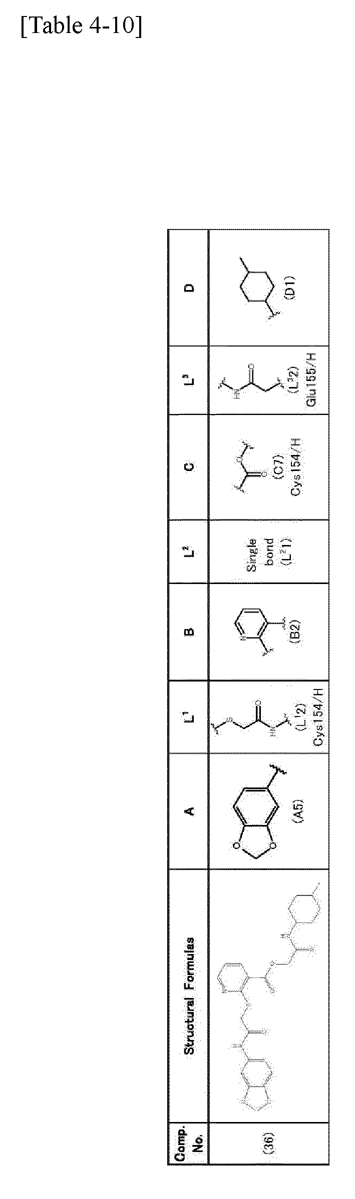

The compound (1) is a compound represented by the following Structural Formula (1).

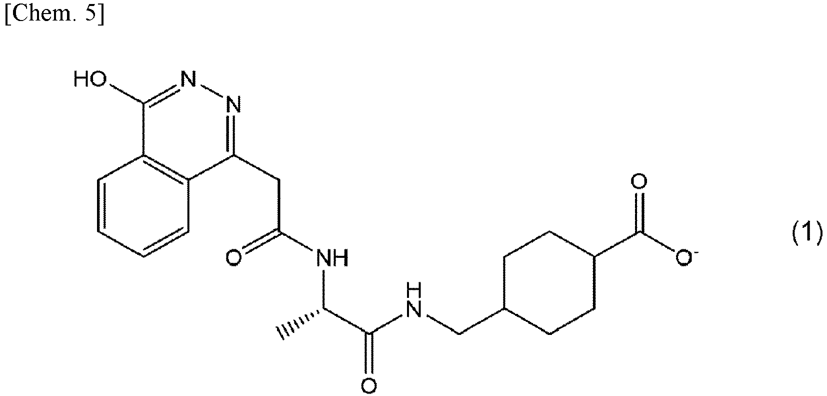

As illustrated in Fig. 2, the compound (1) can stably bind in the interaction region by an action of a van der Waals force between the compound (1) and Asp121, Pro122, Gln124, Cys154, Glu155, Lys160, Pro164, Serl68, Gly169, Ser170, Ser258, Cys259, Asp262, Cys263, and Leu264 among the predetermined amino acid residues constituting the interaction region, and further, by an action of a non-covalent interaction other than the van der Waals force between the compound (1) and some amino acid residues of Asp121, Pro122, Gln124, Cys154, Glu155, Lys160, Pro164, Serl68, Gly169, Serl70, Ser258, Cys259, Asp262, Cys263, and Leu264. A "phthalazine ring" (a benzene ring part of a condensed ring) contained in the site A in General Formula (I) is a site at which a CH-π interaction with Pro122 is generated, two "carbamoyl groups" (amide bonds) contained in each of the site B and the site C in General Formula (I) are sites (serving as donors) at which a hydrogen bonding with Cys154 is generated, respectively, and an "(ionized) carboxyl group as a substituent of a cyclohexyl group" contained in the site D in the General Formula (I) is a site at which an ionic bonding with an ionized amino group of Lysl60 is generated.

An embodiment of the derivative of the compound (1) can include a derivative (1-X) obtained by modifying the original compound (1) so that a van der Waals force between the derivative (1-X) and Asp121, Pro122, Gln124, Cys154, Glu155, Lys160, Pro164, Serl68, Gly169, Serl70, Ser258, Cys259, Asp262, Cys263, and Leu264 is increased as compared with the compound (1).

The dotted line drawn in Fig. 2 (and other drawings) represents a contact surface between the atoms of the compound (1) (and another compound of the present invention) and the atoms of the amino acid residues around the atoms of the compound (1). The smaller the gap between the atoms in the structural formula and the dotted line, the tighter the bond. The wider the gap, the looser the bond. Therefore, in order to make the gap between the atoms in the structural formula and the dotted line smaller, a van der Waals force between the compound (1) (and the compound of the present invention) and the amino acid residues (and other predetermined amino acid residues constituting the interaction region) can be increased by modifying a structure of at least one site selected from the group consisting of the sites A, B, C, D, L¹, L², and L³ in the structural formula, for example, by changing the group to a bulkier group or by introducing a substituent.

An embodiment of the derivative of the compound (1) can include a derivative (1-Y) obtained by modifying the original compound (1) so that the derivative has a site at which at least one of a CH-π interaction with Pro 122, a hydrogen bonding with Cys154, and an ionic bonding with Lys160 is increased, or a site at which at least one non-covalent interaction different from the CH-π interaction with Pro122, the hydrogen bonding with Cys154, and the ionic bonding with Lys160 (different in at least one of the type and strength of intermolecular interaction and a target amino acid residue) is generated between the derivative (1-Y) and at least one amino acid residue selected from the group consisting of Asp121, Pro122, Gln124, Cys154, Glu155, Lys160, Pro164, Serl68, Gly169, Serl70, Ser258, Cys259, Asp262, Cys263, and Leu264, the site being included in the compound (1).

Examples of the derivative (1-Y) modified from the above viewpoint may include the following:
a derivative with improved stability of a CH-π interaction with Pro122 through modification of the site A (the phthalazine ring substituted with a hydroxyl group) in General Formula (I);
a derivative with improved stability of a hydrogen bonding with Cys154 through modification of the site B and/or C (both are carbamoyl groups) in General Formula (I);
a derivative with improved stability of an ionic bonding with Lys160 through modification of the site D (the cyclohexyl group substituted with a carboxylic group) in General Formula (I); and
a derivative obtained by modifying the sites A, L¹, B, L², C, L³, and D in General Formula (I) to generate a new non-covalent interaction with Asp121, Gln124, Glu155, Pro164, Serl68, Gly169, Serl70, Ser258, Cys259, Asp262, Cys263, or Leu264 (an amino acid residue other than Pro122, Cys154, and Lys160), and further, with other predetermined amino acid residues constituting the interaction region.

An embodiment of the derivative of the compound (1) can include a derivative (1-Z) obtained by modifying the original compound (1) so that the derivative has a site at which exposure, to a solvent, of at least one amino acid residue selected from the group consisting of Asp121, Pro122, Gln124, Cys154, Glu155, Lys160, Pro164, Serl68, Gly169, Serl70, Ser258, Cys259, Asp262, Cys263, and Leu264 is reduced as compared with the compound (1).

A shadow around the circle representing the amino acid residue constituting the interaction region illustrated in Fig. 2 (and other drawings) represents that exposure of the amino acid residue to a solvent is reduced by binding of the compound (1) (and other compounds of the present invention), and a magnitude of the reduction is increased as a size of the shadow is increased (for example, see Leu264 in Fig. 2). The amino acid residue of which exposure to the solvent is reduced has a strong hydrophobic interaction with the compound of the present invention, and can further competitively and strongly inhibit binding of IL-17A to IL-17RA

The derivative of the compound (1) may simultaneously satisfy all two or three properties relating to (1-X), (1-Y), and (1-Z).

The compound (2) is a compound represented by the following Structural Formula (2).

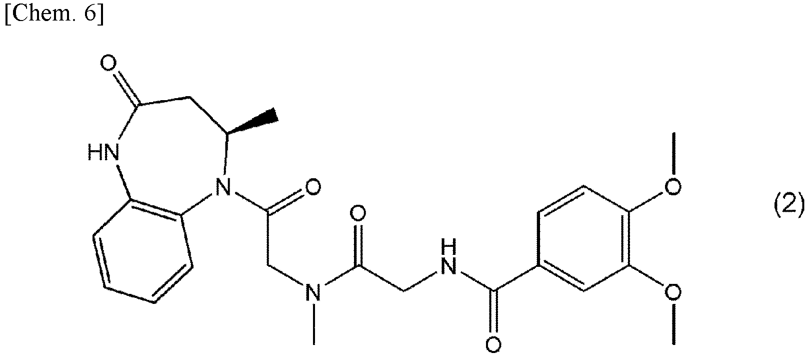

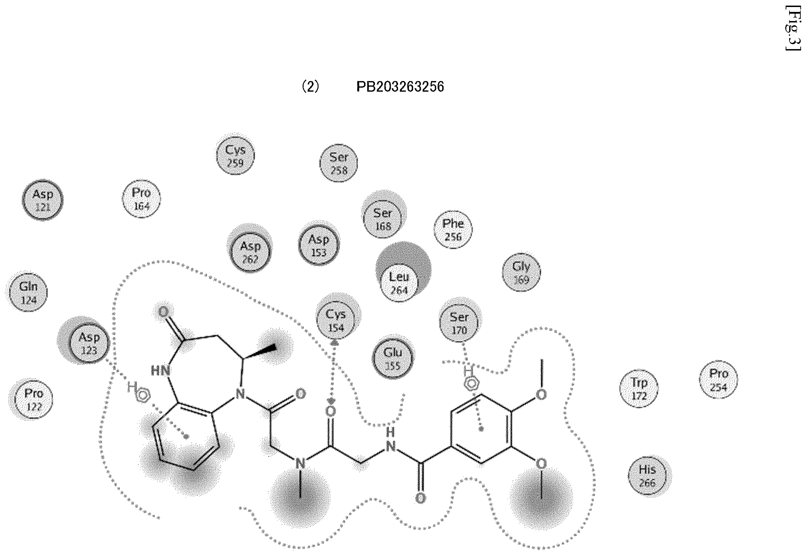

As illustrated in Fig. 3, the compound (2) can stably bind in the interaction region by an action of a van der Waals force between the compound (2) and Asp121, Pro122, Asp123, Gln124, Asp153, Cys154, Glu155, Pro164, Serl68, Gly169, Serl70, Trp172, Pro254, Phe256, Ser258, Cys259, Asp262, Leu264, and His266 among the predetermined amino acid residues constituting the interaction region, and further, by an action of a non-covalent interaction other than the van der Waals force between the compound (2) and some amino acid residues of Asp121, Pro122, Asp123, Gln124, Asp153, Cys154, Glu155, Pro164, Serl68, Gly169, Ser170, Trp172, Pro254, Phe256, Ser258, Cys259, Asp262, Leu264, and His266. A ring (a benzene ring part of a condensed ring) contained in the site A in General Formula (I) is a site at which a CH-π interaction with Asp123 is generated, a carbamoyl group contained in the site B in General Formula (I) is a site (serving as a donor) at which a hydrogen bonding with Cys154 is generated, and a phenyl group (substituted with two methoxy groups) contained in the site D in General Formula (I) is a site at which a CH-π interaction with Serl70 is generated.

An embodiment of the derivative of the compound (2) can include a derivative (2-X) obtained by modifying the original compound (2) so that a van der Waals force between the derivative (2-X) and Asp121, Pro122, Asp123, Gln124, Asp153, Cys154, Glu155, Pro164, Serl68, Gly169, Serl70, Trp172, Pro254, Phe256, Ser258, Cys259, Asp262, Leu264, and His266 is increased as compared with the compound (2).

An embodiment of the derivative of the compound (2) can include a derivative (2-Y) obtained by modifying the original compound (2) so that the derivative has a site at which at least one of the CH-π interaction with Asp123, the hydrogen bonding with Cys154, and the CH-π interaction with Serl70 is increased, or a site at which at least one non-covalent interaction other than a van der Waals force different from the CH-π interaction with Asp123, the hydrogen bonding with Cys154, and the CH-π interaction with Serl70 is generated between the derivative (2-Y) and at least one amino acid residue selected from the group consisting of Asp121, Pro122, Asp123, Gln124, Asp153, Cys154, Glu155, Pro164, Serl68, Gly169, Ser170, Trp172, Pro254, Phe256, Ser258, Cys259, Asp262, Leu264, and His266, the site being included in the compound (2).

An embodiment of the derivative of the compound (2) can include a derivative (2-Z) obtained by modifying the original compound (2) so that the derivative has a site at which exposure, to a solvent, of at least one amino acid residue selected from the group consisting of Asp121, Pro122, Asp123, Gln124, Asp153, Cys154, Glu155, Pro164, Serl68, Gly169, Serl70, Trp172, Pro254, Phe256, Ser258, Cys259, Asp262, Leu264, and His266 is reduced as compared with the compound (2).

The compound (5) is a compound represented by the following Structural Formula (5).

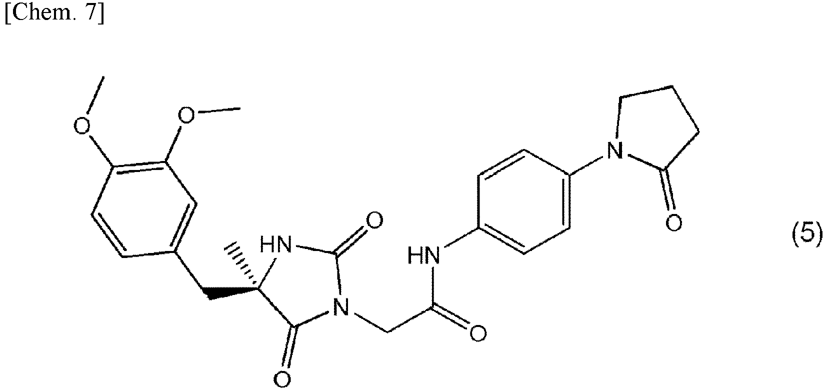

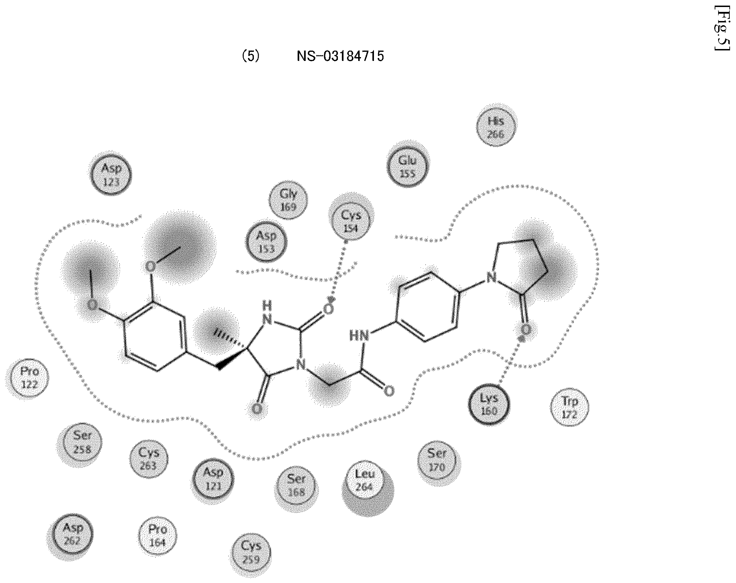

As illustrated in Fig. 5, the compound (5) can stably bind in the interaction region by an action of a van der Waals force between the compound (5) and Asp121, Pro122, Asp123, Asp153, Cys154, Glu155, Lys160, Pro164, Serl68, Gly169, Serl70, Trp172, Ser258, Cys259, Asp262, Cys263, Leu264, and His266 among the predetermined amino acid residues constituting the interaction region, and further, by an action of a non-covalent interaction other than the van der Waals force between the compound (5) and some amino acid residues of Asp121, Pro122, Asp123, Asp153, Cys154, Glu155, Lys160, Pro164, Serl68, Gly169, Serl70, Trp172, Ser258, Cys259, Asp262, Cys263, Leu264, and His266. A keto group (an oxo group as a substituent) contained in the site B in General Formula (I) is a site (serving as an acceptor) at which a hydrogen bonding with Cys154 is generated, and a keto group (an oxo group binding to a carbon atom of a pyrrolidine ring (substituting a hydrogen atom) as a substituent of a phenyl group) contained in the site D in General Formula (I) is a site (serving as an acceptor) at which a hydrogen bonding with Lys160 is generated.

An embodiment of the derivative of the compound (5) can include a derivative (5-X) obtained by modifying the original compound (5) so that a van der Waals force between the derivative (5-X) and Asp121, Pro122, Asp123, Asp153, Cys154, Glu155, Lys160, Pro164, Serl68, Gly169, Serl70, Trp172, Ser258, Cys259, Asp262, Cys263, Leu264, and His266 is increased as compared with the compound (5).

An embodiment of the derivative of the compound (5) can include a derivative (5-Y) obtained by modifying the original compound (5) so that the derivative has a site at which at least one of the hydrogen bonding with Cys154 and the hydrogen bonding with Lys160 is increased, or a site at which at least one non-covalent interaction other than a van der Waals force different from the hydrogen bonding with Cys154 and the hydrogen bonding with Lys160 is generated between the derivative (5-Y) and at least one amino acid residue selected from the group consisting of Asp121, Pro122, Asp123, Asp153, Cys154, Glu155, Lys160, Pro164, Serl68, Gly169, Serl70, Trp172, Ser258, Cys259, Asp262, Cys263, Leu264, and His266, the site being included in the compound (5).

An embodiment of the derivative of the compound (5) can include a derivative (5-Z) obtained by modifying the original compound (5) so that the derivative has a site at which exposure, to a solvent, of at least one amino acid residue selected from the group consisting of Asp121, Pro122, Asp123, Asp153, Cys154, Glu155, Lys160, Pro164, Ser168, Gly169, Serl70, Trp172, Ser258, Cys259, Asp262, Cys263, Leu264, and His266 is reduced as compared with the compound (5).

The compound (9) is a compound represented by the following Structural Formula (9).

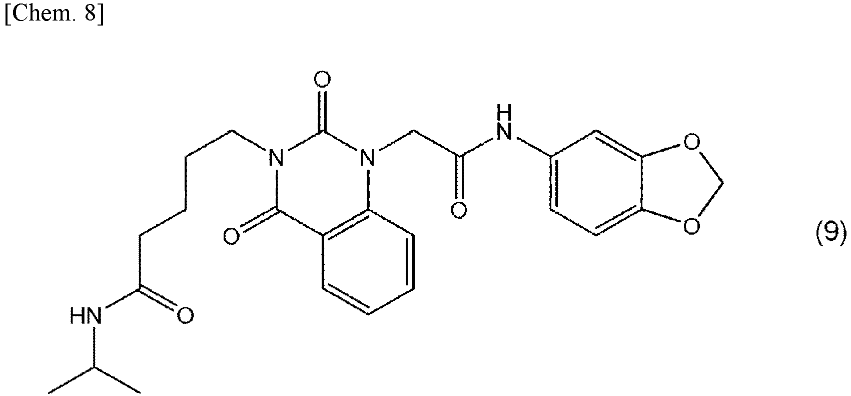

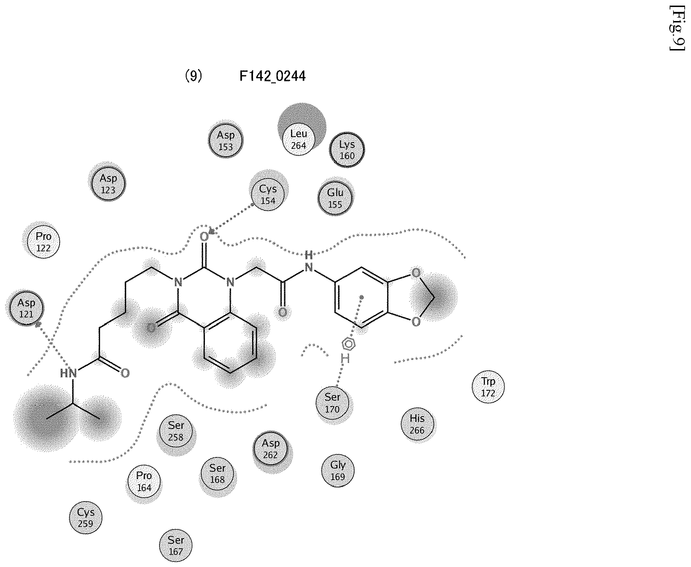

As illustrated in Fig. 9, the compound (9) can stably bind in the interaction region by an action of a van der Waals force between the compound (9) and Asp121, Pro122, Asp123, Asp153, Cys154, Glu155, Lys160, Pro164, Serl67, Serl68, Gly169, Serl70, Trp172, Ser258, Cys259, Asp262, Leu264, and His266 among the predetermined amino acid residues constituting the interaction region, and further, by an action of a non-covalent interaction other than the van der Waals force between the compound (9) and some amino acid residues of Asp121, Pro122, Asp123, Asp153, Cys154, Glu155, Lys160, Pro164, Serl67, Serl68, Gly169, Serl70, Trp172, Ser258, Cys259, Asp262, Leu264, and His266. A substituted amino group contained in the site A in General Formula (I) is a site (serving as a donor) at which a hydrogen bonding with Asp121 is generated, a keto group (an oxo group as a substituent) of a ring contained in the site B in General Formula (I) is a site (serving as an acceptor) at which a hydrogen bonding with Cys154 is generated, and a ring (a benzene ring part of a condensed ring) contained in the site D in General Formula (I) is a site at which a CH-π interaction with Serl70 is generated.

An embodiment of the derivative of the compound (9) can include a derivative (9-X) obtained by modifying the original compound (9) so that a van der Waals force between the derivative (9-X) and Asp121, Pro122, Asp123, Asp153, Cys154, Glu155, Lys160, Pro164, Serl67, Serl68, Gly169, Serl70, Trp172, Ser258, Cys259, Asp262, Leu264, and His266 is increased as compared with the compound (9).

An embodiment of the derivative of the compound (9) can include a derivative (9-Y) obtained by modifying the original compound (9) so that the derivative has a site at which at least one of the CH-π interaction with Asp121, the hydrogen bonding with Cys154, and the CH-π interaction with Serl70 is increased, or a site at which at least one non-covalent interaction other than a van der Waals force different from the CH-π interaction with Asp121, the hydrogen bonding with Cys154, and the CH-π interaction with Serl70 is generated between the derivative (9-Y) and at least one amino acid residue selected from the group consisting of Asp121, Pro122, Asp123, Asp153, Cys154, Glu155, Lys160, Pro164, Serl67, Serl68, Gly169, Serl70, Trp172, Ser258, Cys259, Asp262, Leu264, and His266, the site being included in the compound (9).

An embodiment of the derivative of the compound (9) can include a derivative (9-Z) obtained by modifying the original compound (9) so that the derivative has a site at which exposure, to a solvent, of at least one amino acid residue selected from the group consisting of Asp121, Pro122, Asp123, Asp153, Cys154, Glu155, Lys160, Pro164, Serl67, Serl68, Gly169, Serl70, Trp172, Ser258, Cys259, Asp262, Leu264, and His266 is reduced as compared with the compound (9).

The compound (11) is a compound represented by the following Structural Formula (11).

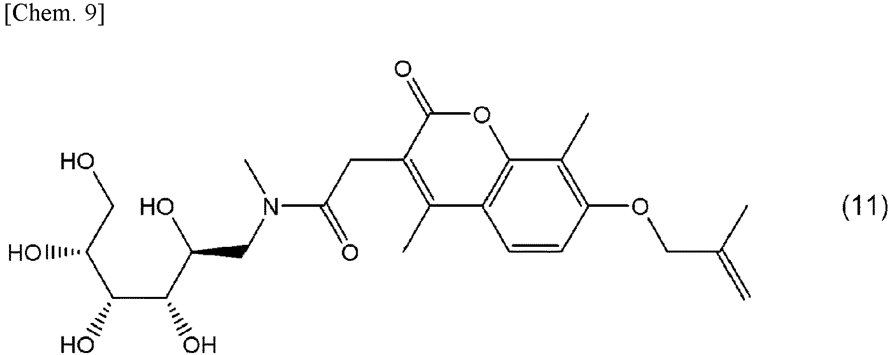

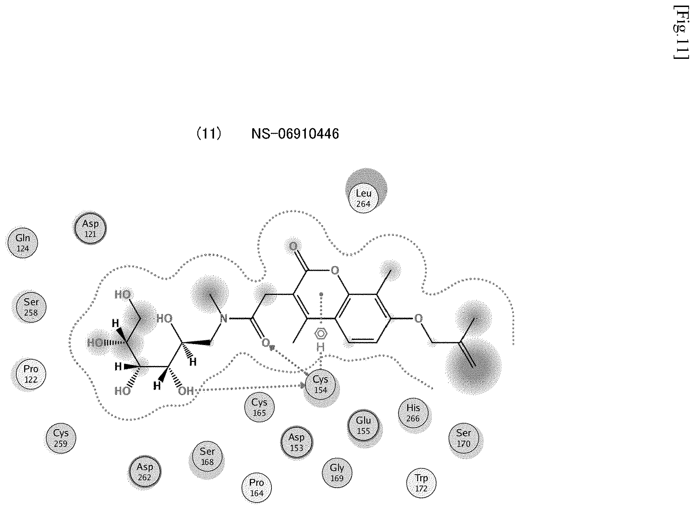

As illustrated in Fig. 11, the compound (11) can stably bind in the interaction region by an action of a van der Waals force between the compound (11) and Asp121, Pro122, Gln124, Asp153, Cys154, Glu155, Pro164, Cys165, Ser168, Gly169, Ser170, Trp172, Ser258, Cys259, Asp262, Leu264, and His266 among the predetermined amino acid residues constituting the interaction region, and further, by an action of a non-covalent interaction other than the van der Waals force between the compound (11) and some amino acid residues of Asp121, Pro122, Gln124, Asp153, Cys154, Glu155, Pro164, Cys165, Serl68, Gly169, Serl70, Trp172, Ser258, Cys259, Asp262, Leu264, and His266. A hydroxyl group contained in the site A in General Formula (I) is a site (serving as a donor) at which a hydrogen bonding with Cys154 is generated, a carbamoyl group (oxygen atom) contained in the site B in General Formula (I) is a site (serving as an acceptor) at which a hydrogen bonding with Cys154 is generated, and a ring contained in the site C in General Formula (I) is a site at which a CH-π interaction with Cys154 is generated.

An embodiment of the derivative of the compound (11) can include a derivative (11-X) obtained by modifying the original compound (11) so that a van der Waals force between the derivative (11-X) and Asp121, Pro122, Gln124, Asp153, Cys154, Glu155, Pro164, Cys165, Serl68, Gly169, Serl70, Trp172, Ser258, Cys259, Asp262, Leu264, and His266 is increased as compared with the compound (11).

An embodiment of the derivative of the compound (11) can include a derivative (11-Y) obtained by modifying the original compound (11) so that the derivative has a site at which at least one of the CH-π interaction and hydrogen bonding with Cys154 is increased, or a site at which at least one non-covalent interaction other than a van der Waals force different from the CH-π interaction and hydrogen bonding with Cys154 is generated between the derivative (11-Y) and at least one amino acid residue selected from the group consisting of Asp121, Pro122, Gln124, Asp153, Cys154, Glu155, Pro164, Cys165, Serl68, Gly169, Serl70, Trp172, Ser258, Cys259, Asp262, Leu264, and His266, the site being included in the compound (11).

An embodiment of the derivative of the compound (11) can include a derivative (11-Z) obtained by modifying the original compound (11) so that the derivative has a site at which exposure, to a solvent, of at least one amino acid residue selected from the group consisting of Asp121, Pro122, Gln124, Asp153, Cys154, Glu155, Pro164, Cys165, Serl68, Gly169, Serl70, Trp172, Ser258, Cys259, Asp262, Leu264, and His266 is reduced as compared with the compound (11).

Derivatives of compounds other than the compounds (1), (2), (5), (9), and (11) can also be derived in the same manner as described above based on the contents illustrated in the drawings and shown in the tables. That is, in a case where a van der Waals force acts between the original compound and amino acid residues among the predetermined amino acid residues constituting the interaction region, a set of the amino acid residues is defined as "P", and in a case where a non-covalent interaction other than the van der Waals force acts between the original compound and amino acid residues among the predetermined amino acid residues constituting the interaction region, a set of the amino acid residues is defined as "Q". Examples of a derivative of each compound in this case can include a derivative obtained by modifying the original compound to satisfy at least one property selected from the group consisting of the following [x], [y], and [z].
[x] A total van der Waals force between a derivative and the amino acid residues of the set P is increased as compared with the original compound;
[y] a derivative has a site at which a non-covalent interaction other than the van der Waals force between the derivative and at least one amino acid residue selected from the group consisting of the amino acid residues of the set Q is increased as compared with the original compound, or a site at which at least one non-covalent interaction other than the van der Waals force different from the above non-covalent interaction is generated between the derivative and at least one amino acid residue selected from the group consisting of the set P, the site being included in the original compound; and
[z] a derivative has a site at which exposure of at least one amino acid residue selected from the group consisting of the set P to a solvent is reduced as compared with the original compound.

The compound (I) can be in a form of pharmaceutically acceptable salt, solvate, or prodrug. In the present specification, the compound (I) (the compound represented by General Formula (I)), and a pharmaceutically acceptable salt, solvate, and prodrug thereof are collectively referred to as "the compound of the present invention".

The pharmaceutically acceptable salt means that when a salt of the compound is used as an active ingredient of a medicament, it is not harmful in terms of treatment, prophylaxis, or other purposes. Examples of the pharmaceutically acceptable salt can include the following:
as a basic salt, an alkali metal salt such as a sodium salt or a potassium salt; an alkaline earth metal salt such as a calcium salt or a magnesium salt; an ammonium salt; an aliphatic amine salt such as a trimethylamine salt, a triethylamine salt, a dicyclohexylamine salt, an ethanolamine salt, a diethanolamine salt, a triethanolamine salt, or a procaine salt; an aralkylamine salt such as N,N-dibenzylethylenediamine; a heterocyclic aromatic amine salt such as a pyridine salt, a picoline salt, a quinoline salt, or an isoquinoline salt; a quaternary ammonium salt such as a tetramethylammonium salt, a tetraethylammonium salt, a benzyltrimethylammonium salt, a benzyltriethylammonium salt, a benzyltributylammonium salt, a methyltrioctylammonium salt, or a tetrabutylammonium salt; and a basic amino acid salt such as an arginine salt or a lysine salt; and
as an acidic salt, an inorganic acid salt such as hydrochloride, sulfate, nitrate, phosphate, carbonate, hydrogen carbonate, or perchlorate; an organic acid salt such as acetate, propionate, lactate, maleate, fumarate, tartrate, malate, citrate, or ascorbate; a sulfonic acid salt such as methanesulfonate, isethionate, benzenesulfonate, or p-toluenesulfonate; and an acidic amino acid such as aspartate and glutamate.

The solvate is typically a hydrate. The solvate may be a monosolvate (monohydrate), a disolvate (dihydrate), or a solvate (hydrate) higher than those solvates.

The prodrug is a derivative having a group which can be chemically or metabolically degraded, and is converted to a pharmaceutically active compound by solvolysis (for example, degradation in phosphate buffer (pH 7.4)-ethanol) or under a physiological condition (in vivo).

Examples of a prodrug of a compound having carboxyl can include an ester derivative produced by a reaction of an original acidic compound with a suitable alcohol, and an amide derivative produced by a reaction of an original acidic compound with a suitable amine. Examples of a particularly preferred ester as a prodrug can include methyl ester, ethyl ester, n-propyl ester, isopropyl ester, n-butyl ester, isobutylester, tert-butyl ester, morpholinoethyl ester, and N,N-diethylglycolamide ester.

Examples of a prodrug of a compound having hydroxyl can include an acyloxy derivative produced by a reaction of an original compound having a hydroxyl group with a suitable acyl halide or a suitable acid anhydride. Examples of a particularly preferred acyloxy as a prodrug can include -O(=O)-CH₃, -OC(=O)-C₂H₅, -OC(=O)-(tert-Bu), -OC(=O)-C₁₅H₃₁, -OC(=O)-(m-COONa-Ph), -OC(=O)-CH₂CH₂COONa, - O(C=O)-CH(NH₂)CH₃, and -OC(=O)-CH₂-N(CH₃)₂.

Examples of a prodrug of a compound having amino can include an amide derivative produced by a reaction of an original compound having amino with a suitable acid halide or a suitable mixed acid anhydride. Examples of a particularly preferred amide as a prodrug can include -NHC(=O)-(CH₂)₂₀CH₃ and -NHC(=O)-CH(NH₂)CH₃.

The use of the IL-17 activity inhibitor of the present invention is not particularly limited. The IL-17 activity inhibitor can be used in various situations in vitro, ex vivo, and in vivo depending on a purpose of inhibiting binding of IL-17 to IL-17RA, typically to IL-17RA (extracellular domain) expressed on a cell surface.

In an embodiment of the present invention, the IL-17 activity inhibitor is used as an expression regulator (in a case where an expression regulator is prepared as a composition, as a component thereof) to be described below.

In an embodiment of the present invention, the IL-17 activity inhibitor is used as a medicament (in a case where a medicament is prepared as a composition, as an active ingredient thereof) to be described below. In other words, in an embodiment of the present invention, the IL-17 activity inhibitor is used to prepare a medicament (pharmaceutical composition) to be described below.

In an embodiment of the present invention, the IL-17 activity inhibitor is used in a method of inhibiting binding of IL-17A to IL-17RA to be described below.

### -Expression Regulator-

An "expression regulator" provided in an aspect of the present invention is an agent for regulating an expression level of a gene whose expression level is changed by binding of IL-17A to IL-17RA in a cell expressing IL-17RA. The expression regulator contains the IL-17A activity inhibitor of the present invention described above.

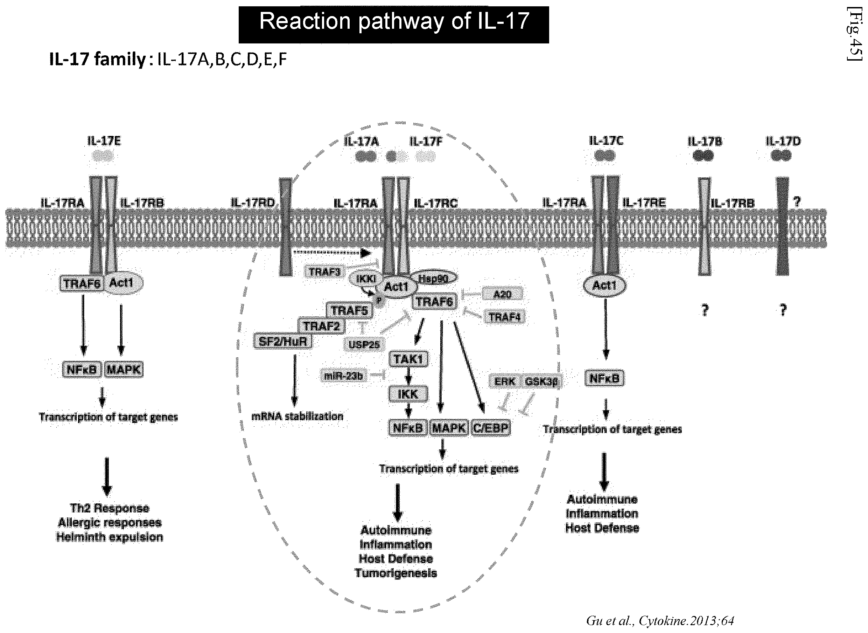

The "gene whose expression level is changed by binding of IL-17A to IL-17RA" is not particularly limited. Examples thereof can include a gene whose expression level is increased or reduced by signal transduction as illustrated in Fig. 45 (the expression is enhanced or suppressed).

In a typical embodiment of the present invention, the gene whose expression level is changed by binding of IL-17A to IL-17RA is a gene whose expression is enhanced by binding of IL-17Ato IL-17RA. It is widely known that IL-17A is an inflammatory cytokine and induces expression of a mediator (proteins such as cytokines, chemokines, and growth factors) causing inflammation and the like by binding to IL-17RA (for example, see Patent Document 2).

In a representative embodiment of the present invention, a gene whose expression is enhanced by binding of IL-17A to IL-17RA is at least one selected from the group consisting of IL-6, COX-2, mPGES1, MMP-3, MMP-13, and CXCL1. These genes are deeply related to symptoms of diseases such as intervertebral disc degeneration. It is demonstrated in examples to be described below that the expression levels of these genes are enhanced by binding of IL-17A to IL-17RA, and the composition of the present invention can reduce the expression levels of the genes by inhibiting the binding of IL-17A to IL-17RA.

IL-6 is known as a cytokine that cooperates with TGFβ to induce expression of IL-17A by Thl7 cells (Ivanov, II et al., Cell 126, 1121-1133, 2006; and Gaffen, S. L., Current opinion in immunology 23, 613-619, 2011). In addition, it is also reported that IL-6 is secreted from an intervertebral disc even in the absence of macrophages (Rand et al., Spine 22, 2598-2601, 1997), and an expression level thereof is increased in an intervertebral disc hernia cell (Andrade, P. et al., European spine journal 22, 714-720, 2013). Further, it is shown that IL-6 accelerates degeneration by causing a reduction of an extracellular matrix production in an intervertebral disc (Kang, J. D. et al., Spine 21, 271-277, 1996; Phillips, K. L. et al., Arthritis research & therapy 15, R213, 2013; Studer. R.K. et al., Spine 36, 593-599, 2011; and Patel, K. P. et al., Spine 32, 2596-2603, 2007), IL-6 contributes to expression of an inflammatory mediator such as TNFα and PGE-2 (Phillips, K. L. et al., 2013, supra; and Patel, K. P. et al., 2007, supra), and IL-6 causes neuropathic pain (Murata, Y. et al., Spine 36, 926-932, 2011; and Murata. Y, et al., Spine 33, 155-162, 2008). Therefore, since IL-6 plays an important role in progression of nucleus pulposus cell degeneration and symptoms associated with degenerative diseases, it can be expected that, by controlling the expression of IL-6, the progression of intervertebral disc degeneration is suppressed and the symptoms associated with degenerative diseases is alleviated.

It is known that cyclooxygenase-2 (COX-2) is a key enzyme for biosynthesis of prostaglandin in an intervertebral disc cell (Miyamoto et al., Spine 27, 2477-2483, 2002; and van Dijk. B. et al., Journal of orthopaedic research 33, 1724-1731, 2015) and the biosynthesis thereof is induced by mechanical stress to trigger degenerative cascade (Seibert, K. et al., Proceedings of the National Academy of Sciences of the United States of America 91, 12013-12017, 1994; and Williams, C. S. et al., Oncogene 18, 7908-7916, 1999). In addition, it is reported that IL-6 is related to production of COX-2 (Studer. R.K. et al, 2011, supra; and van Dijk. B. et al., 2015, supra). Therefore, it can be expected that, also by suppressing expression of COX-2, the progression of intervertebral disc generation is suppressed and the symptoms associated with degenerative diseases are alleviated.

Microsomal prostaglandin E synthase-1 (mPGES1) is selectively and functionally associated with COX-2 to produce prostaglandin E2 (PGE2). PGE2 causes sensitization, which leads to severe back pain (Kang, J. D. et al., 1996, supra).

Matrix metalloproteinases-3 (MMP-3) and matrix metalloproteinases-13 (MMP-13) are proteins known as stromemycin-1 and collagenase 3, respectively, and when an extracellular matrix such as collagen fibers or hydrophilic proteoglycan is separated by MMP-3 and MMP-13, an intervertebral disc degeneration process is promoted (Antoniou, J. et al., The Journal of Clinical Investigation 98, 996-1003, 1996).

CXCL1 is one of chemokines that induces activation or migration of neutrophils and is involved in formation of inflammation (Charo et al., N Engl J Med. 354, 610-621, 2006). CXCL1 is produced from macrophages, mast cells, or keratinocytes (De Filippo et al., Blood. 121, 4930-4937, 2013; and Lowes et al., Trends Immunol. 34.174-181, 2013). CXCL1 produced from these cells is also produced by stimulation of IL-17A (Iwakura et al., Immunity. 34, 149-162, 2011). In a disease state of psoriasis, it is considered that infiltration of neutrophils into the stratum corneum is caused by promotion of production of CXCL1 by an action of IL-17A on keratinocytes, which is involved in formation of microabscess and is thus involved in epidermal hyperplasia or abnormal keratinization (Girolomoni et al., Br J Dermatol., 167(4), 717-724, 2012; and Lin et al., FASEB. 32, 2018). In addition, it is reported that p38 or JNK which is a MAPK factor is activated by stimulation of inflammatory cytokine such as TNFα, which is likely to promote expression of CXCL1 (Shieh et al., Cell Physiol BioChem. 34, 1373-1384, 2014).

In still another embodiment of the present invention, a gene whose expression is enhanced by binding of IL-17A to IL-17RA is a gene whose expression is enhanced by phosphorylation of p38. COX-2, IL-6, CXCL1, and the like are presumed to be these genes.

It is reported that expression of COX-2 is activated by phosphorylation of p38 and c-Jun N-terminal kinase (JNK) by IL-17A in a p38 pathway and a JNK pathway, respectively, among mitogen-activated protein kinase (MAPK) pathways (see Fig. 45) (Li. J. K. et al., Journal of translational medicine 14, 77, 2013). As described in [Example 3] (Fig. 43), in the present invention, it is considered that at least the phosphorylation of p38 can be suppressed by administration of the expression regulator, which also affects suppression of expression of each of COX-2, IL-6, and CXCL1.

The use of the expression regulator of the present invention is not particularly limited. The expression regulator can be used in various situations in vitro, ex vivo, and in vivo depending on a purpose of regulating an expression level of a gene whose expression level is changed by binding of IL-17A to IL-17RA in a cell expressing IL-17RA.

It is preferable that a cell expressing IL-17RA targeted by the expression regulator of the present invention is, for example, an intervertebral disc nucleus pulposus cell or an epidermal cell. It is more preferable that an intervertebral disc nucleus pulposus cell cultured under a low oxygen condition (for example, an oxygen concentration in atmosphere of a medium is about 1%) or an intervertebral disc nucleus pulposus cell present in an intervertebral disc tissue (nucleus pulposus) is targeted as the intervertebral disc nucleus pulposus cell.

The intervertebral disc nucleus pulposus cell, the epidermal cell, and other cells expressing IL-17RA may be human cells or non-human mammalian cells, for example, cells from disease model animals such as non-human primates (a cynomolgus macaque, a rhesus macaque, a chimpanzee, and the like), a cow, a pig, a mouse, and a rat. That is, the expression regulator of the present invention may target human IL-17RA or non-human mammalian (for example, a rat used in examples) IL-17RA. The intervertebral disc nucleus pulposus cell, the epidermal cell (keratinocyte or the like), and other cells expressing IL-17RA may be a primary cell or a passage cell thereof collected from a tissue including a cell expressing IL-17RA such as a human or non-human mammalian intervertebral disc tissue (nucleus pulposus) or a skin tissue (epidermis), and may be an established (immortalized) cell line.

It is desirable that, when the cell expressing IL-17RA is cultured in vitro or ex vivo, the culturing is performed under a microenvironment of a tissue in which the cell expressing IL-17RA is present, in particular, under a condition as close as possible to a microenvironment in which symptoms of inflammation or degeneration occur. For example, it is desirable that the intervertebral disc nucleus pulposus cell is cultured under a low oxygen condition close to the degenerated intervertebral disc tissue (nucleus pulposus). The "low oxygen condition" generally refers to a condition in which an oxygen concentration in atmosphere of a medium is 0.5 to 10%, and preferably 1 to 5%, for example, about 1%. The intervertebral disc nucleus pulposus cell may be cultured under conditions such as an acidic condition, a low glucose (hypoglycemic) condition, and a high osmotic pressure condition, if necessary. The "acidic condition" refers to a condition in which, for example, a pH of the medium is in a range of 6.5 to 7.4 or less at room temperature (for example, 25°C). The "low glucose condition" refers to a condition in which, for example, a glucose concentration in the medium is %4.5 g/L or less.

In an embodiment of the present invention, the expression regulator is used as a medicament of the present invention (in a case where a medicament is prepared as a composition, as an active ingredient thereof) to be described below. In other words, in an embodiment of the present invention, the expression regulator is used to prepare a medicament (pharmaceutical composition) of the present invention.

In an embodiment of the present invention, the expression regulator is used in a method of regulating expression of a gene whose expression level is changed by binding of IL-17Ato IL-17RAto be described below.

### -Medicament for Treatment or Prophylaxis-

A "medicament for treatment or prophylaxis" provided in an aspect of the present invention is a medicament containing the IL-17A activity inhibitor of the present invention or the expression inhibitor of the present invention, as an active ingredient. The drug is a drug for treating or prophylaxis of a "disease with a symptom associated with binding of IL-17A to IL-17RA".

The "treatment" (which can also be referred to as "remedy") refers to any attenuation or amelioration of a disease, disorder, or condition based on any objective or subjective parameters such as alleviating, remitting, or reducing a symptom; making a disease, disorder, or condition more tolerable to a target (for example, by alleviation of pain or itchiness); slowing down the rate of degeneration or exacerbation; debilitating a degree of the final point of degeneration or exacerbation; improving physical or mental health of a target; and prolonging a survival period. The "prophylaxis" refers to suppression of an occurrence of a symptom. The effects of the "treatment" and the "prophylaxis" can be evaluated based on the objective or subjective parameters including the results of a physical test and/or neurological test (psychiatric assessment).

The "disease with a symptom associated with binding of IL-17A to IL-17RA" is not particularly limited. Examples thereof can include a disease generally classified into an inflammatory disease, an allergic disease, and an immunologic disease, such as inflammatory skin diseases such as psoriasis vulgaris, articular psoriasis, pustular psoriasis, and psoriatic erythroderma; inflammatory articular diseases such as ankylosing spondylitis and rheumatoid arthritis; inflammatory large intestinal diseases such as Crohn's disease; autoimmune diseases such as Behcet's disease; and an organ or tissue transplantation rejection and sepsis. The medicament of the present invention may be formulated into a form that is suitable for delivery to an organ, a tissue, or a cell associated with a symptom of each disease.

In a representative embodiment of the present invention, the medicament of the present invention is a medicament for treating or prophylaxis of a disease with a symptom associated with binding of IL-17A to IL-17RA, such as a disease in which intervertebral disc (nucleus pulposus) inflammation or degeneration appears as symptoms thereof, for example, a lumbar or cervical intervertebral disc disease, intervertebral disc hernia, cervical spondylotic myelopathy, radiculopathy, spondylolysis and spondylolisthesis, lumbar spinal canal stenosis, lumbar degenerative spondylolisthesis, or lumbar degenerative scoliosis. In the embodiment, the medicament of the present invention is formulated into a form that is suitable for delivery to a cell in an intervertebral disc tissue (nucleus pulposus, transition zone, or annulus fibrosus), in particular, to a nucleus pulposus cell. The intervertebral disc tissue may be a tissue with a certain degree of degeneration, aging, disorder, damage, or the like (including a healthy tissue with substantially no degeneration or the like) and may be a herniated tissue.

In a more representative embodiment of the present invention, the medicament of the present invention is a medicament for treating or prophylaxis of a disease with a symptom associated with binding of IL-17A to IL-17RA, such as an inflammatory skin disease such as psoriasis vulgaris, articular psoriasis, pustular psoriasis, or psoriatic erythroderma. In the embodiment, the medicament of the present invention is formulated into a form that is suitable for delivery to a cell in a skin tissue (epidermis or dermis), in particular, to a cell in a stratum basale, a stratum spinosum, a stratum granulosum, or a stratum corneum of epidermis (keratinocyte or corneocyte). The skin tissue may be a tissue with a certain degree of symptoms of erythema, infiltration and hypertrophy, scale, or desquamation. In addition, in psoriasis, in addition to the symptoms of skin, symptoms such as pain or deformation of joint may appear, and any symptom of skin and joint can also be a target of the treatment or the prophylaxis.

The medicament of the present invention can be produced (prepared as a pharmaceutical composition) using the IL-17A activity inhibitor of the present invention or the expression inhibitor of the present invention and a pharmaceutically acceptable carrier by a method known in the field of formulation technology. Examples of a formulation of the medicament can include a formulation for parenteral administration (for example, a liquid preparation such as an injection) in which a customary adjuvant such as a buffer and/or a stabilizer is contained, and a topical formulation such as an ointment, a cream, a liquid preparation or a salve in which a customary pharmaceutical carrier is contained.

The "target" to which the medicament of the present invention is administered refers to a target (for treatment) with the disease with a symptom associated with binding of IL-17A to IL-17RA or a target (for prophylaxis) which is likely to have the disease with a symptom associated with binding of IL-17A to IL-17RA. In addition, the "target" may be a human or a non-human mammal, for example, a disease model animal such as non-human primates (a cynomolgus macaque, a rhesus macaque, a chimpanzee, and the like), a cow, a pig, a mouse, and a rat.

The medicament of the present invention may be administered in an effective dose for exerting a desired treatment or prophylaxis effect. The effective dose can be appropriately adjusted by an administration dose, the number of times of administrations, and an administration interval (the number of times of administrations within a predetermined period) per time while taking into consideration of a dosage form, an administration target, an administration route, and the like.

The medicament of the present invention may be administered in an effective dose for exerting a desired treatment or prophylaxis of effect. The effective dose can be appropriately adjusted by an administration dose, the number of times of administrations, and an administration interval (the number of times of administrations within a predetermined period) per time while taking into consideration of a dosage form, an administration target, an administration route, and the like.

### -Screening Method for IL-17A Activity Inhibitor-

A "screening method for an IL-17A activity inhibitor" provided in an aspect of the present invention includes: from a three-dimensional molecular model of a space surrounded by amino acid residues of Phe60, Gln87, Asp121, Pro122, Asp123, Gln124, Asp153, Cys154, Glu155, Lys160, Pro164, Cys165, Ser167, Ser168, Gly169, Ser170, Leu171, Trp172, Asp173, Pro174, Pro254, Phe256, Ser258, Cys259, Asp262, Cys263, Leu264, and His266 that are contained in an extracellular domain of IL-17RA, and a three-dimensional molecular model of a candidate compound, evaluating binding stability between the candidate compound and IL-17RA through a non-covalent interaction including a van der Waals force generated between an atom or an atomic group included in at least 13 amino acid residues among the amino acid residues and an atom or an atomic group included in the candidate compound, to determine whether the candidate compound has an action of inhibiting binding of IL-17A to IL-17RA by binding to IL-17RA competitively with IL-17A.

The screening method for an IL-17A activity inhibitor may further include comparing binding stability of the candidate compound with binding stability of each of the compounds (1) to (36). The screening method for an IL-17A activity inhibitor of the embodiment is preferably used, for example, for preparing derivatives of the compounds (1) to (36), and in particular, for preparing derivatives having improved IL-17A activity inhibiting ability as compared to those of the compounds (1) to (36).

In the present specification, the "IL-17A activity inhibitor" and the matters described in other inventions can apply a "binding inhibiting method".

### -Binding Inhibiting Method-

A "binding inhibiting method" provided in an aspect of the present invention is a method for inhibiting binding of IL-17A to IL-17RA, the method including bringing the IL-17A activity inhibitor of the present invention into contact with IL-17RA as described above.

The contact of the IL-17A activity inhibitor with IL-17RA can be performed in vitro, ex vivo, or in vivo, in other words, in a living body of a human or another animal or outside of a living body of a human or another animal.

In the present specification, the "IL-17A activity inhibitor" and the matters described in other inventions can apply the "binding inhibiting method".

### -Expression Regulation Method-

An "expression regulation method" provided in an aspect of the present invention is for regulating expression of a gene whose expression level is changed by binding of IL-17A to IL-17RA, the method including bringing the IL-17A activity inhibitor of the present invention into contact with a cell expressing IL-17RA as described above.

The contact of the IL-17A activity inhibitor with IL-17RA can be performed in vitro, ex vivo, or in vivo, in other words, in a living body of a human or another animal or outside of a living body of a human or another animal.

In the present specification, the "expression regulator" and the matters described in other inventions can apply the "expression regulation method".

### -Treatment Method-

A "treatment method" provided in an aspect of the present invention includes administering the IL-17A activity inhibitor, expression regulator, or medicament of the present invention as described above to a target with the "disease with a symptom associated with binding of IL-17A to IL-17RA" or a target who is likely to have the "disease with a symptom associated with binding of IL-17A to IL-17RA".

In the present specification, the "medicament for treatment or prophylaxis" and the matters described in other inventions can apply the "treatment method".

### [Examples]

### [Reference Example 1] Immunostaining of IL-17A Expressed in Human Intervertebral Disc Nucleus Pulposus Tissue

Patients gave written informed consent in accordance with the Declaration of Helsinki. Ethical approval was obtained from the ethics committee in Tokai University School of Medicine. A total of 10 samples of intervertebral disc tissues were resected from three lumbar intervertebral disc hernia patients below the age of 16 and three idiopathic scoliosis patients below the age of 16. As a result of evaluating a degeneration level of each of the resected intervertebral disc samples according to the Pfirrmann classification on MRI, the samples resected from the lumbar intervertebral disc hernia patients (grades 3, 4, and 5) were degenerated, whereas the intervertebral disc samples resected from the idiopathic scoliosis patients were normal (grades 1 and 2).

In order to check an expression level of IL-17A, these intervertebral disc samples were subjected to tissue immunostaining according to the following procedure. The sample was fixed in PBS containing 4% paraformaldehyde and embedded in paraffin. A section was deparaffinized with xylene and re-hydrated with ethanol whose concentration was diluted in a stepwise manner, and then the section was incubated in anti-IL-17A antibodies (#bs-2140R, Bioss, specific to human IL-17A) diluted with PBS containing 1% BSA at 4°C overnight. Subsequently, the sample was stained with a horseradish peroxidase (HRP)-conjugated goat anti-rabbit IgG antibody (Sigma-Aldrich Co., LLC) and visualized by a reaction with diaminobenzidine (NACALAI TESQUE, INC.). A cell nucleus was stained with hematoxylin. All of the samples were observed with a microscope (IX70, Olympus Corporation), a total number of cells included in a high-magnification field and the number of stained cells in each sample were measured to calculate a percentage of the latter to the former.

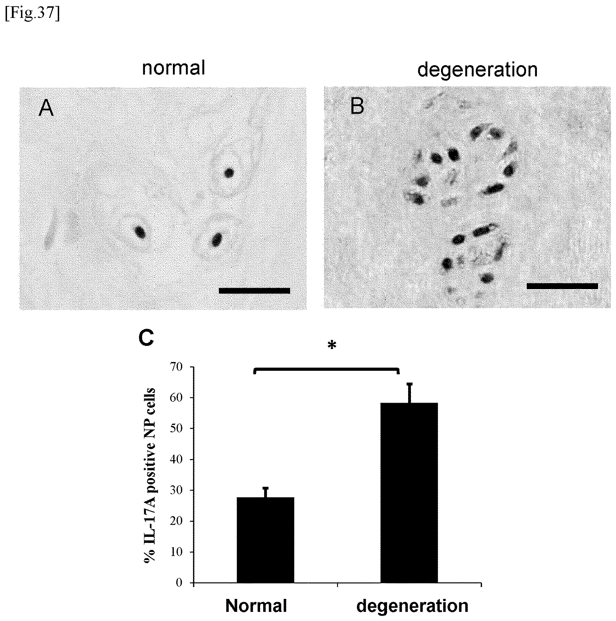

The results are shown in Fig. 37. It was confirmed that, in the degenerated intervertebral disc tissue (degeneration), the staining of IL-17A on the image was prominent as compared with the normal intervertebral disc tissue (normal), and a percentage of the nucleus pulposus cells expressing IL-17A (positive) was significantly high.

### [Reference Example 2] Action of Stimulation of IL-17A on Expression Levels of Various Genes in Rat Nucleus Pulposus Cell

Nucleus pulposus cells were separated from a Sprague Dawley rat aged 11 weeks according to a method in Risbud et al (Journal of cellular biochemistry 98, 152-159, 2006; doi:10.1002/jcb.20765). In short, lumbar and coccygeal intervertebral discs of a deeply anesthetized rat were dissected under an aseptic condition, gel-like nucleus pulposus was separated from intervertebral disc annulus fibrosus (AF), the nucleus pulposus was minced and pipetted, and then the nucleus pulposus was cultured in a Dulbecco's Modified Eagle Medium (DMEM) in which 20% FBS and antibiotics were added at 20%O₂, 5%CO₂, and 37°C for about 1 to 2 weeks, and then was cultured in DMEM in which 10%FBS and antibiotics were added for about 1 to 2 weeks. The nucleus pulposus cells thus obtained were cultured in a low oxygen chamber (MIC-101, Billups Rothenberg Inc., USA) containing 1%O₂, 5%CO₂, and 94%N₂ for 15 minutes to 24 hours.

The cultured rat nucleus pulposus cells were treated with 20 or 50 ng/mL of recombinant mouse IL-17A (Pepro Tech Inc., USA, #210-17) for 24 hours, expression levels of mRNAs of IL-6, COX-2, mPGES1, MMP-3, and MMP-13 were determined by real time RT-PCR according to the following procedure. Total RNA was extracted from the nucleus pulposus cells using an RNAeasy mini-column (Qiagen, Germany). Before elution from the column, RNA was treated with RNase-Free DNase I (Qiagen, Germany). The purified DNA-free RNA was transformed into cDNA using a High Capacity cDNA Reverse Transcription Kit (Applied Biosystems, USA). Template cDNA and a primer specific to each gene were added to Power SYBR Green master mix (Applied Biosystems) and an expression level of mRNA of each gene was determined using Step One Plus Real-time PCR System (Applied Biosystems). The expression level was normalized to β actin. It was verified by melting curve analysis that RT-PCR was specific and a primer dimer was not formed.

The results are shown in [A] of Fig. 38. In the evaluation by the real time PCR, it was observed that, in particular, IL-6 and COX-2 were remarkably increased and MMP-3, MMP-13, and mPGES1 were significantly increased, as compared with a non-treated group (cont).

In rat nucleus pulposus cells subjected to treatment for 24 hours in 50 ng/mL of IL-17A in which the most remarkable increases of IL-6 and COX-2 were observed, an expression level of a protein of each of IL-6 and COX-2, and β actin used as a control was determined by western blotting according to the following procedure. The nucleus pulposus cells were placed on ice and then washed with ice-cold PBS. In order to prepare total cell proteins, the cells were lysed with a lysis buffer containing 10 mM Tris-HCl (pH 7.6), 50 mM NaCl, 5 mM EDTA, 1% Nonidet P-40, a complete protease inhibitor cocktail (Roche AG, USA), 1 mM NaF, and 1 mM Na₃VO₄. The proteins were fractionated by SDS-PAGE and transferred onto Immobilon-P polyvinylidene difluoride membrane (Millipore Corporation, USA). The membrane was blocked with a blocking buffer (PBS in which 5%BSA and 0. 1%NaN₃ were dissolved), and the membrane was incubated in anti-IL-6 antibodies (#bs-0782R, Bios), anti-COX-2 antibodies (#NB100-689SS, Novus Biologicals), or anti-β actin antibodies (#A2228, Sigma-Aldrich Co., LLC) at 4°C overnight. Each antibody was diluted with a Can Get Signal Immunoreaction Enhancer Solution (Toyobo Co., Ltd., Japan). A chemiluminescent signal was visualized using an immobilion western chemilunescent HRP substrate (Millipore Corporation) and was scanned using Ez-Capture MG imaging system (ATTO Corporation, Japan). Western blotting data were quantified by densitometric scanning of a film using Macintosh computer software "CS Analyzer" (ATTO Corporation, Japan). In this case, a concentration of a band of each gene was normalized by a concentration of a band of β actin used as a control.

The results are shown in [B] of Fig. 38. It was observed that the expression levels of COX-2 and IL-6 as proteins were also significantly increased by performing a treatment of administering 50 ng/ml of recombinant mouse IL-17Ato the rat nucleus pulposus cell for 24 hours.

Further, in the rat nucleus pulposus cells treated with 50 ng/mL of recombinant mouse IL-17A for 24 hours, transcriptional activity of COX-2 was measured by a promoter assay method according to the following procedure. 24 hours before transfection, the rat nucleus pulposus cells were transferred to a 96-well plate (8 x 10³ cells/well). phPES2-1432/+59 (provided by Dr. Akihiko Hiyama of Tokai Universty) which is a plasmid including a construct of COX-2 promoter and luciferase (Hiyama A., et al., Journal of orthopaedic research 33, 1756-1768, 2015; doi:10.1002/jor.22959) or pGL4.74 which is a backbone plasmid including only a Renilla reniformis luciferase gene (Promega Corporation, USA) as an internal control was transfected to the cells. Lipofectamine 2000 (Invitrogen, USA) was used as a transfection reagent. The cells were cultured under a low oxygen condition for 24 hours, reporter activity thereof was measured. Activity of each of firefly luciferase and Renilla luciferase was measured by dual-luciferase reporter assay system (Promega Corporation) using a luminometer (TD-20/20, Turner Designs Inc., USA).

The results are shown in [C] of Fig. 38. It was observed that the transcriptional activity of COX-2 was significantly increased by performing a treatment of administering 50 ng/ml of recombinant mouse IL-17Ato the rat nucleus pulposus cells for 24 hours.

### [Reference Example 3] Reaction When IL-17A Activity Is Suppressed by Anti-IL-17A-Neutralizing Antibody

An expression level of mRNA of each of IL-6, COX-2, mPGES1, MMP-3, and MMP-13 was determined, an expression level of a protein of each of IL-6 and COX-2 was determined, and transcriptional activity of COX-2 was measured according to the same procedure as that of [Reference Example 2], except that a group to which a solution was administered was provided in advance, the solution being prepared by mixing 50 ng/ml of recombinant mouse IL-17A with 0.5 µg/ml of anti-IL-17A antibody (#DDX0336P-50, Novus Biologicals LLC, specific to human and mouse IL-17A) as a neutralizing antibody thereof and performing a reaction for 1 hour.

The results are shown in each of [A], [B], and [C] of Fig. 39. It was observed from [A] that all expression levels of mRNAs of IL-6, COX-2, mPGES1, MMP-3, and MMP-13 were significantly reduced in an anti-IL-17A neutralizing antibody combination group as compared with an IL-17A single administration group ("IL-17A" is "+" and "anti-IL-17A" is "-"). It was observed from [B] that the expression level of the protein of each of IL-6 and COX-2 was also significantly reduced in the anti-IL-17A neutralizing antibody combination group as compared with the IL-17A single administration group. It was observed from [C] that the transcriptional activity of COX-2 was also significantly reduced in the anti-IL-17A neutralizing antibody combination group as compared with the IL-17A single administration group. It was confirmed from these results that an enhancing action of IL-17A on the expression level of each gene was inhibited by the anti-IL-17A neutralizing antibody.

### [Reference Example 4] Action of Stimulation of IL-6 on Expression Levels of Various Genes in Rat Nucleus Pulposus (NP) Cell

IL-6 whose mRNA expression level was remarkably increased by IL-17A was used as an analysis target, and an influence of IL-6 on a rat NP cell was evaluated. 50 ng/ml of IL-6 was administered to the rat NP cells, the cells were cultured under a 1% oxygen condition for 24 hours, and an expression level of mRNA of each of COX-2, IL-17A, MMP-3, and MP-13 was determined by real time RT-PCR according to the same procedure as that of [Reference Example 2]. Further, an expression level of a protein of COX-2 was determined and transcriptional activity of COX-2 was evaluated according to the same procedure as that of [Reference Example 2].

The results are shown in each of [A], [B], and [C] of Fig. 40. It was observed from [A] that the expression level of mRNA of each of the COX-2, MMP-3, and MMP-13 was significantly increased in an IL-6 administration group as compared with the non-treated group, but the expression level of mRNA of IL-17A was not significantly changed. It was observed from [B] that the expression level of the protein of COX-2 was significantly increased in the IL-6 administration group as compared with the non-treated group. It was observed from [C] that the transcriptional activity of COX-2 was also significantly increased in the IL-6 administration group as compared with the non-treated group.

### [Example 1] Evaluation of Compound of Present Invention as IL-17A Activity Inhibitor in Rat Nucleus Pulposus (NP) Cell

(A) An expression level of mRNA of each of IL-6, COX-2, mPGES1, MMP-3, and MMP-13 was determined, (B) an expression level of a protein of each of IL-6 and COX-2 was determined, and (C) transcriptional activity of COX-2 was measured according to the same procedure as that of [Reference Example 2], except that a group to which a solution was administered was provided in advance, the solution being prepared by mixing 50 ng/ml of recombinant mouse IL-17A with any one of 50 µg/ml of the compound (3) (STK630921), 50 µg/ml of the compound (2) (PB203263256), 50 µg/ml of the compound (5) (Z9215), and 50 µg/ml of the compound (11) (P2000N-53454), in other words, according to the same procedure as that of the "anti-IL-17A neutralizing antibody combination group" of [Reference Example 3], except that any one of the compounds (3), (2), (5), and (11) with a concentration of 50 µg/ml was used instead of the anti-IL-17A antibody with a concentration of 0.5 µg/ml. In (B) and (C), among the compounds of the present invention, only the compound (3) which is considered from the results of (A) described below to have the highest effect on IL-6 and COX-2 was used.

The results are shown in each of [A], [B], and [C] of Fig. 41. It was observed from [A] that all expression levels of mRNAs of IL-6, COX-2, mPGES1, MMP-3, and MMP-13 were significantly reduced in a group in which IL-17A and any one of the compounds (3), (2), (5), and (11) of the present invention were used in combination as compared with a group in which only IL-17A was administered, and in particular, the expression level of mRNA of each of IL-6 and COX-2 was remarkably reduced in the case of the compound (3). It was observed from [B] that the expression level of the protein of each of IL-6 and COX-2 was also significantly reduced in the IL-17+STK group as compared with the IL-17 group. It was observed from [C] that the transcriptional activity of COX-2 was also significantly reduced in the IL-17+STK group as compared with the IL-17 group. It was confirmed from these results that the compounds of the present invention had an action of inhibiting the enhancing action of IL-17A on the expression level of each gene, similarly to the anti-EL-17A neutralizing antibody.

As a result of determining an expression level of mRNA of IL-6 by using the compound (9) (F3382) as the compound of the present invention in the same manner as described above, it was confirmed that the expression level thereof was significantly reduced in a group in which IL-17A and the compound (9) were used in combination as compared with the group in which only IL-17A was administered (*p < 0.05, not illustrated), and similarly to the other compounds of the present invention, the compound (11) also had an action of inhibiting the enhancing action of IL-17A on the expression level of each gene.

### [Example 2] Evaluation of Compound of Present Invention as IL-17A Activity Inhibitor in Human Nucleus Pulposus (NP) Cell

An expression level of mRNA of each of IL-6 and COX-2 was determined according to the same procedure as that of [Example 1], except that the sample was changed from the rat NP cells to the human NP cells (obtained in [Reference Example 1]), and the compound 1 (STK) was used as the compound of the present invention at two concentrations of 50 µg/ml and 100 µg/ml.

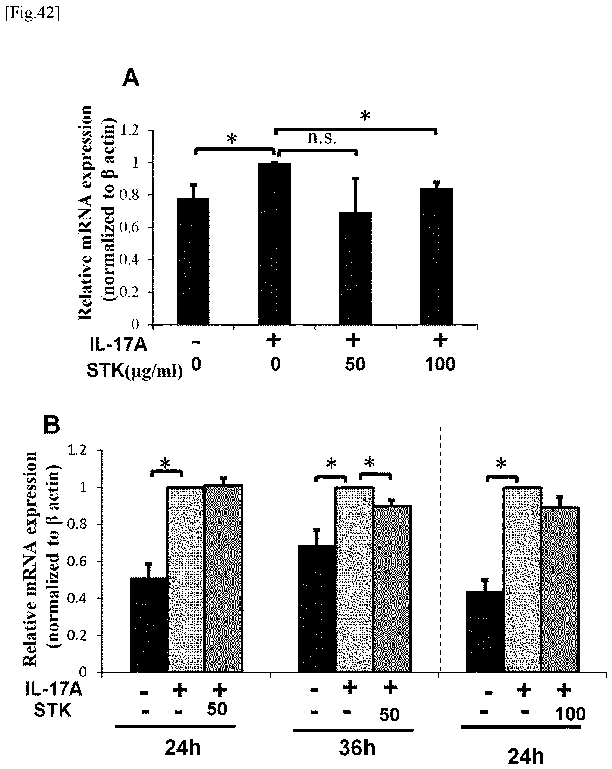

The results are shown in Fig. 42. The expression of mRNA of IL-6 in the human NP cell tended to be reduced after the administration of 50 µg/ml of STK for 24 hours, and was significantly reduced by the administration of 100 µg/ml of STK as compared with the IL-17A single administration group. In the expression of mRNA of COX-2, a clear suppression effect was not observed 24 hours after the administration of 50 µg/ml or 100 µg/ml of STK, but a significant reduction was observed 36 hours after the administration of 50 µg/ml of STK.

### [Example 3] Verification of Actions of IL-17A and Compound of Present Invention on MAPK Pathway

It is reported that IL-17A is likely to be involved in the expression of COX-2 through a MAPK pathway. Involvement of IL-17A in MAPK factors (p38, JNK, and ERK) with respect to the expression of each of COX-2 and IL-6, and influence of the compound (1) of the present invention on these MAPK factors were evaluated by the following method.

Ap38 phosphorylation inhibitor "SB203580" with a concentration of 10 µM, a JNK phosphorylation inhibitor "SP600125" with a concentration of 10 µM, or an ERK phosphorylation inhibitor "PD98059" with a concentration of 10 µM was administered to rat NP cells together with recombinant mouse IL-17A with a concentration of 50 ng/ml, or alternatively, these inhibitors were not administered, and the cells were cultured under a 1% oxygen condition for 24 hours, and an expression level of mRNA of each of COX-2 and IL-6 was determined by real time RT-PCR according to the same procedure as that of [Reference Example 2].

The results are shown in [A] and [B] of Fig. 43. It was confirmed that, in each administration group of SB, SP, and PD, the expression level of mRNA of COX-2 was significantly suppressed, and in each administration group of SB and PD, the expression level of mRNA of IL-6 was significantly suppressed. From these results, it was shown that the activation of each of p38, JNK, and ERK is likely to be involved in the expression of COX-2 by IL-17A, and the activation of each of p38 and ERK is likely to be involved in the expression of IL-6.

Next, 50 µg/ml of the compound (1) was administered to rat NP cells together with IL-17A with a concentration of 50 ng/ml, or the administration was omitted, the cells were cultured under a 1% oxygen condition for 15 minutes or 30 minutes, and an expression level of a protein of each of phosphorylated p38, p38, phosphorylated JNK, JNK, phosphorylated ERK, and ERK was determined by western blotting according to the same procedure as that of [Reference Example 2].

The results are shown in [C], [D], [E], and [F] of Fig. 43. The phosphorylation of p38 was reduced 15 minutes after the administration of the compound (1) (C, E), and a significant reduction thereof was observed 30 minutes after the administration as compared with the IL-17A single administration group (D, F). Therefore, it was shown that IL-17A promotes the phosphorylation (activation) of p38 and ERK in the MAPK pathway, and the administration of the compound (1) has at least an effect of suppressing the activation of p38 by IL-17A, and as a result, the compound (1) is likely to be involved in suppression of the expression of COX-2 or IL-6.

### [Comparative Example 1]

An expression level of mRNA of COX-2 was determined according to the same procedure as that of [Reference Example 2], except that a group to which a solution was administered (synd group) was provided in advance, the solution being prepared by mixing 50 ng/ml of recombinant mouse IL-17A with 50 µg/ml of the compound of Non-Patent Document 3 (Liu et al., Science Signaling 2017) and performing a reaction for 1 hour. In addition, the expression level of mRNA of COX-2 in the synd group was compared with the expression level of mRNA of COX-2 in the IL-17+STK group obtained in [Example 1].

The results are shown in [A] and [B] of Fig. 44. The action of reducing the expression level of mRNA of COX-2 in the rat NP cells by inhibiting the activity of IL-17A was not observed in the compound of Non-Patent Document 3, and the compound (1) of the present invention was excellent in the action.

### [Example 4] Observation of Treatment Effect of Medicament Containing Compound of Present Invention Using Mouse Psoriasis Skin Model

About 1 x 1.5 cm of back of 10-week-old male BJ6J mouse was shaved, and imiquimod (IMQ, a drug causing psoriasis-like dermatitis in a mouse) cream was applied every day from day 1 to day 4. From the 5^{th} day (day 5) after the first IMQ cream application, 6 to 8 hours after the application of the IMQ cream, a DMSO solution containing 1 mg of the compound (3) (database registration name: STK630921) was applied (STK group = compound (3) treated group). The same IMQ cream and the solution of the compound (3) were applied every day from the 6^{th} day (day 6) to the 9^{th} day (day 9). As a control group, a group to which DMSO which is a solvent of the solution was applied in the same amount as that in the STK group instead of the IMQ cream and the DMSO solution containing 1 mg of the compound (3) from the 5^{th} day (day 5) to the 9^{th} day (day 9) (Sham group); a group to which only the IMQ cream was applied from the 5^{th} day (day 5) to the 9^{th} day (day 9) (IMQ group); and a group not subjected to the first IMQ cream application and the treatment from the 5^{th} day (day 5) to the 9^{th} day (day 9) (normal group) were provided. Three mice were used in each group.

On the 10^{th} day (day 10), skin of the mice in each of the STK group, the Sham group, the IMQ group, and the normal group was collected, and one sample obtained by hematoxylin eosin (HE) staining and one sample obtained by immunofluorescence staining using an anti-CXCLl antibody were prepared per mouse. In the HE stained samples, for each sample, a thickness of an epidermis layer was measured at two or more locations with the same magnification field of view, and an average value was statistically analyzed (significant difference: p < 0.05, n = 3). In the immunofluorescence stained samples, for each sample, an area exhibiting fluorescence intensity of a predetermined value or more (that is, expression of CXCL1 is positive) within a designated range of the same area was measured using image analysis software "Image J" (National Institutes of Health: NIH), and statistical analysis was performed (significant difference: p < 0.05, n = 3).

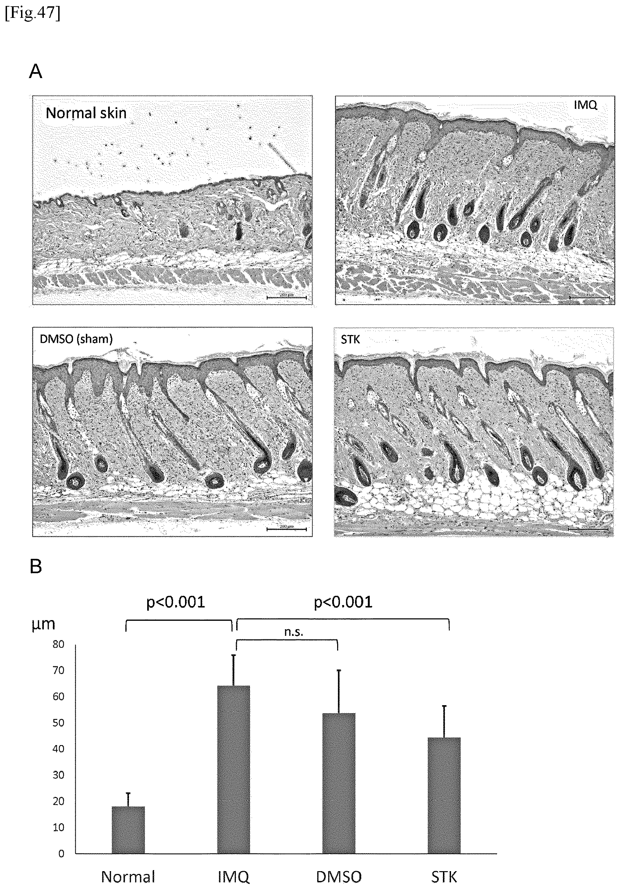

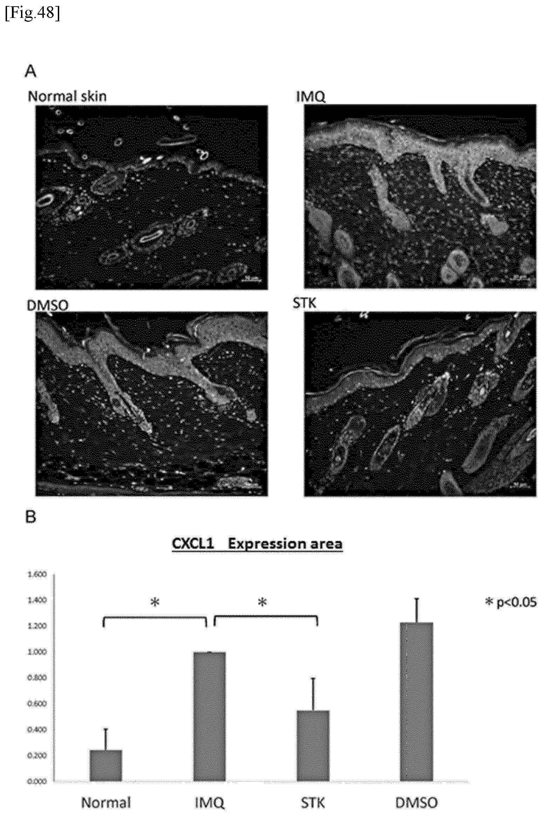

The results of the thickness of the epidermis layer and the expression of CXCL1 are shown in Figs. 47 and 48, respectively. In the STK group (compound (3) treated group), the thickness of the epidermis layer exhibiting abnormal hypertrophy which is a representative symptom of psoriasis was significantly reduced (p < 0.001), and the expression of CXCL1 which is one of the factors causing epidermis inflammation in psoriasis was significantly reduced (p < 0.05), that is, the effect of treating psoriasis was confirmed.

### [Example 5] Observation of Treatment Effect of Medicament Containing Compound of Present Invention Using Rat Intervertebral Disc Degeneration Model

A23G needle was inserted into a caudal intervertebral disc of a 11-weeks-old male SD rat (weight: 300 to 350 g) by about 5 mm, rotated at 360°, and left for 30 seconds to cause intervertebral disc degeneration (day 0). 14 days (day 14) after being subjected to the intervertebral disc degeneration, 10 µL of a DMSO solution containing 1 mg of the compound (3) (database registration number: STK630921) was injected to the degenerated intervertebral disc (STK group = compound (3) treated group). As control groups, a group in which only DMSO which is a solvent of the solution was injected in the same amount as that in the STK group instead of 10 µL of the DMSO solution containing 1 mg of the compound (3) (Sham group), a group subjected no treatment after being subjected to the intervertebral disc degeneration (degeneration group), and a group not subjected to intervertebral disc degeneration and a subsequent treatment (normal group) were provided.

28 days (day 28) after being subjected to the intervertebral disc degeneration, the caudal vertebrae of the rats in the STK group, the Sham group, the degeneration group, and the normal group were collected, fixed in 4% PFA, and decalcified, thereby preparing sample sections. Each sample section was subjected to immunostaining using an anti-IL-6 antibody. In each sample subjected to the immunostaining, the number of IL-6 positive cells in spots of the same area arbitrarily set at 3 or 4 locations in the intervertebral disc tissue was counted with the same magnification field of view, and an expression rate of the IL-6 positive cells with respect to a total number of cells in the same spot was calculated.

The results are shown in Fig. 49. In the STK group (compound (3) treated group), the expression rate of the IL-6 positive cells were significantly reduced (p < 0.05), that is, the effect of treating intervertebral disc degeneration was confirmed.

## Claims

1. An interleukin-17A (IL-17A) activity inhibitor comprising:
a compound having an action of inhibiting binding of IL-17A to human or non-human animal interleukin-17 receptor A (IL-17RA), or a pharmaceutically acceptable salt, solvate, or prodrug thereof,
the compound being capable of binding to IL-17RA through a non-covalent interaction including a van der Waals force acting between the compound and at least 13 amino acid residues among 28 amino acid residues of Phe60, Gln87, Asp121, Pro122, Asp123, Gln124, Asp153, Cys154, Glu155, Lys160, Pro164, Cys165, Ser167, Ser168, Gly169, Ser170, Leu171, Trp172, Asp173, Pro174, Pro254, Phe256, Ser258, Cys259, Asp262, Cys263, Leu264, and His266 that are contained in an extracellular domain of human IL-17RA in a space surrounded by the 28 amino acid residues,
or being capable of binding to IL-17RA through a non-covalent interaction including a van der Waals force acting between the compound and at least 13 amino acid residues among amino acid residues (where homology between the amino acid residues is 80% or more) corresponding to the 28 amino acid residues contained in an extracellular domain of non-human animal IL-17RA in the space surrounded by the amino acid residues corresponding to the 28 amino acid residues.

2. The IL-17A activity inhibitor according to claim 1,
wherein the non-covalent interaction includes at least one intermolecular interaction selected from the group consisting of an ionic bonding, a hydrogen bonding, a CH-π interaction, a cation-π interaction, and a hydrophobic interaction,
the intermolecular interaction acting between the compound and at least one amino acid residue selected from the group consisting of Asp121, Pro122, Asp123, Gln124, Asp153, Cys154, Glu155, Lys160, Ser168, Ser170, Ser258, Asp262, Leu264, and His266.

3. The IL-17A activity inhibitor according to claim 2, wherein the intermolecular interaction includes at least a hydrogen bonding or CH-π interaction with Cys154.

4. The IL-17A activity inhibitor according to claim 2 or 3,
wherein the intermolecular interaction optionally includes at least one selected from the group consisting of a hydrogen bonding with Asp121, a CH-π interaction or hydrogen bonding with Pro122, a CH-π interaction or hydrogen bonding with Asp123, an ionic bonding, hydrogen bonding, or CH-π interaction with Lys160, and a CH-π interaction with Ser170.

5. An IL-17A activity inhibitor comprising a compound represented by General Formula (I) (hereinafter, referred to as a "compound (I)"), or a pharmaceutically acceptable salt, solvate, or prodrug thereof,
[Chem. 1]
A-L¹-B-L²-C-L³-D (I)
in General Formula (I),
A represents (A1) a C₃₋₁₀ cycloalkyl group which is optionally substituted, (A2) a C₃₋₁₀ cycloalkenyl group which is optionally substituted, (A3) a 6- to 14-membered aromatic hydrocarbon cyclic group (aryl group) which is optionally substituted, (A4) a 5- to 14-membered aromatic heterocyclic group which is optionally substituted, (A5) a 3- to 14-membered non-aromatic heterocyclic group which is optionally substituted, or (A6) a C₄₋₆ alkyl group which is optionally substituted,
L¹ represents (L¹1) a single bond, (L¹2) a C₁₋₃ alkylene group, which is optionally linked to a divalent group (amide bond) derived from a carbamoyl group and/or is optionally linked to an ether bond or a thioether bond, (L¹3) a divalent group (amide bond) derived from a carbamoyl group, which is optionally linked to a divalent group derived from an amino group, (L¹4) a sulfonyl group, or (L¹5) a C₁₋₃ alkenylene group (a carbon-carbon double bond is optionally formed with a carbon atom of B or C adjacent to L²),
B represents (B1) a divalent group (amide bond) derived from a carbamoyl group, which is optionally substituted and/or is optionally linked to a divalent group derived from a C₁₋₃ alkyl-carbonyl group, (B2) a divalent group derived from a 5- to 14-membered aromatic heterocyclic ring, which is optionally substituted, (B3) a divalent group derived from a 3- to 14-membered non-aromatic heterocyclic ring, which is optionally substituted, (B4) a C₃₋₁₀ cycloalkyl group which is optionally substituted, (B5) a C₃₋₁₀ cycloalkenyl group which is optionally substituted, (B6) a 6- to 14-membered aromatic hydrocarbon cyclic group (aryl group) which is optionally substituted, (B7) an ester bond or a thioester bond, or (B8) a keto group or a thioketo group,
L² represents (L²1) a single bond, (L²2) a C₁₋₆ alkylene group, or (L²3) a C₁₋₃ alkenylene group (a carbon-carbon double bond is optionally formed with a carbon atom of B or C adjacent to L²),
C represents (C1) a divalent group (amide bond) derived from a carbamoyl group, which is optionally N-substituted, (C2) a divalent group derived from a 5- to 14-membered aromatic heterocyclic ring, which is optionally substituted, (C3) a divalent group derived from a 3- to 14-membered non-aromatic heterocyclic ring, which is optionally substituted, (C4) a C₃₋₁₀ cycloalkyl group which is optionally substituted, (C5) a C₃₋₁₀ cycloalkenyl group which is optionally substituted, (C6) a 6- to 14-membered aromatic hydrocarbon cyclic group (aryl group) which is optionally substituted, or (C7) an ester bond or a thioester bond,
L³ represents (L³1) a single bond, (L³2) a C₁₋₃ alkylene group, which is optionally linked to a divalent group (amide bond) derived from a carbamoyl group and/or a divalent group derived from an imino group and/or is optionally substituted, (L³3) an ether bond or a thioether bond which is optionally linked to a C₁₋₃ alkenylene group, or (L³4) a divalent group (amide bond) derived from a carbamoyl group, which is optionally linked to a divalent group derived from an amino group, and
D represents (D1) a C₃₋₁₀ cycloalkyl group which is optionally substituted, (D2) a C₃₋₁₀ cycloalkenyl group which is optionally substituted, (D3) a 6- to 14-membered aromatic hydrocarbon cyclic group (aryl group) which is optionally substituted, (D4) a 5- to 14-membered aromatic heterocyclic group which is optionally substituted, (D5) a 3- to 14-membered non-aromatic heterocyclic group which is optionally substituted, or (D6) a C₁₋₃ alkyl group which is optionally substituted.

6. The IL-17A activity inhibitor according to claim 5, wherein the requirements according to any one of claims 1 to 4 are further satisfied.

7. The IL-17A activity inhibitor according to claim 5 or 6, wherein the compound (I) has, as a site at which the hydrogen bonding or CH-π interaction with Cys154 is generated, at least one of:
the site A which is (A6) having a group serving as a donor or an acceptor of a hydrogen atom;
the site B which is (B1) or (B3) having a group serving as a donor or an acceptor of a hydrogen atom;
the site C which is (C1), (C2), (C3), (C6), or (C7) having a group serving as a donor or an acceptor of a hydrogen atom;
the site L¹ which is (L¹2) or (L¹4) having a group serving as a donor or an acceptor of a hydrogen atom, optionally as a substituent;
the site L² which is (L²2) having a group serving as a donor or an acceptor of a hydrogen atom, optionally as a substituent; and
the site C which is (C2) or (C6) having a π electron.

8. The IL-17A activity inhibitor according to claim 5 or 6, wherein the compound (I) has, as a site at which the hydrogen bonding with Asp121 is generated, at least one of site A which is (A3), (A4), or (A6) or at least one site L¹ which is (L¹2).

9. The IL-17A activity inhibitor according to claim 5 or 6, wherein the compound (I) has, as a site at which the CH-π interaction or hydrogen bonding with Pro122 is generated, at least one site A which is (A4) or (A5) or at least one site B which is (B3) or (B5).

10. The IL-17A activity inhibitor according to claim 5 or 6, wherein the compound (I) has, as a site at which the CH-π interaction or hydrogen bonding with Asp 123 is generated, at least one site A which is (A5) or at least one site C which is (C6) or (C8).

11. The IL-17A activity inhibitor according to claim 5 or 6, wherein the compound (I) has, as a site at which the ionic bonding, hydrogen bonding, or cation-π interaction with Lys160 is generated, at least one site D which is (D1), (D3), or (D5).

12. The IL-17A activity inhibitor according to claim 5 or 6, wherein the compound (I) has, as a site at which the CH-π interaction with Ser170 is generated, at least one site D which is (D3) or (D5).

13. The IL-17A activity inhibitor according to any one of claims 5 to 12, wherein the compound (I) is any one of compounds represented by the following structural formulas (1) to (36), respectively, (hereinafter, referred to as "compounds (1) to (36)") or derivatives thereof.
**[Table 1-1]**
| No. | 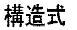 |
|---|---|
| (1) | 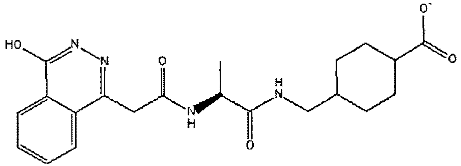 |
| (2) | 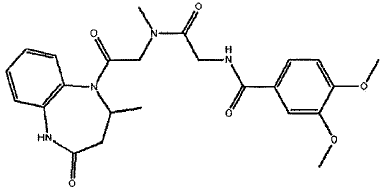 |
| (3) | 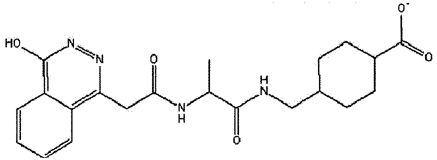 |
| (4) | 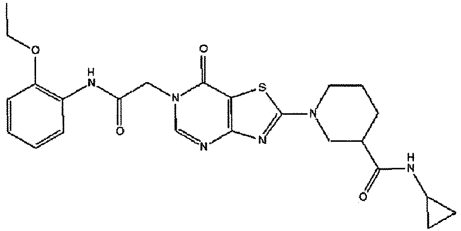 |
| (5) | 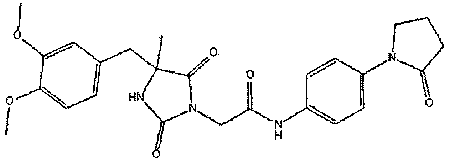 |
| (6) | 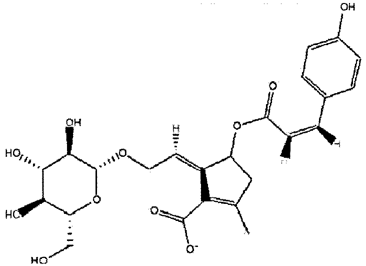 |
**[Table 1-2]**
| | |
|---|---|
| (7) | 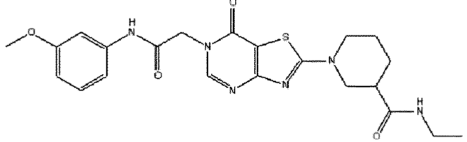 |
| (8) | 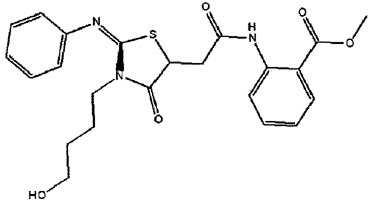 |
| (9) | 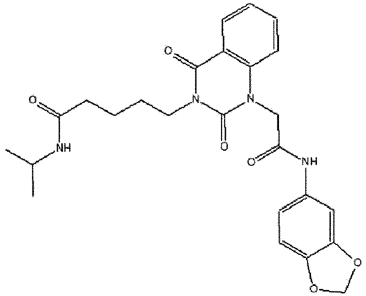 |
| (10) | 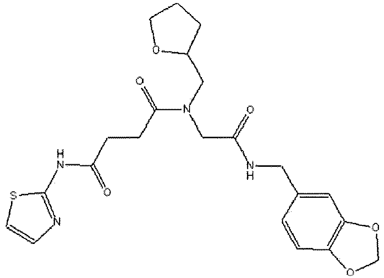 |
| (11) | 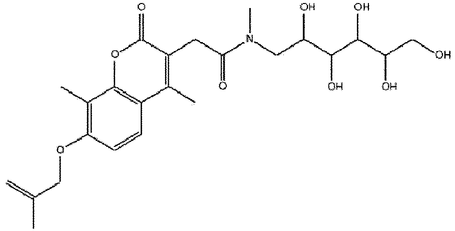 |
**[Table 1-3]**
| | |
|---|---|
| (12) | 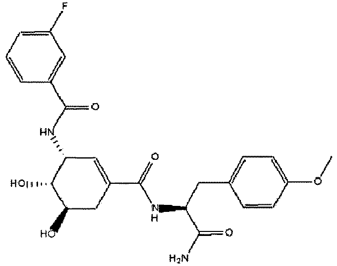 |
| (13) | 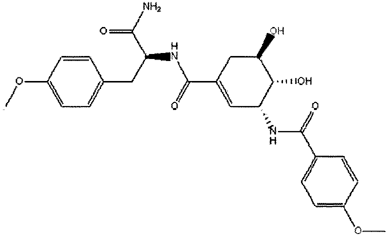 |
| (14) | 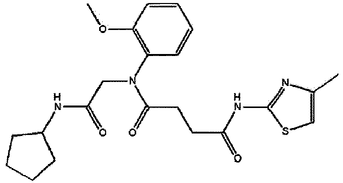 |
| (15) | 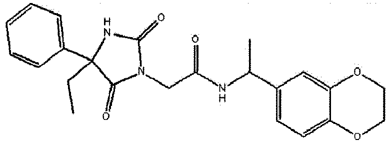 |
| (16) | 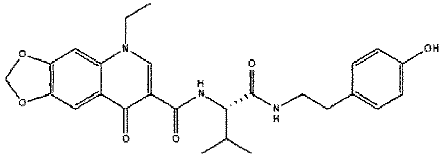 |
| (17) | 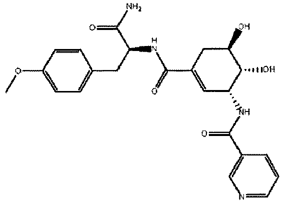 |
**[Table 1-4]**
| | |
|---|---|
| (18) | 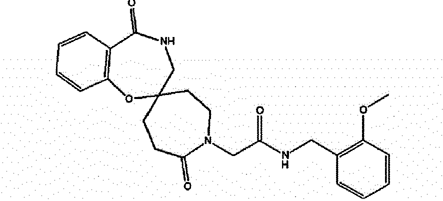 |
| (19) | 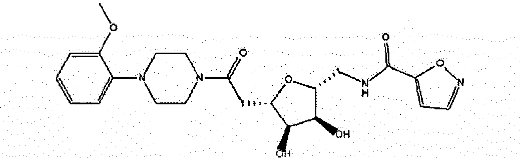 |
| (20) | 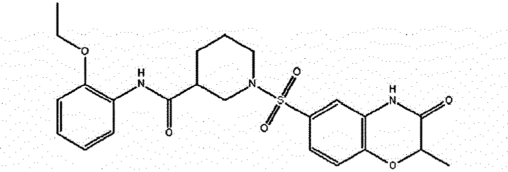 |
| (21) | 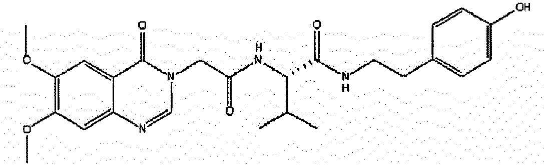 |
| (22) | 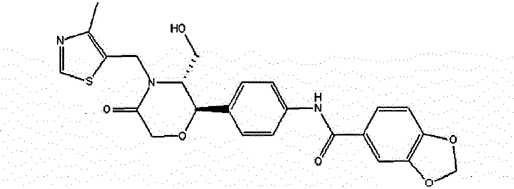 |
| (23) | 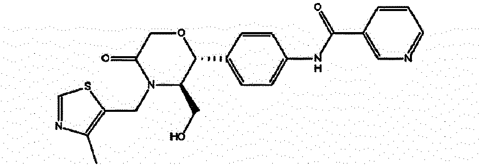 |
| (24) | 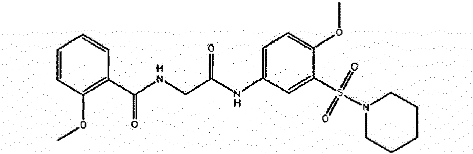 |
**[Table 1-5]**
| | |
|---|---|
| (25) | 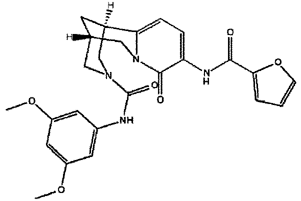 |
| (26) | 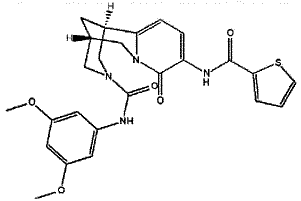 |
| (27) | 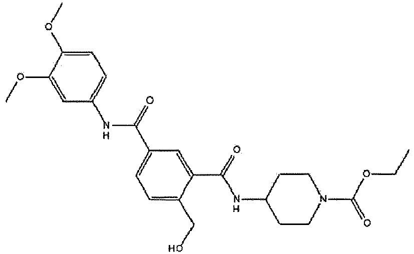 |
| (28) | 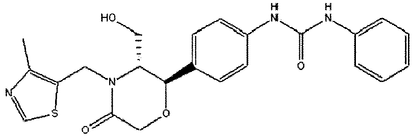 |
| (29) | 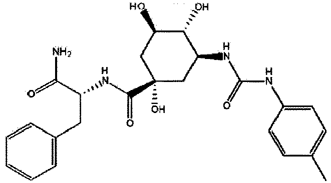 |
| (30) | 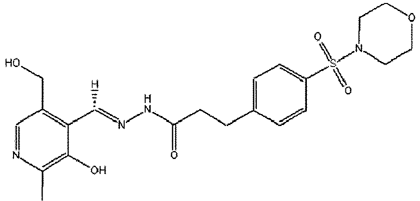 |
**[Table 1-6]**
| | |
|---|---|
| (31) | 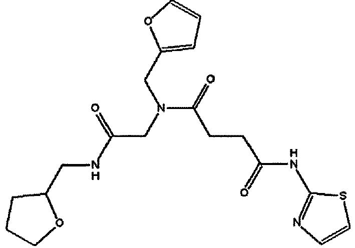 |
| (32) | 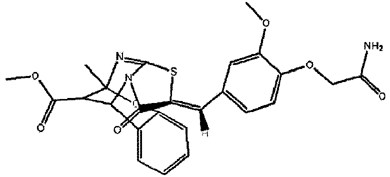 |
| (33) | 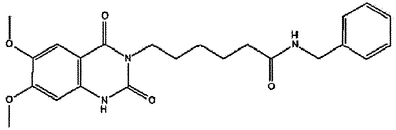 |
| (34) | 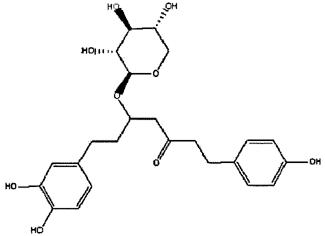 |
| (35) | 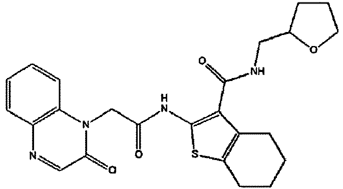 |
| (36) | 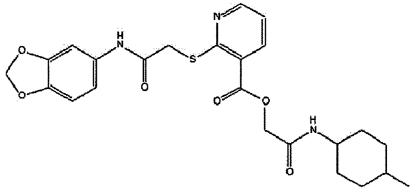 |

14. The IL-17A activity inhibitor according to claim 13, wherein the compound (I) is the compound (1) or the derivative thereof, the compound (I) being obtained by modifying an original compound (1) so that at least one condition selected from the group consisting of [X], [Y], and [Z] below is satisfied:
[X] a total van der Waals force between the compound (I) and Asp121, Pro122, Gln124, Cys154, Glu155, Lys160, Pro164, Ser168, Gly169, Ser170, Ser258, Cys259, Asp262, Cys263, and Leu264 is increased as compared with the compound (1);
[Y] the compound (I) has a site at which at least one of the CH-π interaction with Pro122, the hydrogen bonding with Cys154, and the ionic bonding with Lys160 is increased, or a site at which at least one non-covalent interaction different from the CH-π interaction with Pro122, the hydrogen bonding with Cys154, and the ionic bonding with Lys160 other than the van der Waals force is generated between the compound (I) and at least one amino acid residue selected from the group consisting of Asp121, Pro122, Gln124, Cys154, Glu155, Lys160, Pro164, Ser168, Gly169, Ser170, Ser258, Cys259, Asp262, Cys263, and Leu264, the site being included in the compound (1); and
[Z] the compound (I) has a site at which exposure of at least one amino acid residue selected from the group consisting of Asp121, Pro122, Gln124, Cys154, Glu155, Lys160, Pro164, Ser168, Gly169, Ser170, Ser258, Cys259, Asp262, Cys263, and Leu264 to a solvent is reduced as compared with the compound (1).

15. The IL-17A activity inhibitor according to claim 13, wherein the compound (I) is the compound (2) or the derivative thereof, the compound (I) being obtained by modifying an original compound (2) so that at least one condition selected from the group consisting of [X], [Y], and [Z] below is satisfied:
[X] a total van der Waals force between the compound (I) and Asp121, Pro122, Asp123, Gln124, Asp153, Cys154, Glu155, Pro164, Ser168, Gly169, Ser170, Trp172, Pro254, Phe256, Ser258, Cys259, Asp262, Leu264, and His266 is increased as compared with the compound (2);
[Y] the compound (I) has a site at which at least one of the CH-π interaction with Asp123, the hydrogen bonding with Cys154, and the CH-π interaction with Ser170 is increased, or a site at which at least one non-covalent interaction different from the CH-π interaction with Asp123, the hydrogen bonding with Cys154, and the CH-π interaction with Ser170 other than the van der Waals force is generated between the compound (I) and at least one amino acid residue selected from the group consisting of Asp121, Pro122, Asp123, Gln124, Asp153, Cys154, Glu155, Pro164, Serl68, Gly169, Ser170, Trp172, Pro254, Phe256, Ser258, Cys259, Asp262, Leu264, and His266, the site being included in the compound (2); and
[Z] the compound (I) has a site at which exposure of at least one amino acid residue selected from the group consisting of Asp121, Pro122, Asp123, Gln124, Asp153, Cys154, Glu155, Pro164, Ser168, Gly169, Ser170, Trp172, Pro254, Phe256, Ser258, Cys259, Asp262, Leu264, and His266 to a solvent is reduced as compared with the compound (2).

16. The IL-17A activity inhibitor according to claim 13, wherein the compound (I) is the compound (5) or the derivative thereof, the compound (I) being obtained by modifying an original compound (5) so that at least one condition selected from the group consisting of [X], [Y], and [Z] below is satisfied:
[X] a total van der Waals force between the compound (I) and Asp121, Pro122, Asp123, Asp153, Cys154, Glu155, Lys160, Pro164, Ser168, Gly169, Ser170, Trp172, Ser258, Cys259, Asp262, Cys263, Leu264, and His266 is increased as compared with the compound (5);
[Y] the compound (I) has a site at which at least one of the hydrogen bonding with Cys154 and the hydrogen bonding with Lys160 is increased, or a site at which at least one non-covalent interaction different from the hydrogen bonding with Cys154 and the hydrogen bonding with Lys160 other than the van der Waals force is generated between the compound (I) and at least one amino acid residue selected from the group consisting of Asp121, Pro122, Asp123, Asp153, Cys154, Glu155, Lys160, Pro164, Ser168, Gly169, Ser170, Trp172, Ser258, Cys259, Asp262, Cys263, Leu264, and His266, the site being included in the compound (5); and
[Z] the compound (I) has a site at which exposure of at least one amino acid residue selected from the group consisting of Asp121, Pro122, Asp123, Asp153, Cys154, Glu155, Lys160, Pro164, Ser168, Gly169, Ser170, Trp172, Ser258, Cys259, Asp262, Cys263, Leu264, and His266 to a solvent is reduced as compared with the compound (5).

17. The IL-17A activity inhibitor according to claim 13, wherein the compound (I) is the compound (9) or the derivative thereof, the compound (I) being obtained by modifying an original compound (9) so that at least one condition selected from the group consisting of [X], [Y], and [Z] below is satisfied:
[X] a total van der Waals force between the compound (I) and Asp121, Pro122, Asp123, Asp153, Cys154, Glu155, Lys160, Pro164, Ser167, Ser168, Gly169, Ser170, Trp172, Ser258, Cys259, Asp262, Leu264, and His266 is increased as compared with the compound (9);
[Y] the compound (I) has a site at which at least one of the CH-π interaction with Asp121, the hydrogen bonding with Cys154, and the CH-π interaction with Ser170 is increased, or a site at which at least one non-covalent interaction different from the CH-π interaction with Asp121, the hydrogen bonding with Cys154, and the CH-π interaction with Ser170 other than the van der Waals force is generated between the compound (I) and at least one amino acid residue selected from the group consisting of Asp121, Pro122, Asp123, Asp153, Cys154, Glu155, Lys160, Pro164, Ser167, Ser168, Gly169, Ser170, Trp172, Ser258, Cys259, Asp262, Leu264, and His266, the site being included in the compound (9); and
[Z] the compound (I) has a site at which exposure of at least one amino acid residue selected from the group consisting of Asp121, Pro122, Asp123, Asp153, Cys154, Glu155, Lys160, Pro164, Ser167, Ser168, Gly169, Ser170, Trp172, Ser258, Cys259, Asp262, Leu264, and His266 to a solvent is reduced as compared with the compound (9).

18. The IL-17A activity inhibitor according to claim 13, wherein the compound (I) is the compound (11) or the derivative thereof, the compound (I) being obtained by modifying an original compound (11) so that at least one condition selected from the group consisting of [X], [Y], and [Z] below is satisfied:
[X] a total van der Waals force between the compound (I) and Asp121, Pro122, Gln124, Asp153, Cys154, Glu155, Pro164, Cys165, Ser168, Gly169, Ser170, Trp172, Ser258, Cys259, Asp262, Leu264, and His266 is increased as compared with the compound (11);
[Y] the compound (I) has a site at which at least one of the CH-π interaction or hydrogen bonding with Cys154 is increased, or a site at which at least one non-covalent interaction different from the CH-π interaction or hydrogen bonding with Cys154 other than the van der Waals force is generated between the compound (I) and at least one amino acid residue selected from the group consisting of Asp121, Pro122, Gln124, Asp153, Cys154, Glu155, Pro164, Cys165, Ser168, Gly169, Ser170, Trp172, Ser258, Cys259, Asp262, Leu264, and His266, the site being included in the compound (11); and
[Z] the compound (I) has a site at which exposure of at least one amino acid residue selected from the group consisting of Asp121, Pro122, Gln124, Asp153, Cys154, Glu155, Pro164, Cys165, Ser168, Gly169, Ser170, Trp172, Ser258, Cys259, Asp262, Leu264, and His266 to a solvent is reduced as compared with the compound (11).

19. An expression regulator comprising the IL-17A activity inhibitor according to any one of claims 1 to 18, wherein the expression regulator is used for regulating an expression level of a gene whose expression level is changed by binding of IL-17Ato IL-17RA in a cell expressing IL-17RA

20. The expression regulator according to claim 19, wherein the gene is a gene whose expression is enhanced by binding of IL-17A to IL-17RA, and the expression regulator is used for suppressing the expression of the gene.

21. The expression regulator according to claim 20, wherein the gene is at least one selected from the group consisting of IL-6, COX-2, mPGES1, MMP-3, MMP-13, and CXCL1.

22. The expression regulator according to claim 20, wherein the gene is a gene whose expression is enhanced by phosphorylation of p38, and the expression regulator is used for suppressing the expression of the gene.

23. The expression regulator according to any one of claims 19 to 22, wherein the cell expressing IL-17RA is an intervertebral disc nucleus pulposus cell.

24. The expression regulator according to claim 23, wherein the intervertebral disc nucleus pulposus cell is an intervertebral disc nucleus pulposus cell cultured under a low oxygen condition or an intervertebral disc nucleus pulposus cell present in an intervertebral disc tissue.

25. The expression regulator according to any one of claims 19 to 24, wherein the cell expressing IL-17RA is a keratinocyte or another epidermal cell.

26. A medicament for the treatment or prophylaxis of a disease with a symptom associated with binding of IL-17A to IL-17RA, the medicament comprising the IL-17A activity inhibitor according to any one of claims 1 to 18, or the expression regulator according to any one of claims 19 to 25, as an active ingredient.

27. The medicament according to claim 26, wherein the disease with a symptom associated with binding of IL-17A to IL-17RA is a lumbar or cervical intervertebral disc disease, intervertebral disc hernia, spondylolysis and spondylolisthesis, lumbar spinal canal stenosis, lumbar degenerative spondylolisthesis, or lumbar degenerative scoliosis.

28. The medicament according to claim 26, wherein the disease with a symptom associated with binding of IL-17Ato IL-17RA is psoriasis vulgaris, articular psoriasis, pustular psoriasis, or psoriatic erythroderma.

29. A screening method for an IL-17A activity inhibitor, comprising:
from a three-dimensional molecular model of a space surrounded by 28 amino acid residues of Phe60, Gln87, Asp121, Pro122, Asp123, Gln124, Asp153, Cys154, Glu155, Lys160, Pro164, Cys165, Ser167, Ser168, Gly169, Ser170, Leu171, Trp172, Asp173, Pro174, Pro254, Phe256, Ser258, Cys259, Asp262, Cys263, Leu264, and His266 that are contained in an extracellular domain of human IL-17RA, or a three-dimensional molecular model of a space surrounded by amino acid residues (where homology between the amino acid residues is 80% or more) corresponding to the 28 amino acid residues contained in an extracellular domain of non-human animal IL-17RA, and a three-dimensional molecular model of a candidate compound,
evaluating binding stability between the candidate compound and IL-17RA through a non-covalent interaction including a van der Waals force generated between an atom or an atomic group included in at least 13 amino acid residues among the amino acid residues, and an atom or an atomic group included in the candidate compound, to determine whether the candidate compound has an action of inhibiting binding of IL-17A to IL-17RA by binding to IL-17RA competitively with IL-17A.

30. The screening method according to claim 29, further comprising:
comparing binding stability of the candidate compound with binding stability of each of the compounds (1) to (36).

31. A method of inhibiting binding of IL-17A to IL-17RA, the method comprising:
bringing the IL-17A activity inhibitor according to any one of claims 1 to 16 into contact with IL-17RA outside a living body of a human or another animal.

32. A method of regulating expression of a gene whose expression level is changed by binding of IL-17A to IL-17RA, the method comprising:
bringing the expression regulator according to any one of claims 17 to 22 into contact with a cell expressing IL-17RA outside a living body of a human or another animal.
